(19) **Europäisches Patentamt European Patent Office Office européen des brevets**

(11) **EP 2 376 655 B1**

(12) **EUROPEAN PATENT SPECIFICATION**

(45) Date of publication and mention of the grant of the patent:
**09.10.2019 Bulletin 2019/41**

(21) Application number: **09826923.6**

(22) Date of filing: **16.11.2009**

(51) Int Cl.:
*C12Q 1/6883* (2018.01)   *C07K 14/705* (2006.01)

(86) International application number:
**PCT/US2009/064617**

(87) International publication number:
**WO 2010/057112 (20.05.2010 Gazette 2010/20)**

(54) **GENETIC VARIANTS UNDERLYING HUMAN COGNITION AND METHODS OF USE THEREOF AS DIAGNOSTIC AND THERAPEUTIC TARGETS**

GENETISCHE VARIANTEN DER MENSCHLICHEN KOGNITION UND VERFAHREN ZU IHRER VERWENDUNG ALS DIAGNOSTISCHE UND THERAPEUTISCHE ZIELE

VARIANTS GÉNÉTIQUES INTERVENANT DANS LA COGNITION HUMAINE ET LEURS PROCÉDÉS D'UTILISATION COMME CIBLES DIAGNOSTIQUES ET THÉRAPEUTIQUES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **14.11.2008 US 114921 P**

(43) Date of publication of application:
**19.10.2011 Bulletin 2011/42**

(73) Proprietors:
• **The Children's Hospital Of Philadelphia**
  **Philadelphia, PA 19104 (US)**
• **Trustees of the University of Pennsylvania**
  **Philadelphia, PA 19104 (US)**
• **The Regents of the University of California**
  **Oakland, CA 94607-5200 (US)**

(72) Inventors:
• **HAKONARSON, Hakon**
  **Malvern, PA 19355 (US)**
• **ABRAHAMS, Brett**
  **Los Angeles, CA 90035 (US)**
• **BUCAN, Maja**
  **Philadelphia, PA 19104 (US)**
• **GESCHWIND, Dan**
  **Santa Monica, CA 90402 (US)**
• **HERMAN, Edward**
  **New Haven, CT 06511 (US)**
• **WANG, Kai**
  **Lansdale, PA 19446 (US)**

(74) Representative: **Mewburn Ellis LLP**
  **City Tower**
  **40 Basinghall Street**
  **London EC2V 5DE (GB)**

(56) References cited:
**US-A1- 2005 209 181    US-A1- 2006 051 786**

• **M. Kusenda ET AL: "The role of rare structural variants in the genetics of autism spectrum disorders", Cytogenet Genome Res, 10 September 2008 (2008-09-10), pages 36-43, XP055048619, DOI: 10.1159/000184690 Retrieved from the Internet: URL:http://www.ncbi.nlm.nih.gov/pmc/articl es/PMC2920182/pdf/cgr0123-0036.pdf [retrieved on 2013-01-03]**
• **ROPERS ET AL: "Genetics of intellectual disability", CURRENT OPINION IN GENETICS & DEVELOPMENT, CURRENT BIOLOGY LTD, XX, vol. 18, no. 3, 1 June 2008 (2008-06-01), pages 241-250, XP025472460, ISSN: 0959-437X, DOI: 10.1016/J.GDE.2008.07.008 [retrieved on 2008-08-28]**
• **COSTA E SILVA ET AL: "Autism, a brain developmental disorder: some new pathopysiologic and genetics findings", METABOLISM, CLINICAL AND EXPERIMENTAL, W.B. SAUNDERS CO., PHILADELPHIA, PA, US, vol. 57, 1 October 2008 (2008-10-01), pages S40-S43, XP025412926, ISSN: 0026-0495 [retrieved on 2008-09-16]**

**(Cont. next page)**

- ST CLAIR DAVID: "Copy number variation and schizophrenia", SCHIZOPHRENIA BULLETIN, OXFORD UNIVERSITY PRESS, vol. 35, no. 1, 5 November 2008 (2008-11-05), pages 9-12, XP009123717, ISSN: 0586-7614, DOI: 10.1093/SCHBUL/SBN147
- THE AUTISM GENOME PROJECT CONSORTIUM ET AL: "Genome-wide linkage analyses of quantitative and categorical autism sub-phenotypes", BIOLOGICAL PSYCHIATRY, ELSEVIER SCIENCE, NEW YORK, NY; US, vol. 64, no. 7, 1 October 2008 (2008-10-01), pages 561-570, XP026046033, ISSN: 0006-3223, DOI: 10.1016/J.BIOPSYCH.2008.05.023 [retrieved on 2008-07-16]
- Samuel Levy ET AL: "The Diploid Genome Sequence of an Individual Human", , 1 October 2007 (2007-10-01), XP055339811, DOI: 10.1371/journal.pbio Retrieved from the Internet: URL:http://journals.plos.org/plosbiology/article/file?id=10.1371/journal.pbio.0050254&type=printable [retrieved on 2017-01-27] & Anonymous: "esv1634494 - dbVar Variant - NCBI", , 1 October 2007 (2007-10-01), XP055339813, Retrieved from the Internet: URL:https://www.ncbi.nlm.nih.gov/dbvar/variants/esv1634494/ [retrieved on 2017-01-27]
- Agilent: "Open to Anything: The Agilent Genomics Portfolio", , 1 June 2008 (2008-06-01), XP055339867, Retrieved from the Internet: URL:http://www.agilent.com/cs/library/brochures/IBS-231 genomics catalog mech FINAL.pdf [retrieved on 2017-01-27]
- SEO ET AL.: 'An accurate method for quantifying and analyzing copy number variation in porcine KIT by an oligonucleotide ligation assay.' BMC GENET. vol. 8, no. 1, 23 November 2007, pages 1 - 11, XP021032607 DOI: 10.1186/1471-2156-8-81
- MARSHALL ET AL.: 'Structural variation of chromosomes in autism spectrum disorder.' AM. J. HUM. GENET. vol. 82, no. 2, 01 February 2008, pages 477 - 488, XP002637348 DOI: 10.1016/J.AJHG.2007.12.009
- KUOKKANEN ET AL.: 'Genomewide scan of multiple sclerosis in Finnish multiplex families.' AM. J. HUM. GENET. vol. 61, no. 6, 01 December 1997, pages 1379 - 1387, XP055276648 DOI: 10.1086/301637
- CHARDENOT ET AL.: 'Expression profile and up-regulation of PRAX-1 mRNA by antidepressant treatment in the rat brain.' MOL. PHARMACOL. vol. 62, no. 6, 01 December 2002, pages 1314 - 1320, XP055276650 DOI: 10.1124/MOL.62.6.1314
- GALLIEGUE ET AL.: 'Cloning and characterization of PRAX-1. A new protein that specifically interacts with the peripheral benzodiazepine receptor.' J. BIOL. CHEM. vol. 274, no. 5, 29 January 1999, pages 2938 - 2952, XP002122735 DOI: 10.1074/JBC.274.5.2938
- BUCAN ET AL.: 'Genome-wide analyses of exonic copy number variants in a family-based study point to novel autism susceptibility genes.' PLOS GENET. vol. 5, no. 6, 26 June 2009, pages 1 - 12, XP009140103

**Description**

**FIELD OF THE INVENTION**

This invention relates to the fields of genetics and the diagnosis and treatment of cognitive and neurological disorders. More specifically, the invention provides nucleic acids comprising copy number variations (CNVs) which are associated with the multiple disorders of human cognition and behavior and methods of use thereof in diagnostic and therapeutic applications.

**BACKGROUND OF THE INVENTION**

Neurologic diseases can result from disorders of the brain, spinal cord and nerves. Patients experiencing neurological disease may have trouble moving, speaking, swallowing, breathing or learning. Problems with memory, senses behavior or mood are also associated with neurological disorders. There are many different underlying causes of neurological dysfunction. These can include genetic mutation, exposure to toxic substances and injury.

There are more than 600 neurologic diseases. Major types include diseases caused by faulty genes, such as Huntington's disease and muscular dystrophy; aberrant embryonal development of the nervous system, such as spina bifida; degenerative diseases, where nerve cells are damaged or die, such as Parkinson's disease and Alzheimer's disease; diseases of the blood vessels that supply the brain, such as stroke; injuries to the spinal cord and brain; seizure disorders, such as epilepsy; cancer, such as brain tumors and infections, such as meningitis.

Multiple disorders of human cognition and behavior appear to be modulated by genetic factors. However, the manner by which genetic variation impacts disease is complex and poorly understood. Similarly elusive are the identity of specific genes that may be useful with regards to diagnosis and therapeutic intervention. It is an object of the invention to provide these genetic markers and to further characterize the alterations therein that lead to a loss of cognitive function and neurological development.

Kusenda et al (Cytogenet Genome Res. 123:36-43 (2008)) provide a review of rare mutations that have been identified at many different locations in the genome associated with autism spectrum disorders. Ropers (Current Opinion in Genetics & Development, Current Biology Ltd, XX Vol. 18, No. 3 (2008), p.241-250) also provides a review of the genetics of intellectual disability.

Costa e Silva (Metabolism, Clinical and Experimental, vol. 57, (2008), p. S40-S43) further reviews the etiology of autism and suggests that it may involve an interaction between genetic susceptibility (mediated by multiple genes) and environmental factors influencing brain development.

David St Clair (Schizophrenia Bulletin, Oxford University Press, Vol. 35, no. 1, (2008), p. 9-12) discusses copy number variants associated with schizophrenia and are also associated with increased risk of other disorders such as autism.

Lui et al (Biol. Psychiatry 2008; 64:561-570) discloses genome-wide linkage analyses of quantitative and catergorical autism subphenotypes. They conclude that the selection of informative subphenotypes to define a homogeneous set of ASD families could be very important in detecting the susceptibility loci in Autism.

Levy et al (PLoS Biology, October 2007, vol. 5, Issue 10) discloses the whole genome sequence of an individual human in which a CNV in BZRAP1 was detected.

**SUMMARY OF THE INVENTION**

In accordance with the present invention, there is a provided a method for detecting a propensity for developing a neurological disorder, the method comprising: detecting the presence of at least one copy number variation (CNV) in a target polynucleotide obtained from a patient wherein if said CNV is present, said patient has an increased risk for developing a neurological disorder, wherein said deletion containing CNV is BZRAP1 Benzodiazapine receptor (peripheral) associated protein 1 17q22-q23 chr17:53733592-53761151; and wherein the neurological disorder is autism or autism spectrum disorder.

The method may comprise detecting the presence of at least one further deletion containing CNV selected from MDGA2 MAM domain containing glycosylphosphatidylinositol anchor 214q21.3 chr14:46,170,380-47,422,368, ; CLCNKA chloride channel Ka chr1:16221072-16233132, ; NTRK1 Neurotrophic tyrosine kinase, receptor, type 1 1q21-q22 chr1:155,013,407-155,202,154, USH2A Usher syndrome 2A (autosomal recessive, mild) 1q41 chr1:213,752,880-214,875,391,; NRXN1 Neurexin 1 2p16.3 chr2:49,712,184-51,360,413, ; GALNT13 UDP-N-acetyl-alpha-D-galactosamine:polypeptide N-acetylgal 2q23.3-q24.1 chr2:153,854,689-155,600,757, ; GMPS Guanine monophosphate synthetase 3q24 chr3:157,059,820-157,149,414, ; SPON2 Spondin 2, extracellular matrix protein 4pl6.3 chr4:1,124,285-1,183,034, ; LRBA LPS-responsive vesicle trafficking, beach and anchor containin4q31.3 chr4:151,217,225-152,344,150, ; TPPP Tubulin polymerization promoting protein 5p15.3 chr5:567,501-892,810, ;

SKIV2L2 Superkiller viralicidic activity 2-like 2 (S. cerevisiae) 5q11.2 chr5:54,522,183-54,873,752, ; KIAA1586 KIAA1586 6p12.1 chr6:56,980,593-57,066,702, ; BTN2A1 Butyrophilin, subfamily 2, member A1 6p22.1 chr6:26566167-26577844, ; BXDC1 Brix domain containing 1 6q21 chr6:111409983-111453487, ; LAMA2 Laminin, alpha 2 (merosin, congenital muscular dystrophy) 6q22-q23 chr6:128,945,101-130,370,307, ; DGKB Diacylglycerol kinase, beta 90kDa 7p21.2 chr7:14,015,810-15,013,734, ; RNF133 Ring finger protein 133 7q31.32 chr7:122,118,508-122,132,937, ; RNF148 Ring finger protein 148 7q31.33 chr7:122,118,508-122,132,937,; SLC18A1 Solute carrier family 18 (vesicular monoamine), member 1 8p21.3 chr8:19,874,095-20,257,554, ; COL27A1 Collagen, type XXVII, alpha 1 9q32 chr9:115958051-116112796, ; OR2AG1 Olfactory receptor, family 2, subfamily AG, member 1 11p15.4 chr11:6762845-6763795, ; OR2AG2 Olfactory receptor, family 2, subfamily AG, member 2 11p15.4 chr11:6745814-6746764, ; SSSCA1 Sjogren syndrome/scleroderma autoantigen 1 11q13.1 chr11:65094518-65095815, ; FAM89B Family with sequence similarity 89, member B 11q23 chr11:65,094,554-65,100,079, ; PRB3 Proline-rich protein BstNI subfamily 3 12p13.2 chr12:11310124-11313908, ; KRT3 Keratin 3 12q12-q13 chr12:51,444,040-51,501,855, ; SLC6A15 Solute carrier family 6, member 15 12q21.3 chr12:83,670,976-83,958,489,; DACH1 Dachshund homolog 1 (Drosophila) 13q22 chr13:70910098-71339331, ; LOC650137 Seven transmembrane helix receptor 15q11.2 chr15:19,812,808-20,018,007, ; OR4M2 Olfactory receptor, family 4, subfamily M, member 2 15q11.2 chr15:19,812,808-20,018,007, ; OR4N4 Hypothetical LOC727924 15q11.2 chr15:19,812,808-20,018,007, ; LOC162073 hypothetical protein LOC162073 16p12.3 chr16:19,008,005-19,060,144, ; DLGAP1 Discs, large (Drosophila) homolog-associated protein 1 18p11.3 chr18:3,393,512-3,965,460, ; FLJ44894 Homo sapiens cDNA FLJ44894 fis, clone BRAMY3000692, m 19p12 chr19:20,227,461-20,491,547, ; CYP4F22 Cytochrome P450, family 4, subfamily F, polypeptide 22 19p13.12 chr19:15480335-15524128, ; GRIK5 Glutamate receptor, ionotropic, kainate 5 19q13.2 chr19:47,126,828-47,329,282, ; GYG2 Glycogenin 2 Xp22.3 chrX:2,656,547-2,925,352, ; XG Xg pseudogene, Y-linked 2 Xp22.33 chrX:2,656,547-2,925,353, ; FGF13 Fibroblast growth factor 13 Xq26.3 chrX:137,421,326-138,459,367, ; SPANXB1 SPANX family, member B2 Xq27.1 chrX: 139,908,245-139,941,724, and SPANXB2 SPANX family, member B2 Xq27.1 chrX:139,908,245-139,941,724; or the group of CNVs consisting of CNVs set forth in Table 6.

The invention further provides a method for identifying therapeutic agents which alter neuronal signaling and/or morphology, comprising a) providing cells comprising at least one CNV; b) providing cells which comprise the cognate wild type sequences corresponding to the CNV of step a); c) contacting the cells of steps a) and b) with a test agent and d) analyzing whether said agent alters neuronal signaling and/or morphology of cells of step a) relative to those of step b), thereby identifying agents which alter neuronal signaling and morphology; wherein said at least one CNV is

BZRAP1 Benzodiazapine receptor (peripheral) associated protein 117q22-q23 chr17:53733592-53761151.

Also disclosed herein is at least one isolated neurological disorder related CNV-containing nucleic acid selected from the group that are either exclusive to or significantly overrepresented in neurological disorders, particularly ASD (see Table 1 and Table 7). Such CNV containing nucleic acids may optionally be contained in a suitable expression vector for expression in neuronal cells. Alternatively, they may be immobilized on a solid support.

## BRIEF DESCRIPTION OF THE DRAWINGS

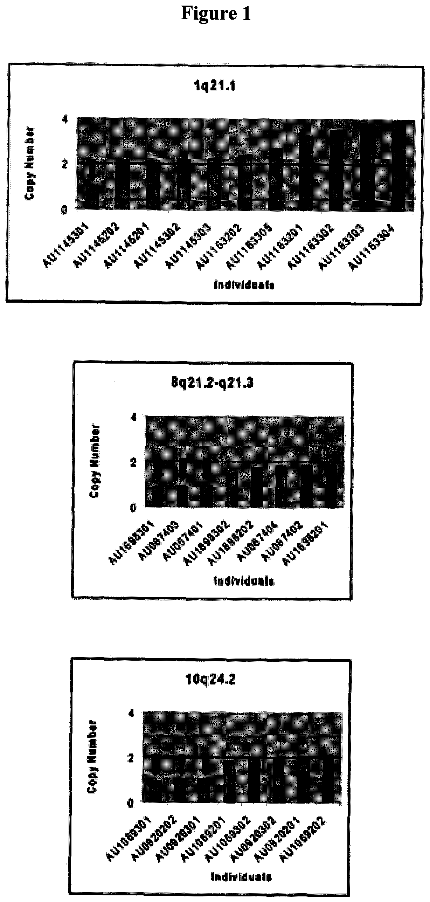

**Figure 1 - TaqMan experiments validate copy number calls determined by PennCNV.** To validate results using an independent method we designed TaqMan assays to evaluate gene dosage. Results from representative experiments highlight results at loci at 1q21, 8q21, and 10q24. AGRE individual harboring deletions (red arrows) or gains (green arrows) are indicated.

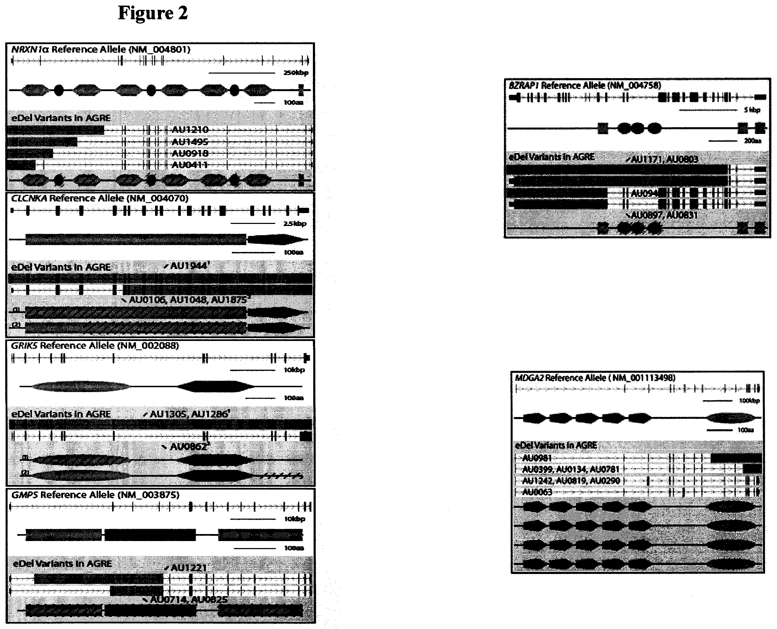

**Figure 2 - Rare exonic deletions (eDels) in *NRXN1* and novel candidate genes alter predicted protein structures.** For each of *NRXN1* (a), *CLCKNKA* (b), *GRIK5* (c), and *GMPS* (d) reference loci and encoded proteins (top) are contrasted against mutant loci and proteins (bottom; grey shading). Unique genomic deletions and corresponding protein truncations are highlighted in red and with black hatching, respectively. Schematized protein domains genes are as follows: *NRXN1* -- Laminin G (orange hexagon), EGF-like (blue oval), 4.1 binding motif (green rectangle); *CLCNKA* -- Chloride channel, core (orange rectangle), Cystathionine beta-synthase, core (blue pentagon); *GRIK5* -- Extracellular ligand-binding receptor (orange oval), Ionotropic glutamate receptor (blue hexagon); *GMPS* -- Glutamine amidotransferase class-I, C-terminal (orange rectangle), Exoenzyme S synthesis protein B/queuosine synthesis (blue rectangle), (GMP synthase, C-terminal (green rectangle). Rare exonic deletions (eDels) in NRXN1 and novel candidate genes alter predicted protein structures. For each of BZRAP1 and MDGA2 (c) reference loci and encoded proteins (top) are contrasted against mutant loci and corresponding proteins (bottom; grey shading). Unique genomic deletions and corresponding protein truncations are highlighted in red and with black hatching, respectively. Schematized protein domains genes are as follows: BZRAP1-Src homology-3 (orange square), Fibronectin, type III (blue oval); MDGA2-IG-like domains (blue pentagon), MAM aka Meprin/A5-protein/PTPmu (blue oval).

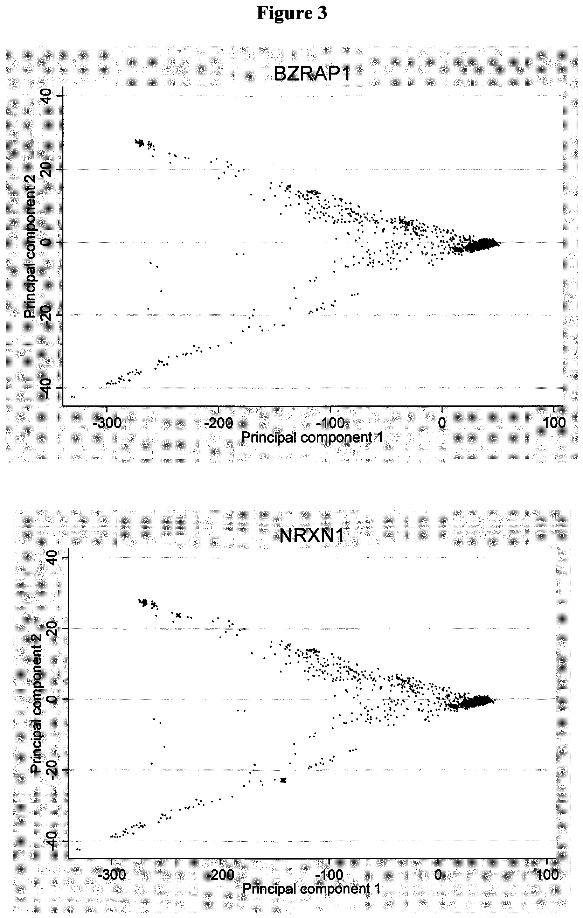

**Figure 3 - Multi-dimensional scaling plot of AGRE affected subjects, with red cross highlighting subjects carrying the eDels.** Subjects of European ancestry are clustered toward the right side of the triangle.

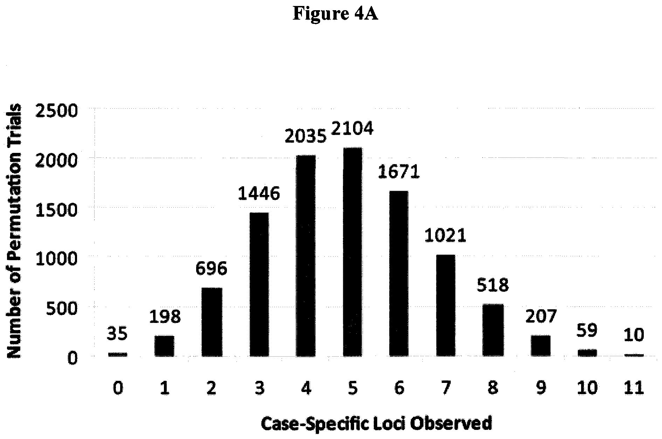

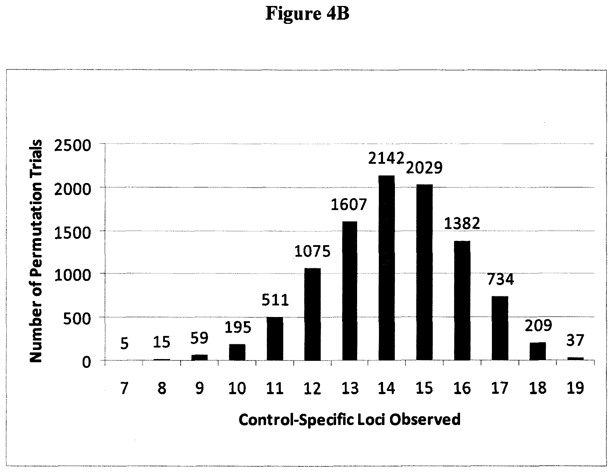

**Figure 4A** - **Observed replication unlikely to be attributable to chance alone.** We performed 10,000 phenotype permutation trials on replication data and determined for each the number of loci harboring CNVs in cases but not controls. Thus, within each trial, the number of loci absent from controls in the replication cohort was determined. None of the permutation trials generated as many case-specific loci as observed in our actual dataset (n = 14; p<0.0001). Figure 4B. We also performed 10,000 phenotype permutation trials on replication data and determined for each the number of loci harboring CNVs exclusively in controls. During each trial a new set of control-specific loci was identified and the number of these absent from cases determined. We observed results comparable to those obtained experimentally (n=18) in 246 of 10,000 trials (p=0.02)

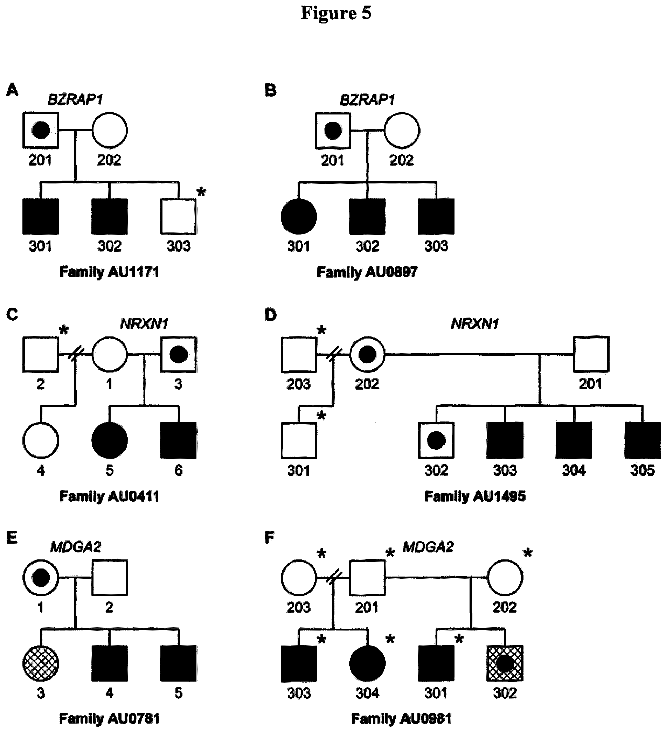

**Figure 5 - Exonic deletions, although enriched in cases versus controls, show imperfect segregation with disease in multiplex families.** Pedigrees for representative AGRE families harboring exonic deletions in BZRAP1 (A,B), kb), NRXN1 (C,D), and MDGA2 (E,F) are illustrated. Red filled circles correspond to exonic deletions. Black stars (upper right) highlight individuals for which CNV calls were not obtained (not genotyped or failing to meet criteria for quality control).

## DETAILED DESCRIPTION OF THE INVENTION

The genetics underlying the neurological disorders (e.g., autism, autism spectrum disorder (ASD) schizophrenia, bipolar disorder, Tourette's syndrome, obsessive compulsive disorder (OCD) is highly complex and remains poorly understood. Previous work has demonstrated an important role for structural variation in a subset of cases, but the analysis lacked the resolution necessary to move beyond detection of large regions of potential interest to identification of individual genes. Autism spectrum disorders (ASDs) are common neurodevelopmental syndromes with a strong genetic component. ASDs are characterized by disturbances in social behavior, impaired verbal and nonverbal communication, as well as repetitive behaviors and/or a restricted range of interests. To identify genes likely to contribute to ASD etiology, we performed high density genotyping in 912 multiplex families from the Autism Genetics Resource Exchange (AGRE) collection and contrasted results to those obtained for 1,488 healthy controls. To enrich for variants most likely to interfere with gene function, we restricted our analyses to deletions and gains encompassing exons. Of the many genomic regions highlighted, 27 were seen to harbor rare variants in cases and not controls, both in the first phase of our analysis, and also in an independent replication cohort comprised of 859 cases and 1,051 controls. The genes identified by this method include *NRXN1*, a molecule in which rare ASD-related variation has been well documented by multiple groups. We find comparable support for several genes not previously implicated in the ASDs, including *BZRAP1, MDGA2, CLCNKA, GRIK5* and *GMPS.* For each of these, mutant alleles eliminate entirely or remove the majority of protein coding sequences. Importantly, interrogation of an independently ascertained and non-overlapping ASD cohort identified eDels in these same genes in almost a third of cases, a result unlikely to occur by chance alone (p = 1 x 10$^{-36}$ by Fisher Exact). These newly identified autism susceptibility genes will be useful in understanding key signaling pathways dysregulated in this group of disorders.

**Definitions:**

A "copy number variation (CNV)" refers to the number of copies of a particular gene in the genotype of an individual. CNVs represent a major genetic component of human phenotypic diversity. Susceptibility to genetic disorders is known to be associated not only with single nucleotide polymorphisms (CNV), but also with structural and other genetic variations, including CNVs. A CNV represents a copy number change involving a DNA fragment that is -1 kilobases (kb) or larger (Feuk et al. 2006 Nature. 444:444-54.). CNVs described herein do not include those variants that arise from the insertion/deletion of transposable elements (e.g., -6-kb Kpnl repeats) to minimize the complexity of future CNV analyses. The term CNV therefore encompasses previously introduced terms such as large-scale copy number variants (LCVs; Iafrate et al. 2004, Nature Genetics 36: 949-51), copy number polymorphisms (CNPs; Sebat et al. 2004 Science 305:525-8.), intermediate-sized variants (ISVs; Tuzun et al. 2006 Genome Res. 16: 949-961), and eDELs, but not retroposon insertions.

A "single nucleotide polymorphism (SNP)" refers to a change in which a single base in the DNA differs from the usual base at that position. These single base changes are called SNPs or "snips." Millions of SNPs have been cataloged in the human genome. Some SNPs such as that which causes sickle cell are responsible for disease. Other SNPs are normal variations in the genome.

A neurological disorder includes, without limitation, schizophrenia, bipolar disorder, autism, autism spectrum disorder (ASD), Tourette Syndrome, and obsessive compulsive disorder.

The term "genetic alteration" which encompasses a CNV or SNP as defined above, refers to a change from the wild-type or reference sequence of one or more nucleic acid molecules. Genetic alterations include without limitation, base pair substitutions, additions and deletions of at least one nucleotide from a nucleic acid molecule of known sequence.

The term "solid matrix" as used herein refers to any format, such as beads, microparticles, a microarray, the surface of a microtitration well or a test tube, a dipstick or a filter. The material of the matrix may be polystyrene, cellulose, latex, nitrocellulose, nylon, polyacrylamide, dextran or agarose.

The phrase "consisting essentially of" when referring to a particular nucleotide or amino acid means a sequence having the properties of a given SEQ ID NO:. For example, when used in reference to an amino acid sequence, the phrase includes the sequence per se and molecular modifications that would not affect the functional and novel characteristics of the sequence.

"Target nucleic acid" as used herein refers to a previously defined region of a nucleic acid present in a complex nucleic acid mixture wherein the defined wild-type region contains at least one known nucleotide variation which may or may not be associated with neurological disorder. The nucleic acid molecule may be isolated from a natural source by cDNA cloning or subtractive hybridization or synthesized manually. The nucleic acid molecule may be synthesized manually by the triester synthetic method or by using an automated DNA synthesizer. With regard to nucleic acids used in the invention, the term "isolated nucleic acid" is sometimes employed. This term, when applied to DNA, refers to a DNA molecule that is separated from sequences with which it is immediately contiguous (in the 5' and 3' directions) in the naturally occurring genome of the organism from which it was derived. For example, the "isolated nucleic acid" may comprise a DNA molecule inserted into a vector, such as a plasmid or virus vector, or integrated into the genomic DNA of a prokaryote or eukaryote. An "isolated nucleic acid molecule" may also comprise a cDNA molecule. An isolated nucleic acid molecule inserted into a vector is also sometimes referred to herein as a recombinant nucleic acid molecule.

With respect to RNA molecules, the term "isolated nucleic acid" primarily refers to an RNA molecule encoded by an isolated DNA molecule as defined above. Alternatively, the term may refer to an RNA molecule that has been sufficiently separated from RNA molecules with which it would be associated in its natural state (i.e., in cells or tissues), such that it exists in a "substantially pure" form.

By the use of the term "enriched" in reference to nucleic acid it is meant that the specific DNA or RNA sequence constitutes a significantly higher fraction (2-5 fold) of the total DNA or RNA present in the cells or solution of interest than in normal cells or in the cells from which the sequence was taken. This could be caused by a person by preferential reduction in the amount of other DNA or RNA present, or by a preferential increase in the amount of the specific DNA or RNA sequence, or by a combination of the two. However, it should be noted that "enriched" does not imply that there are no other DNA or RNA sequences present, just that the relative amount of the sequence of interest has been significantly increased.

It is also advantageous for some purposes that a nucleotide sequence be in purified form. The term "purified" in reference to nucleic acid does not require absolute purity (such as a homogeneous preparation); instead, it represents an indication that the sequence is relatively purer than in the natural environment (compared to the natural level, this level should be at least 2-5 fold greater, e.g., in terms of mg/ml). Individual clones isolated from a cDNA library may be purified to electrophoretic homogeneity. The claimed DNA molecules obtained from these clones can be obtained directly from total DNA or from total RNA. The cDNA clones are not naturally occurring, but rather are preferably obtained via manipulation of a partially purified naturally occurring substance (messenger RNA). The construction of a cDNA library from mRNA involves the creation of a synthetic substance (cDNA) and pure individual cDNA clones can be isolated from the synthetic library by clonal selection of the cells carrying the cDNA library. Thus, the process which includes the construction of a cDNA library from mRNA and isolation of distinct cDNA clones yields an approximately $10^{-6}$-fold purification of the native message. Thus, purification of at least one order of magnitude, preferably two or three orders, and more preferably four or five orders of magnitude is expressly contemplated.

The term "substantially pure" refers to a preparation comprising at least 50-60% by weight the compound of interest (e.g., nucleic acid, oligonucleotide, etc.). More preferably, the preparation comprises at least 75% by weight, and most preferably 90-99% by weight, the compound of interest. Purity is measured by methods appropriate for the compound of interest.

The term "complementary" describes two nucleotides that can form multiple favorable interactions with one another. For example, adenine is complementary to thymine as they can form two hydrogen bonds. Similarly, guanine and cytosine are complementary since they can form three hydrogen bonds. Thus, if a nucleic acid sequence contains the following sequence of bases, thymine, adenine, guanine and cytosine, a "complement" of this nucleic acid molecule would be a molecule containing adenine in the place of thymine, thymine in the place of adenine, cytosine in the place of guanine, and guanine in the place of cytosine. Because the complement can contain a nucleic acid sequence that forms optimal interactions with the parent nucleic acid molecule, such a complement can bind with high affinity to its parent molecule.

With respect to single stranded nucleic acids, particularly oligonucleotides, the term "specifically hybridizing" refers to the association between two single-stranded nucleotide molecules of sufficiently complementary sequence to permit such hybridization under pre-determined conditions generally used in the art (sometimes termed "substantially complementary"). In particular, the term refers to hybridization of an oligonucleotide with a substantially complementary sequence contained within a single-stranded DNA or RNA molecule of the invention, to the substantial exclusion of hybridization of the oligonucleotide with single-stranded nucleic acids of non-complementary sequence. For example, specific hybridization can refer to a sequence which hybridizes to any neurological disorder specific marker gene or nucleic acid, but does not hybridize to other nucleotides. Also polynucleotide which "specifically hybridizes" may hybridize only to a neurospecific specific marker, such a neurological disorder-specific marker shown in the Tables contained herein. Appropriate conditions enabling specific hybridization of single stranded nucleic acid molecules of varying complementarity are well known in the art.

For instance, one common formula for calculating the stringency conditions required to achieve hybridization between nucleic acid molecules of a specified sequence homology is set forth below (Sambrook et al., Molecular Cloning, Cold Spring Harbor Laboratory (1989):

$$T_m = 81.5°C + 16.6Log\ [Na+] + 0.41(\%\ G+C) - 0.63\ (\%\ formamide) - 600/\#bp\ in\ duplex$$

As an illustration of the above formula, using [Na+] = [0.368] and 50% formamide, with GC content of 42% and an average probe size of 200 bases, the $T_m$ is 57°C. The $T_m$ of a DNA duplex decreases by 1 - 1.5°C with every 1% decrease in homology. Thus, targets with greater than about 75% sequence identity would be observed using a hybridization temperature of 42°C.

The stringency of the hybridization and wash depend primarily on the salt concentration and temperature of the solutions. In general, to maximize the rate of annealing of the probe with its target, the hybridization is usually carried out at salt and temperature conditions that are 20-25°C below the calculated $T_m$ of the hybrid. Wash conditions should be as stringent as possible for the degree of identity of the probe for the target. In general, wash conditions are selected to be approximately 12-20°C below the $T_m$ of the hybrid. In regards to the nucleic acids of the current invention, a moderate stringency hybridization is defined as hybridization in 6X SSC, 5X Denhardt's solution, 0.5% SDS and 100 μg/ml denatured salmon sperm DNA at 42°C, and washed in 2X SSC and 0.5% SDS at 55°C for 15 minutes. A high stringency hybridization is defined as hybridization in 6X SSC, 5X Denhardt's solution, 0.5% SDS and 100 μg/ml denatured salmon sperm DNA at 42°C, and washed in 1X SSC and 0.5% SDS at 65°C for 15 minutes. A very high stringency hybridization is defined as hybridization in 6X SSC, 5X Denhardt's solution, 0.5% SDS and 100 μg/ml denatured salmon sperm DNA at 42°C, and washed in 0.1X SSC and 0.5% SDS at 65°C for 15 minutes.

The term "oligonucleotide," as used herein is defined as a nucleic acid molecule comprised of two or more ribo- or deoxyribonucleotides, preferably more than three. The exact size of the oligonucleotide will depend on various factors and on the particular application and use of the oligonucleotide. Oligonucleotides, which include probes and primers, can be any length from 3 nucleotides to the full length of the nucleic acid molecule, and explicitly include every possible number of contiguous nucleic acids from 3 through the full length of the polynucleotide. Preferably, oligonucleotides are at least about 10 nucleotides in length, more preferably at least 15 nucleotides in length, more preferably at least about 20 nucleotides in length.

The term "probe" as used herein refers to an oligonucleotide, polynucleotide or nucleic acid, either RNA or DNA, whether occurring naturally as in a purified restriction enzyme digest or produced synthetically, which is capable of annealing with or specifically hybridizing to a nucleic acid with sequences complementary to the probe. A probe may be either single-stranded or double-stranded. The exact length of the probe will depend upon many factors, including temperature, source of probe and use of the method. For example, for diagnostic applications, depending on the complexity of the target sequence, the oligonucleotide probe typically contains 15-25 or more nucleotides, although it may contain fewer nucleotides. The probes herein are selected to be complementary to different strands of a particular target nucleic acid sequence. This means that the probes must be sufficiently complementary so as to be able to "specifically hybridize" or anneal with their respective target strands under a set of pre-determined conditions. Therefore, the probe sequence need not reflect the exact complementary sequence of the target. For example, a non-complementary nucleotide fragment may be attached to the 5' or 3' end of the probe, with the remainder of the probe sequence being complementary to the target strand. Alternatively, non-complementary bases or longer sequences can be interspersed into the probe, provided that the probe sequence has sufficient complementarity with the sequence of the target nucleic acid to anneal therewith specifically.

The term "primer" as used herein refers to an oligonucleotide, either RNA or DNA, either single-stranded or double-stranded, either derived from a biological system, generated by restriction enzyme digestion, or produced synthetically which, when placed in the proper environment, is able to functionally act as an initiator of template-dependent nucleic acid synthesis. When presented with an appropriate nucleic acid template, suitable nucleoside triphosphate precursors of nucleic acids, a polymerase enzyme, suitable cofactors and conditions such as a suitable temperature and pH, the primer may be extended at its 3' terminus by the addition of nucleotides by the action of a polymerase or similar activity to yield a primer extension product. The primer may vary in length depending on the particular conditions and requirement of the application. For example, in diagnostic applications, the oligonucleotide primer is typically 15-25 or more nucleotides in length. The primer must be of sufficient complementarity to the desired template to prime the synthesis of the desired extension product, that is, to be able anneal with the desired template strand in a manner sufficient to provide the 3' hydroxyl moiety of the primer in appropriate juxtaposition for use in the initiation of synthesis by a polymerase or similar enzyme. It is not required that the primer sequence represent an exact complement of the desired template. For example, a non-complementary nucleotide sequence may be attached to the 5' end of an otherwise complementary primer. Alternatively, non-complementary bases may be interspersed within the oligonucleotide primer sequence, provided that the primer sequence has sufficient complementarity with the sequence of the desired template strand to functionally provide a template-primer complex for the synthesis of the extension product. Polymerase chain reaction (PCR) has been described in US Patents 4,683,195, 4,800,195, and 4,965,188, the entire disclosures of which are incorporated by reference herein.

The term "vector" relates to a single or double stranded circular nucleic acid molecule that can be infected, transfected or transformed into cells and replicate independently or within the host cell genome. A circular double stranded nucleic acid molecule can be cut and thereby linearized upon treatment with restriction enzymes. An assortment of vectors, restriction enzymes, and the knowledge of the nucleotide sequences that are targeted by restriction enzymes are readily available to those skilled in the art, and include any replicon, such as a plasmid, cosmid, bacmid, phage or virus, to which another genetic sequence or element (either DNA or RNA) may be attached so as to bring about the replication of the attached sequence or element. A nucleic acid molecule of the invention can be inserted into a vector by cutting the vector with restriction enzymes and ligating the two pieces together.

Many techniques are available to those skilled in the art to facilitate transformation, transfection, or transduction of the expression construct into a prokaryotic or eukaryotic organism. The terms "transformation", "transfection", and "transduction" refer to methods of inserting a nucleic acid and/or expression construct into a cell or host organism. These methods involve a variety of techniques, such as treating the cells with high concentrations of salt, an electric field, or detergent, to render the host cell outer membrane or wall permeable to nucleic acid molecules of interest, microinjection, PEG-fusion, and the like.

The term "promoter element" describes a nucleotide sequence that is incorporated into a vector that, once inside an appropriate cell, can facilitate transcription factor and/or polymerase binding and subsequent transcription of portions of the vector DNA into mRNA. In one embodiment, the promoter element of the present invention precedes the 5' end of the neurological disorder specific marker nucleic acid molecule such that the latter is transcribed into mRNA. Host cell machinery then translates mRNA into a polypeptide.

Those skilled in the art will recognize that a nucleic acid vector can contain nucleic acid elements other than the promoter element and the neurological disorder specific marker gene nucleic acid molecule. These other nucleic acid elements include, but are not limited to, origins of replication, ribosomal binding sites, nucleic acid sequences encoding drug resistance enzymes or amino acid metabolic enzymes, and nucleic acid sequences encoding secretion signals, localization signals, or signals useful for polypeptide purification.

A "replicon" is any genetic element, for example, a plasmid, cosmid, bacmid, plastid, phage or virus, that is capable of replication largely under its own control. A replicon may be either RNA or DNA and may be single or double stranded.

An "expression operon" refers to a nucleic acid segment that may possess transcriptional and translational control sequences, such as promoters, enhancers, translational start signals (e.g., ATG or AUG codons), polyadenylation signals, terminators, and the like, and which facilitate the expression of a polypeptide coding sequence in a host cell or organism.

As used herein, the terms "reporter," "reporter system", "reporter gene," or "reporter gene product" shall mean an operative genetic system in which a nucleic acid comprises a gene that encodes a product that when expressed produces a reporter signal that is a readily measurable, e.g., by biological assay, immunoassay, radio immunoassay, or by colorimetric, fluorogenic, chemiluminescent or other methods. The nucleic acid may be either RNA or DNA, linear or circular, single or double stranded, antisense or sense polarity, and is operatively linked to the necessary control elements for the expression of the reporter gene product. The required control elements will vary according to the nature of the reporter system and whether the reporter gene is in the form of DNA or RNA, but may include, but not be limited to, such elements as promoters, enhancers, translational control sequences, poly A addition signals, transcriptional termination signals and the like.

The introduced nucleic acid may or may not be integrated (covalently linked) into nucleic acid of the recipient cell or organism. In bacterial, yeast, plant and mammalian cells, for example, the introduced nucleic acid may be maintained as an episomal element or independent replicon such as a plasmid. Alternatively, the introduced nucleic acid may become integrated into the nucleic acid of the recipient cell or organism and be stably maintained in that cell or organism and further passed on or inherited to progeny cells or organisms of the recipient cell or organism. Finally, the introduced nucleic acid may exist in the recipient cell or host organism only transiently.

The term "selectable marker gene" refers to a gene that when expressed confers a selectable phenotype, such as antibiotic resistance, on a transformed cell.

The term "operably linked" means that the regulatory sequences necessary for expression of the coding sequence are placed in the DNA molecule in the appropriate positions relative to the coding sequence so as to effect expression of the coding sequence. This same definition is sometimes applied to the arrangement of transcription units and other transcription control elements (e.g. enhancers) in an expression vector.

The terms "recombinant organism," or "transgenic organism" refer to organisms which have a new combination of genes or nucleic acid molecules. A new combination of genes or nucleic acid molecules can be introduced into an organism using a wide array of nucleic acid manipulation techniques available to those skilled in the art. The term "organism" relates to any living being comprised of a least one cell. An organism can be as simple as one eukaryotic cell or as complex as a mammal. Therefore, the phrase "a recombinant organism" encompasses a recombinant cell, as well as eukaryotic and prokaryotic organism.

The term "isolated protein" or "isolated and purified protein" is sometimes used herein. This term refers primarily to a protein produced by expression of an isolated nucleic acid molecule of the invention. Alternatively, this term may refer to a protein that has been sufficiently separated from other proteins with which it would naturally be associated, so as to exist in "substantially pure" form. "Isolated" is not meant to exclude artificial or synthetic mixtures with other compounds or materials, or the presence of impurities that do not interfere with the fundamental activity, and that may be present, for example, due to incomplete purification, addition of stabilizers, or compounding into, for example, immunogenic preparations or pharmaceutically acceptable preparations.

A "specific binding pair" comprises a specific binding member (sbm) and a binding partner (bp) which have a particular specificity for each other and which in normal conditions bind to each other in preference to other molecules. Examples of specific binding pairs are antigens and antibodies, ligands and receptors and complementary nucleotide sequences. The skilled person is aware of many other examples. Further, the term "specific binding pair" is also applicable where either or both of the specific binding member and the binding partner comprise a part of a large molecule. In embodiments in which the specific binding pair comprises nucleic acid sequences, they will be of a length to hybridize to each other under conditions of the assay, preferably greater than 10 nucleotides long, more preferably greater than 15 or 20 nucleotides long.

"Sample" or "patient sample" or "biological sample" generally refers to a sample which may be tested for a particular molecule, preferably a neurological disorder specific marker molecule, such as a marker shown in the tables provided below. Samples may include but are not limited to cells, body fluids, including blood, serum, plasma, urine, saliva, tears, pleural fluid and the like.

The terms "agent" and "test compound" are used interchangeably herein and denote a chemical compound, a mixture of chemical compounds, a biological macromolecule, or an extract made from biological materials such as bacteria, plants, fungi, or animal (particularly mammalian) cells or tissues. Biological macromolecules include siRNA, shRNA, antisense oligonucleotides, peptides, peptide/DNA complexes, and any nucleic acid based molecule which exhibits the capacity to modulate the activity of the CNV containing nucleic acids described herein or their encoded proteins. Agents are evaluated for potential biological activity by inclusion in screening assays described hereinbelow.

## METHODS OF USING NEUROLOGICAL DISORDER-ASSOCIATED eCNVS FOR DIAGNOSING AN INCREASED RISK FOR THE DEVELOPMENT OF A NEUROLOGICAL DISORDER

Neurological disorder-related-eCNV containing nucleic acids, including but not limited to those listed in the Tables provided below may be used for a variety of purposes in accordance with the present invention Neurological disorder-associated eCNV containing DNA, RNA, or fragments thereof may be used as probes to detect the presence of and/or expression of neurological disorder specific markers. Methods in which neurological disorder specific marker nucleic acids may be utilized as probes for such assays include, but are not limited to: (1) *in situ* hybridization; (2) Southern hybridization (3) northern hybridization; and (4) assorted amplification reactions such as polymerase chain reactions (PCR).

Further, assays for detecting neurological disorder-associated eCNVs may be conducted on any type of biological sample, including but not limited to body fluids (including blood, CSF, urine, serum, gastric lavage), any type of cell (such as brain cells, white blood cells, mononuclear cells) or body tissue.

From the foregoing discussion, it can be seen that neurological disorder-associated eCNV containing nucleic acids, vectors expressing the same, neurological disorder eCNV containing marker proteins and anti-neurological disorder specific marker antibodies of the invention can be used to detect neurological disorder associated eCNVs in body tissue, cells, or fluid, and alter neurological disorder eCNV containing marker protein expression for purposes of assessing the genetic and protein interactions involved in the development of neurological disorder.

In most cases for screening for neurological disorder-associated CNVs, the neurological disorder-associated CNV containing nucleic acid in the sample will initially be amplified, e.g. using PCR, to increase the amount of the templates as compared to other sequences present in the sample. This allows the target sequences to be detected with a high degree of sensitivity if they are present in the sample. This initial step may be avoided by using highly sensitive array techniques that are becoming increasingly important in the art.

Alternatively, new detection technologies can overcome this limitation and enable analysis of small samples containing as little as 1 μg of total RNA. Using Resonance Light Scattering (RLS) technology, as opposed to traditional fluorescence techniques, multiple reads can detect low quantities of mRNAs using biotin labeled hybridized targets and anti-biotin antibodies. Another alternative to PCR amplification involves planar wave guide technology (PWG) to increase signal-to-noise ratios and reduce background interference. Both techniques are commercially available from Qiagen Inc. (USA).

Thus any of the aforementioned techniques may be used to detect or quantify neurological disorder-associated CNV marker expression and accordingly, diagnose neurological disorder(s).

## KITS AND ARTICLES OF MANUFACTURE

Any of the aforementioned products can be incorporated into a kit which may contain a neurological disorder-associated CNV specific marker polynucleotide or one or more such markers immobilized on a Gene Chip, an oligonucleotide, a polypeptide, a peptide, an antibody, a label, marker, or reporter, a pharmaceutically acceptable carrier, a physiologically acceptable carrier, instructions for use, a container, a vessel for administration, an assay substrate, enzyme, or any combination thereof.

## METHODS OF USING NEUROLOGICAL DISORDER-ASSOCIATED CNVs/SNPs DEVELOPMENT OF THERAPEUTIC AGENTS

Since the CNVs identified herein have been associated with the etiology of a neurological disorder, methods for identifying agents that modulate the activity of the genes and their encoded products containing such CNVs should result in the generation of efficacious therapeutic agents for the treatment of such conditions.

As can be seen from the data provided in the Tables below, several chromosomes contain regions which provide suitable targets for the rational design of therapeutic agents which modulate their activity. Specific organic molecules can thus be identified with capacity to bind to the active site of the proteins encoded by the CNV containing nucleic acids based on conformation or key amino acid residues required for function. A combinatorial chemistry approach will be used to identify molecules with greatest activity and then iterations of these molecules will be developed for further cycles of screening. In certain embodiments, candidate agents can be screening from large libraries of synthetic or natural compounds. Such compound libraries are commercially available from a number of companies including but not limited to Maybridge Chemical Co., (Trevillet,Cornwall, UK), Comgenex (Princeton, NJ), Microsour (New Milford, CT) Aldrich (Milwaukee, WI) Akos Consulting and Solutions GmbH (Basel, Switzerland), Ambinter (Paris, France), Asinex (Moscow, Russia) Aurora (Graz, Austria), BioFocus DPI (Switzerland), Bionet (Camelford, UK), Chembridge (San Diego, CA), Chem Div (San Diego, CA). The skilled person is aware of other sources and can readily purchase the same. Once therapeutically efficacious compounds are identified in the screening assays described herein, they can be formulated in to pharmaceutical compositions and utilized for the treatment of a neurological disorder.

The polypeptides or fragments employed in drug screening assays may either be free in solution, affixed to a solid support or within a cell. One method of drug screening utilizes eukaryotic or prokaryotic host cells which are stably transformed with recombinant polynucleotides expressing the polypeptide or fragment, preferably in competitive binding assays. Such cells, either in viable or fixed form, can be used for standard binding assays. One may determine, for example, formation of complexes between the polypeptide or fragment and the agent being tested, or examine the degree to which the formation of a complex between the polypeptide or fragment and a known substrate is interfered with by the agent being tested.

Another technique for drug screening provides high throughput screening for compounds having suitable binding affinity for the encoded polypeptides and is described in detail in Geysen, PCT published application WO 84/03564, published on Sep. 13, 1984. Briefly stated, large numbers of different, small peptide test compounds, such as those described above, are synthesized on a solid substrate, such as plastic pins or some other surface. The peptide test compounds are reacted with the target polypeptide and washed. Bound polypeptide is then detected by methods well known in the art.

A further technique for drug screening involves the use of host eukaryotic cell lines or cells (such as described above) which have a nonfunctional or altered neurological disorder associated gene. These host cell lines or cells are defective at the polypeptide level. The host cell lines or cells are grown in the presence of drug compound. The rate of neuronal signaling, ion release, or maintenance of neuronal cell morphology of the host cells is measured to determine if the compound is capable of regulating the same in the defective cells. Host cells contemplated for use in the present invention include but are not limited to bacterial cells, fungal cells, insect cells, and mammalian cells, particularly neuronal cells. The neurological disorder-associated CNV encoding DNA molecules may be introduced singly into such host cells or in combination to assess the phenotype of cells conferred by such expression. Methods for introducing DNA molecules are also well known to those of ordinary skill in the art. Such methods are set forth in Ausubel et al. eds., Current Protocols in Molecular Biology, John Wiley & Sons, NY, N.Y. 1995.

A wide variety of expression vectors are available that can be modified to express the novel DNA sequences of this invention. The specific vectors exemplified herein are merely illustrative, and are not intended to limit the scope of the invention. Expression methods are described by Sambrook et al. Molecular Cloning: A Laboratory Manual or Current Protocols in Molecular Biology 16.3-17.44 (1989). Expression methods in Saccharomyces are also described in Current Protocols in Molecular Biology (1989).

Suitable vectors for use in practicing the invention include prokaryotic vectors such as the pNH vectors (Stratagene Inc., 11099 N. Torrey Pines Rd., La Jolla, Calif. 92037), pET vectors (Novogen Inc., 565 Science Dr., Madison, Wis. 53711) and the pGEX vectors (Pharmacia LKB Biotechnology Inc., Piscataway, N.J. 08854). Examples of eukaryotic vectors useful in practicing the present invention include the vectors pRc/CMV, pRc/RSV, and pREP (Invitrogen, 11588 Sorrento Valley Rd., San Diego, Calif. 92121); pcDNA3.1/V5&His (Invitrogen); baculovirus vectors such as pVL1392, pVL1393, or pAC360 (Invitrogen); and yeast vectors such as YRP17, YIP5, and YEP24 (New England Biolabs, Beverly, Mass.), as well as pRS403 and pRS413 Stratagene Inc.); Picchia vectors such as pHIL-DI (Phillips Petroleum Co., Bartlesville, Okla. 74004); retroviral vectors such as PLNCX and pLPCX (Clontech); and adenoviral and adeno-associated viral vectors.

Promoters for use in expression vectors of this invention include promoters that are operable in prokaryotic or eukaryotic cells. Promoters that are operable in prokaryotic cells include lactose (lac) control elements, bacteriophage lambda (pL) control elements, arabinose control elements, tryptophan (trp) control elements, bacteriophage T7 control elements, and hybrids thereof. Promoters that are operable in eukaryotic cells include Epstein Barr virus promoters, adenovirus promoters, SV40 promoters, Rous Sarcoma Virus promoters, cytomegalovirus (CMV) promoters, baculovirus promoters such as AcMNPV polyhedrin promoter, Picchia promoters such as the alcohol oxidase promoter, and Saccharomyces promoters such as the gal4 inducible promoter and the PGK constitutive promoter, as well as neuronal-specific platelet-derived growth factor promoter (PDGF), the Thy-1 promoter, the hamster and mouse Prion promoter (MoPrP), and the Glial fibrillar acidic protein (GFAP) for the expression of transgenes in glial cells.

In addition, a vector of this disclosure may contain any one of a number of various markers facilitating the selection of a transformed host cell. Such markers include genes associated with temperature sensitivity, drug resistance, or enzymes associated with phenotypic characteristics of the host organisms.

Host cells expressing the neurological disorder-associated CNVs of the present disclosure or functional fragments thereof provide a system in which to screen potential compounds or agents for the ability to modulate the development of neurological disorder. Thus, in one case, the nucleic acid molecules of the invention may be used to create recombinant cell lines for use in assays to identify agents which modulate aspects of cellular metabolism associated with neuronal signaling and neuronal cell communication and structure. Also provided herein are methods to screen for compounds capable of modulating the function of proteins encoded by CNV containing nucleic acids.

Another approach entails the use of phage display libraries engineered to express fragment of the polypeptides encoded by the CNV containing nucleic acids on the phage surface. Such libraries are then contacted with a combinatorial chemical library under conditions wherein binding affinity between the expressed peptide and the components of the chemical library may be detected. US Patents 6,057,098 and 5,965,456 provide methods and apparatus for performing such assays.

The goal of rational drug design is to produce structural analogs of biologically active polypeptides of interest or of small molecules with which they interact (e.g., agonists, antagonists, inhibitors) in order to fashion drugs which are, for example, more active or stable forms of the polypeptide, or which, e.g., enhance or interfere with the function of a polypeptide in vivo. See, e.g., Hodgson, (1991) Bio/Technology 9:19-21. In one approach, discussed above, the three-dimensional structure of a protein of interest or, for example, of the protein-substrate complex, is solved by x-ray crystallography, by nuclear magnetic resonance, by computer modeling or most typically, by a combination of approaches. Less often, useful information regarding the structure of a polypeptide may be gained by modeling based on the structure of homologous proteins. An example of rational drug design is the development of HIV protease inhibitors (Erickson et al., (1990) Science 249:527-533). In addition, peptides may be analyzed by an alanine scan (Wells, (1991) Meth. Enzym. 202:390-411). In this technique, an amino acid residue is replaced by Ala, and its effect on the peptide's activity is determined. Each of the amino acid residues of the peptide is analyzed in this manner to determine the important regions of the peptide.

It is also possible to isolate a target-specific antibody, selected by a functional assay, and then to solve its crystal structure. In principle, this approach yields a pharmacore upon which subsequent drug design can be based.

One can bypass protein crystallography altogether by generating anti-idiotypic antibodies (anti-ids) to a functional, pharmacologically active antibody. As a mirror image of a mirror image, the binding site of the anti-ids would be expected to be an analog of the original molecule. The anti-id could then be used to identify and isolate peptides from banks of chemically or biologically produced banks of peptides. Selected peptides would then act as the pharmacore.

Thus, one may design drugs which have, e.g., improved polypeptide activity or stability or which act as inhibitors, agonists, antagonists, etc. of polypeptide activity. By virtue of the availability of CNV containing nucleic acid sequences described herein, sufficient amounts of the encoded polypeptide may be made available to perform such analytical studies as x-ray crystallography. In addition, the knowledge of the protein sequence provided herein will guide those employing computer modeling techniques in place of, or in addition to x-ray crystallography.

The availability of neurological disorder-associated CNV containing nucleic acids enables the production of strains of laboratory mice carrying the neurological disorder-associated CNVs of the invention. Transgenic mice expressing the neurological disorder-associated CNV of the invention provide a model system in which to examine the role of the protein encoded by the CNV containing nucleic acid in the development and progression towards neurological disorder(s). Methods of introducing transgenes in laboratory mice are known to those of skill in the art. Three common methods include: 1. integration of retroviral vectors encoding the foreign gene of interest into an early embryo; 2. injection of DNA into the pronucleus of a newly fertilized egg; and 3. the incorporation of genetically manipulated embryonic stem cells into an early embryo. Production of the transgenic mice described above will facilitate the molecular elucidation of the role that a target protein plays in various cellular metabolic and neuronal processes. Such mice provide an in vivo screening tool to study putative therapeutic drugs in a whole animal model and are encompassed by the present invention.

The term "animal" is used herein to include all vertebrate animals, except humans. It also includes an individual animal in all stages of development, including embryonic and fetal stages. A "transgenic animal" is any animal containing one or more cells bearing genetic information altered or received, directly or indirectly, by deliberate genetic manipulation at the subcellular level, such as by targeted recombination or microinjection or infection with recombinant virus. The term "transgenic animal" is not meant to encompass classical cross-breeding or in vitro fertilization, but rather is meant to encompass animals in which one or more cells are altered by or receive a recombinant DNA molecule. This molecule may be specifically targeted to a defined genetic locus, be randomly integrated within a chromosome, or it may be extrachromosomally replicating DNA. The term "germ cell line transgenic animal" refers to a transgenic animal in which the genetic alteration or genetic information was introduced into a germ line cell, thereby conferring the ability to transfer the genetic information to offspring. If such offspring, in fact, possess some or all of that alteration or genetic information, then they, too, are transgenic animals.

The alteration of genetic information may be foreign to the species of animal to which the recipient belongs, or foreign only to the particular individual recipient, or may be genetic information already possessed by the recipient. In the last case, the altered or introduced gene may be expressed differently than the native gene. Such altered or foreign genetic information would encompass the introduction of neurological disorder-associated CNV containing nucleotide sequences.

The DNA used for altering a target gene may be obtained by a wide variety of techniques that include, but are not limited to, isolation from genomic sources, preparation of cDNAs from isolated mRNA templates, direct synthesis, or a combination thereof.

A preferred type of target cell for transgene introduction is the embryonal stem cell (ES). ES cells may be obtained from pre-implantation embryos cultured in vitro (Evans et al., (1981) Nature 292:154-156; Bradley et al., (1984) Nature 309:255-258; Gossler et al., (1986) Proc. Natl. Acad. Sci. 83:9065-9069). Transgenes can be efficiently introduced into the ES cells by standard techniques such as DNA transfection or by retrovirus-mediated transduction. The resultant transformed ES cells can thereafter be combined with blastocysts from a non-human animal. The introduced ES cells thereafter colonize the embryo and contribute to the germ line of the resulting chimeric animal.

One approach to the problem of determining the contributions of individual genes and their expression products is to use isolated neurological disorder-associated CNV genes as insertional cassettes to selectively inactivate a wild-type gene in totipotent ES cells (such as those described above) and then generate transgenic mice. The use of gene-targeted ES cells in the generation of gene-targeted transgenic mice was described, and is reviewed elsewhere (Frohman et al., (1989) Cell 56:145-147; Bradley et al., (1992) Bio/Technology 10:534-539).

Techniques are available to inactivate or alter any genetic region to a mutation desired by using targeted homologous recombination to insert specific changes into chromosomal alleles. However, in comparison with homologous extrachromosomal recombination, which occurs at a frequency approaching 100%, homologous plasmid-chromosome recombination was originally reported to only be detected at frequencies between $10^{-6}$ and $10^{-3}$. Nonhomologous plasmid-chromosome interactions are more frequent occurring at levels $10^{5}$-fold to $10^{2}$ fold greater than comparable homologous insertion.

To overcome this low proportion of targeted recombination in murine ES cells, various strategies have been developed to detect or select rare homologous recombinants. One approach for detecting homologous alteration events uses the polymerase chain reaction (PCR) to screen pools of transformant cells for homologous insertion, followed by screening of individual clones. Alternatively, a positive genetic selection approach has been developed in which a marker gene is constructed which will only be active if homologous insertion occurs, allowing these recombinants to be selected directly. One of the most powerful approaches developed for selecting homologous recombinants is the positive-negative selection (PNS) method developed for genes for which no direct selection of the alteration exists. The PNS method is more efficient for targeting genes which are not expressed at high levels because the marker gene has its own promoter. Non-homologous recombinants are selected against by using the Herpes Simplex virus thymidine kinase (HSV-TK) gene and selecting against its nonhomologous insertion with effective herpes drugs such as gancyclovir (GANC) or (1-(2-deoxy-2-fluoro-B-D arabinofluranosyl)-5-iodou- racil, (FIAU). By this counter selection, the number of homologous recombinants in the surviving transformants can be increased. Utilizing neurological disorder-associated CNV containing nucleic acid as a targeted insertional cassette provides means to detect a successful insertion as visualized, for example, by acquisition of immunoreactivity to an antibody immunologically specific for the polypeptide encoded by neurological disorder-associated CNV nucleic acid and, therefore, facilitates screening/selection of ES cells with the desired genotype.

As used herein, a knock-in animal is one in which the endogenous murine gene, for example, has been replaced with human neurological disorder-associated CNV containing gene of the invention. Such knock-in animals provide an ideal model system for studying the development of neurological disorder(s).

As used herein, the expression of a neurological disorder-associated CNV containing nucleic acid, fragment thereof, or an neurological disorder-associated CNV fusion protein can be targeted in a "tissue specific manner" or "cell type specific manner" using a vector in which nucleic acid sequences encoding all or a portion of neurological disorder-associated CNV are operably linked to regulatory sequences (e.g., promoters and/or enhancers) that direct expression of the encoded protein in a particular tissue or cell type. Such regulatory elements may be used to advantage for both in vitro and in vivo applications. Promoters for directing tissue specific proteins are well known in the art and described herein.

The nucleic acid sequence encoding the neurological disorder-associated CNV of the invention may be operably linked to a variety of different promoter sequences for expression in transgenic animals. Such promoters include, but are not limited to a prion gene promoter such as hamster and mouse Prion promoter (MoPrP), described in U.S. Pat. No. 5,877,399 and in Borchelt et al., Genet. Anal. 13(6) (1996) pages 159-163; a rat neuronal specific enolase promoter, described in U.S. Pat. Nos. 5,612,486, and 5,387,742; a platelet-derived growth factor B gene promoter, described in U.S. Pat. No. 5,811,633; a brain specific dystrophin promoter, described in U.S. Pat. No. 5,849,999; a Thy-1 promoter; a PGK promoter; a CMV promoter; a neuronal-specific platelet-derived growth factor B gene promoter; and Glial fibrillar acidic protein (GFAP) promoter for the expression of transgenes in glial cells.

Methods of use for the transgenic mice of the disclosure are also provided herein. Transgenic mice into which a nucleic acid containing the neurological disorder-associated CNV or its encoded protein have been introduced are useful, for example, to develop screening methods to screen therapeutic agents to identify those capable of modulating the development of neurological disorder(s).

## PHARMACEUTICALS AND PEPTIDE THERAPIES

The elucidation of the role played by the neurological disorder associated CNVs described herein in neuronal signaling and brain structure facilitates the development of pharmaceutical compositions useful for treatment and diagnosis of neurological disorder(s). These compositions may comprise, in addition to one of the above substances, a pharmaceutically acceptable excipient, carrier, buffer, stabilizer or other materials well known to those skilled in the art. Such materials should be non-toxic and should not interfere with the efficacy of the active ingredient. The precise nature of the carrier or other material may depend on the route of administration, e.g. oral, intravenous, inhalation, cutaneous or subcutaneous, nasal, intramuscular, intraperitoneal routes.

Whether it is a polypeptide, antibody, peptide, nucleic acid molecule, small molecule or other pharmaceutically useful compound according to the present disclosure that is to be given to an individual, administration is preferably in a "prophylactically effective amount" or a "therapeutically effective amount" (as the case may be, although prophylaxis may be considered therapy), this being sufficient to show benefit to the individual.

The following materials and methods are provided to facilitate the practice of the present invention.

## Sample ascertainment

For initial screening we assembled three sample collections: 1) 943 ASD families (4,444 unique subjects) from the Autism Genetic Resource Exchange (AGRE) collection; 2) 1,070 de-identified and unrelated children of European ancestry from the Children's Hospital of Philadelphia (CHOP), with no evidence of neurological disorders; 3) 542 unrelated neurologically normal adults and seniors of European ancestry from the National Institute of Neurological Disorders and Stroke (NINDS) control collection. The AGRE families include 917 multiplex families, 24 simplex families and 2 families without an ASD diagnosis. For all analyses, AGRE cases annotated with "Autism" (n=1,463), "Broad Spectrum" (n=149) or "Not Quite Autism" (n=71) were treated equally and as affected. Samples from AGRE and NINDS were genotyped using DNA extracted from Epstein-Barr Virus (EBV)-transformed lymphoblastoid cell lines, while the CHOP controls were genotyped using DNA extracted from whole blood. All AGRE and control samples included in these analyses were genotyped on the Illumina HumanHap550 version 3 arrays, and 281 samples genotyped on version 1 arrays were excluded from the present analysis. Since the NINDS controls were genotyped at a different location and time, they were used to assess the frequency of specific CNVs in an independent cohort and to address concerns of cell line artifacts. This study was approved by the Institutional Review Board of Children's Hospital of Philadelphia. All subjects provided written informed consent for the collection of samples and subsequent analysis.

The Autism Case-Control (ACC) cohort included 859 cases from multiple sites within the United States, all of whom were of European ancestry affected with ASD. Of those, 703 were male and 156 were female; 828 met diagnostic criteria for autism, and 31 met criteria for other ASDs. Subjects ranged from 2-21 years of age when the Autism Diagnostic Interview (ADI) was given. Of the case subjects, 54% were from simplex families with the balance coming from multiplex families. The control group used for replication included 1051 children of self-reported Caucasian ancestry who had no history of ASDs. These controls were recruited by CHOP nursing and medical assistant staff under the direction of CHOP clinicians within the CHOP Health Care Network, including four primary care clinics and several group practices and outpatient practices that included well child visits.

**Detection and annotation of copy number variation**

For each data set, we applied identical and stringent quality control criteria to remove samples with low signal quality. CNV calls were generated using PennCNV [20], an algorithm which employs multiple sources of information, including total signal intensity, allelic intensity ratios, SNP allele frequencies, distance between neighboring SNPs, and family information to generate calls. We excluded samples meeting any of the following criteria: a) standard deviation for autosomal log R ratio values (LRR_SD) higher than 0.28, b) median B Allele Frequency (BAF_median) higher than 0.55 or lower than 0.45, c) fraction of markers with BAF values between 0.2 and 0.25 or 0.75 and 0.8 (BAF_drift) exceeded 0.002. We also excluded from our analysis CNVs within IGLC1 (22q11.22), IGHG1 (14q32.33) and IGKC (2p11.2), and the T cell receptor constant chain locus (14q11.2), as well as CNVs in chromosomes showing evidence of heterosomic aberrations (chromosome rearrangements in sub-populations of cells) in BeadStudio.

CNV calls were mapped onto genes by identifying overlap with RefSeq exons, the coordinates of which we obtained from the UCSC table browser. Deletion events overlapping with exons retrieved in this way were listed as eDels. eDups were defined as gains overlapping one or more coding exons and seen to be internal to the beginning and end of the corresponding transcript. Gains observed to encompass all exons for a given gene were annotated as gDups. P values for relative CNV burden in cases and controls were calculated at each locus by Fisher's exact test. To compare our CNV calls with other publications that have used AGRE families [10],[11],[21],[22], we examined published calls on the same individuals with the same AGRE identifiers. The CNV calls were retrieved from each corresponding publication. Quantitative PCR for CNV validationTaqMan primer/probe sets were designed to query random CNVs using FileBuilder 3.0 on the repeat-masked human genome (NCBI_36; March 2006 release; http://genome.ucsc.edu/). For each assay, 10 ng of genomic DNA was assayed in quadruplicate in 10-$\mu$L reactions containing 1 $\times$ final concentration TaqMan Universal Master Mix (ABI part number 4304437), and 200 nM of each primer and probe. Cycling was performed under default conditions in 384-well optical PCR plates on an ABI 7900 machine. Copy number was defined as 2-$\Delta\Delta$CT, where $\Delta$CT is the difference in threshold cycles for the sample in question normalized against an endogenous reference (RNAseP) and expressed relative to the average values obtained by three arbitrary control DNAs. A list of TaqMan probes against the 12 CNVs tested is included in Table 5.

**Phylogenetic Analysis**

Phylogenetic trees were estimated using the neighbor-joining algorithm, as implemented in PAUP 4.0, on an additive encoding of autosomal genotypes from one randomly selected child from 912 families.

**EXAMPLE I**

**Genome-wide analyses of exonic copy number variants in a family-based study point to novel autism susceptibility genes**

The Autism spectrum disorders (ASDs, MIM: 209850) are a heterogeneous group of childhood diseases characterized by abnormalities in social behavior and communication, as well as patterns of restricted and repetitive behaviors[1]. Twin studies have demonstrated much higher concordance rates of ASD in monozygotic twins (92%) than dizygotic twins (10%) [2,3] indicating a strong genetic basis for autism susceptibility. Although previous work has implicated numerous genomic regions of interest [4-8], the identification of specific genetic variants that contribute to ASD risk remains challenging.

Substantial progress towards the identification of genetic risk variants has come from recent characterization of structural variation (*i.e.,* copy number variation or CNV). For example, an initial report involving patients with syndromic autism characterized genomic variation using array comparative genomic hybridization (CGH) and identified large de novo CNVs in 28% of cases [9]. Similarly, subsequent work demonstrated that the frequency of de novo CNVs is higher in cases versus controls [7],[8]. CNV analyses have proven useful in the identification of regions that are potentially disease-related [8], [10]-[13] and have begun to be employed to advance the candidacy of individual genes, including NRXN1, CNTNAP2, and NHE9 [6], [14]-[16]. Recent work characterizing structural variation in cases and ethnically matched controls associating ubiquitin-pathway genes with autism with replicating this finding in the AGRE dataset is likewise notable [17], although family data was not reported here. Using the AGRE dataset as a discovery cohort, along with family information available for AGRE samples, we describe distinct and complementary analyses, prioritizing exonic events over CNVs in introns and intergenic intervals, which provide important new insights into the genetic architecture of the ASDs.

Towards the identification of additional genes and regions that may modulate disease risk, we have assembled a resource characterizing genome-wide structural variation from over nine hundred multiplex ASD families. Presented below are results from analyses contrasting events observed in cases and healthy ethnically matched controls, focusing on three classes of genic events: exonic deletions (eDels), exonic duplications (eDups), and whole gene duplication (gDups). Recovery of known ASD loci - together with the identification of novel regions harboring variants in multiple cases but no controls - supports the utility of this dataset. Consistent with enormous inter-individual variation, we further document a large number of events observed in only individual cases (Table 1). Importantly, all of these data have been made available to the scientific community pre-publication (on the world wide web at agre.org), greatly enhancing the utility of existing publicly accessible biomaterials and phenotype data. These data further highlight the extent of structural variation in both human and the ASDs and offer an important resource for hypothesis-generation and interrogation of individual loci.

To characterize structural variation in ASD multiplex families and unrelated controls, we typed individuals at 561,466 SNP markers using Illumina HumanHap550 version 3 arrays. After excluding samples that failed to meet QC thresholds (see Table 2), we obtained array data on 3832 individuals from 912 multiplex families enrolled in the Autism Genetic Resource Exchange (AGRE) [18], 1070 disease-free children from the Children's Hospital of Philadelphia (CHOP), and 418 neurologically normal adults and seniors from the National Institute of Neurological Disorders and Stroke (NINDS) control collection [19]. Using the PennCNV software [20], we detected CNVs with a mean size of 59.9 Kb and mean frequency of 24.3 events per individual (see Table 3). Sensitivity compares favorably with previous BAC array-based [9],[21] and SNP-based methods [8], in which mean resolution was observed to be in the range of Mbs and hundreds of Kbs, respectively.

As a first step towards validation of genotyping accuracy we examined the inheritance of CNVs in the AGRE cohort. Consistent with high quality, 96.2% of CNV calls made in children were also detected in a parent. To explore the issue of genotyping accuracy further, we generated CNV calls for an independently generated data set in which an overlapping set of 2,518 AGRE samples were genotyped using the Affymetrix 5.0 platform [11]. For CNVs (>500 kb) in known ASD regions (e.g. 15q11-13, 16p11.2, and 22q11.21; Table 4) [8],[11],[21],[22], we observed 100% correspondence between the two platforms for individuals genotyped on both platforms. For further confirmation of CNV calls, we compared de novo variants identified here to those highlighted in previous analyses of AGRE families. We identified all five de novo CNVs reported by Sebat et al [7], three of the five de novo CNVs reported by Szatmari et al [6], one de novo CNV within A2BP1 reported by Martin et al [23], and all five 16p11.2 de novo deletions reported by Weiss et al [11] and Kumar et al [10]. Of the two of thirteen de novo CNVs reported by Szatmari et al not detected as de novo in our study, one was very small (2 SNPs, 180 bp on 8p23.2), and the second clearly appears to be inherited (469 SNPs, 1.4 Mb on 17p12). Thus, our data are concordant with several other studies, and provide a more comprehensive picture of de novo CNVs in multiplex autism families. To further evaluate the quality of these data on another independent platform, we used Taqman to determine relative copy number at 12 previously unreported de novo CNVs identified in AGRE probands, confirming 11/12 loci (Figure 1 and Table 5). Together these results suggest that the CNVs calls we report are consistent and reliable.

We therefore undertook additional analyses to identify specific loci in which structural variants were enriched in cases versus controls. Because the majority of such variants were intronic or intergenic, we sought to prioritize CNVs most likely to interfere with the molecular function of specific genes. We first filtered CNV calls to include only exonic deletions (eDels) observed to overlap with a RefSeq gene. Overall, such eDels were observed at similar frequencies in AGRE cases, 1st degree relatives of AGRE cases, and unrelated controls (CHOP and NINDS cohorts), with an average of ~2 such variants per person (Table 3). To identify events related to the ASDs we then looked for genes harboring eDels in at least one case but no unrelated controls. Among the 284 genes that met this criteria (Table 1) we observed several known ASD or mental retardation genes including: ASPM [24], DPP10 [8], CNTNAP2 [25],[26], PCDH9 [16], and NRXN1[6].To enrich for genes most likely to contribute to ASD risk, we used family-based calling to evaluate which of these genes carried eDels in three or more cases from at least two unrelated families (Table 6). This stringent filtering resulted in 72 genes at 55 loci, including NRXN1. This is notable, given that eleven distinct disease-linked NRXN1 variants have been identified [6],[8],[15],[27],[28]. Neurexin family members are known to interact functionally with ASD-related neuroligins [29]-[32], and likewise play an important role in synaptic specification and specialization [33],[34]. eDels in more recently identified candidates, including DPP10 and PCDH9, were likewise retained. Similarly, recovery of RNF133 and RNF148 within intron 2 of CADPS2 [7],[35] highlights additional complexity at this locus. Although CNV breakpoints cannot be mapped precisely using SNP data alone, it is possible to determine overlap with protein coding exons and use these data to predict impact on gene function. Consistent with perturbation of function, distinct alleles at the loci highlighted here are predicted to eliminate or truncate the corresponding protein products (Figure 2).

Importantly, CNVs at a majority of these eDel loci show unique breakpoints in different families and/or result in the loss of distinct exons, demonstrating that they are independent. Moreover, because it is well established that CNVs at a subset of loci show identical breakpoints in unrelated individuals [10], this result is likely to underestimate the extent to which variants described here arose independently. Results from multi-dimensional scaling are likewise consistent with the interpretation that variants we highlight arose independently (Figure 3).

Given the large number of variants identified, it was critically important to confirm in an independent case-control analysis, how many of these eDels were truly overrepresented in cases, as opposed to being potentially attributable to Type I error. To address this concern, we sought to determine eDel frequency in these same genes in a replication dataset comprising 859 independently ascertained ASD cases and 1051 unrelated control subjects from the Autism Case Control cohort (ACC). One third of the loci identified in the discovery phase were observed in one or more ACC controls (18/55; 32.7%), suggesting that while rare, eDels at these loci are not limited to ASD cases and family members. In contrast, and providing evidence for formal replication, 14 separate loci encompassing 22 genes were observed to carry eDels in both AGRE and ACC cases, but none of 2539 controls (Table 3). Our replication data lend strong support to the involvement of specific loci in the ASDs (Table 7). However, to ensure that these results were not observed by chance alone, we performed 10,000 permutation trials on data from the replication cohort by permuting case/control status across individuals. In each permuted dataset, we maintained the same numbers of cases and controls as in the original data, and calculated the number of genes harboring CNVs exclusively in cases. None of the 10,000 permutation trials gave results comparable to experimental observations for replicated case-specific loci (n=14; p<0.0001; Figure 4A). In contrast, findings comparable to those for non-replicated loci (highlighted as case-specific in the discovery phase but subsequently seen in replication controls) were seen in controls in 246/10,000 trials (n=18; p=0.02; Figure 4B).

Despite the challenges associated with obtaining statistical support for individually rare events [7],[36] we next sought to assign P values for replicated eDel loci. We were able to obtain support for each of the following loci: BZRAP1 at 17q22 (p=$8.0\times10^{-4}$), NRXN1 at 2p16.3 (p=$3.3\times10^{-4}$), MDGA2 at 14q21.3 (p=$1.3\times10^{-4}$), MADCAM1 at 19q13 (p=$5.5\times10^{-5}$), and a three gene locus at 15q11 (p=$1.3\times10^{-11}$). CNV calls at each of 15q11 and 19p13 are highly-error prone, suggesting that results here be interpreted with caution (see footnotes C and F in Table 7). Recovery of NRXN1, however, provides confidence for involvement of additional loci that were likewise replicated. Benzodiazapine receptor (peripheral) associated protein 1 (BZRAP1, alternatively referred to as RIMBP1), is an adaptor molecule thought to regulate synaptic transmission by linking vesicular release machinery to voltage gated Ca2+ channels [37]. Identification of this synaptic component here, in a hypothesis-free manner, is particularly satisfying and also provides additional support for synaptic dysfunction in the ASDs [29],[38]. Less is known about MDGA2 [39], although comparison of the predicted protein to all others within GenBank by BLASTP indicated an unexpectedly high similarity to Contactin 4 (24% identity over more than 500 amino acids; Expect=3 × 10-39). Given previous reports of hemizygous loss of CNTN4 in individuals with mental retardation [40] and autism [17],[41]. similarity between MDGA2 and CNTN4, surpassed only by resemblance to MDGA1, is notable. Likewise intriguing in light of the suggestion that common variation in cell adhesion molecules may contribute to autism risk [42] is the structural likeness of MDGA2 to members of this family of molecules. Similar results were observed for three additional genes including the Chloride Channel, Kidney, A (*CLCNKA),* the Kainate-Preferring Glutamate Receptor Subunit KA2 *(GRIK5),* and Guanine Monophosphate synthetase *(GMPS)* (Figure 2); for each, eDels were identified in multiple unrelated cases, but not in any unaffected siblings or 1489 unrelated CHOP/NINDS controls (Figure 2). Moreover, for each of these genes, at least one CNV was observed to eliminate the entire protein coding sequence. Similarly, and also consistent with perturbation of function, separate alleles identified in unrelated individuals are predicted to result in dramatically truncated proteins.

Although some published analyses emphasize the greater contribution of gene deletion events in autism pathogenesis [7], there are also clear examples of duplications that strongly modulate ASD risk [43],[44]. We therefore conducted a parallel analysis of duplications, distinguishing between events involving entire genes (gDups) which might increase dosage and those restricted to internal exons (eDups) which could give rise to a frameshift or map to a chromosomal region distinct from the reference gene. For gDups, we identified 449 genes that were duplicated in at least one AGRE case but no CHOP/NINDS controls (Table 1). Of those, 200 genes at an estimated 63 loci, including genes at 15q11.2 [43], met the more stringent criteria of being present in three or more cases from at least two independent families (Table 6). Of these, 11.5% (23/200) were also seen in ACC controls, whereas 24.5% (49/200) were case-specific in the replication cohort. Strong statistical support was obtained for established loci (e.g. $p=9.3\times10^{-6}$ for UBE3A and other genes in the PWS/AS region at 15q11-q13), and nominal evidence was observed for the following novel loci: CD8A at 2p11.2 (p=0.069), LOC285498 at 4p16.3 (p=0.028), and CARD9/LOC728489 at 9q34.3 (p=0.005).

For eDups, we reasoned that duplication of one or more internal exons could serve to disrupt the corresponding open reading frame and be predicted to impair gene function as a result. Despite the caveat that observed copy number gains need not map to the wild-type locus, known ASD genes including TSC2 [45] and RAI1 [44],[46] within the Potocki-Lupski Syndrome critical interval were amongst the 159 loci observed in at least one AGRE case, but no CHOP/NINDS controls (Table 1). Such events were also seen in one family at the NLGN1 locus, which is of interest given previous support for NLGN3 and NLGN4 [29]. Filtering of these results, using the more stringent criteria employed above in consideration of eDels, limited this set of events to 76 loci observed in at least three cases from two separate families (Table 6). Interestingly, BZRAP1, reported above to harbor eDels at significantly higher frequencies in AGRE and ACC cases versus controls ($p=8.0\times10^{-4}$), was amongst these, with eDups observed here in four unrelated AGRE cases (screening p=0.021). Eight other genes, including the voltage gated potassium channel subunit KCNAB2 ($p=4.7\times10^{-3}$) remained absent from ACC controls and were also replicated in the independent case cohort. Although eDups at BZRAP1 were not detected in ACC cases, eDels at this locus were replicated, underscoring the importance of variation here. When considering eDels and eDups at the BZRAP1 locus together, the likelihood of such an observation occurring by chance alone is small ($p=2.3\times10^{-5}$). Although none of the variants we highlight were observed in any of 2539 unrelated controls, key events, including eDels at NRXN1, BZRAP1, and MDGA2 were observed in both cases and non-autistic family members (Figure 5). This is in keeping with previous work which suggests that haploinsufficiency at NRXN1 may contribute to the ASDs [15], but is insufficient to cause disease. Such data are also consistent with the well established finding of the "broader autism phenotype", such as subclinical language and social impairment in first degree relatives of cases with an ASD, which supports a multilocus model [47],[48]. We were also surprised to see that key variants at these loci appear to be transmitted to only a subset of affected individuals in some families (Figure 5). These observations parallel findings at other major effect loci including 16p11.2 [11] and DISC1 [49],[50] and are consistent with a model in which multiple variants, common and rare, act in concert to shape clinical presentation [51]-[53]. Results are also consistent with the idea that true risk loci are likely to show incomplete penetrance and imperfect segregation with disease [13], a reality that will complicate gene finding efforts. Related to this is that substantial effort will be required to determine whether rare alleles of moderate effect act independently on distinct aspects of disease (endophenotype model) or together to undermine key processes in brain development (threshold model).

By limiting CNV calls to include only exonic deletions (eDels) and duplications (eDups and gDups), we have attempted to enrich for variants most likely to impact gene function and in doing so improve the signal to noise ratio similar to work in other complex diseases [55]. At the same time, like other gene-based strategies, we preserve our ability to consider eDels involving the same transcriptional unit as separate but equivalent. Given that such events appear rare, this is an important consideration.

Pathway analysis by DAVID [56] found support for overrepresentation of cell adhesion molecules amongst recurrent eDel genes (uncorrected p=0.002; CDH17, PCDH9, LAMA2, MADCAM1, NRXN1, POSTN, SPON2), although it should be noted that this analysis does not adjust for gene size and may favor larger genes. Nevertheless, aside from SPON2 no eDels in these genes were observed in any of the controls interrogated. In contrast, no evidence for such overrepresentation was observed for genes in the ubiquitin degradation pathway and neither term was highlighted as overrepresented amongst eDups or gDups. Given that this study focused only on events encompassing RefSeq exons, differences from Glessner and colleagues [17] are to be expected.

In summary, we have performed a high resolution genome-wide analysis to characterize the genomic landscape of copy number variation in ASDs. Through comparison of structural variation in 1,771 ASD cases and 2,539 controls and prioritization of events encompassing exons we identified more than 150 loci harboring rare variants in multiple probands but no control individuals. For each class of structural variant interrogated, the recovery of known loci serves to validate the methods employed and results obtained. Greatest confidence should be placed in loci harboring variants in multiple unrelated cases but no controls and also recovered in both screening and replication cohorts. Amongst novel genes, best support was obtained for BZRAP1 and MDGA2, intriguing candidate genes which provide novel targets for the development of therapeutics useful for the treatment of ASDs.

**EXAMPLE II**

**SCREENING ASSAYS FOR IDENTIFYING EFFICACIOUS THERAPEUTICS FOR THE TREATMENT OF AUTISM AND ASD**

The information herein above can be applied clinically to patients for diagnosing an increased susceptibility for developing autism or autism spectrum disorder and therapeutic intervention. A preferred embodiment of the invention comprises clinical application of the information described herein to a patient. Diagnostic compositions, including microarrays, and methods can be designed to identify the genetic alterations described herein in nucleic acids from a patient to assess susceptibility for developing autism or ASD. This can occur after a patient arrives in the clinic; the patient has blood drawn, and using the diagnostic methods described herein, a clinician can detect a CNV as described in Example I. The information obtained from the patient sample, which can optionally be amplified prior to assessment, will be used to diagnose a patient with an increased or decreased susceptibility for developing autism or ASD. Kits for performing the diagnostic method of the invention are also provided herein. Such kits comprise a microarray comprising at least one of the SNPs provided herein in and the necessary reagents for assessing the patient samples as described above.

The identity of autism/ASD involved genes and the patient results will indicate which variants are present, and will identify those that possess an altered risk for developing ASD. The information provided herein allows for therapeutic intervention at earlier times in disease progression than previously possible. Also as described herein above, BZRAP1, and MDGA2 provide a novel targets for the development of new therapeutic agents efficacious for the treatment of this neurological disease.

REFERENCES

1. Abrahams BS, Geschwind DH (2008) Advances in autism genetics: on the threshold of a new neurobiology. Nat Rev Genet 9: 341-355.

2. Bailey A, Le Couteur A, Gottesman I, Bolton P, Simonoff E, et al. (1995) Autism as a strongly genetic disorder: evidence from a British twin study. Psychol Med 25: 63-77.

3. Steffenburg S, Gillberg C, Hellgren L, Andersson L, Gillberg IC, et al. (1989) A twin study of autism in Denmark, Finland, Iceland, Norway and Sweden. J Child Psychol Psychiatry 30: 405-416.

4. Cantor RM, Kono N, Duvall JA, Alvarez-Retuerto A, Stone JL, et al. (2005) Replication of autism linkage: fine-mapping peak at 17q21. Am J Hum Genet 76: 1050-1056.

5. Vorstman JA, Staal WG, van Daalen E, van Engeland H, Hochstenbach PF, et al. (2006) Identification of novel autism candidate regions through analysis of reported cytogenetic abnormalities associated with autism. Mol Psychiatry 11: 118-28.

6. Szatmari P, Paterson AD, Zwaigenbaum L, Roberts W, Brian J, et al. (2007) Mapping autism risk loci using genetic linkage and chromosomal rearrangements. Nat Genet 39: 319-328.

7. Sebat J, Lakshmi B, Malhotra D, Troge J, Lese-Martin C, et al. (2007) Strong association of de novo copy number mutations with autism. Science 316: 445-449.

8. Marshall CR, Noor A, Vincent JB, Lionel AC, Feuk L, et al. (2008) Structural variation of chromosomes in autism spectrum disorder. Am J Hum Genet 82:477-488.

9. Jacquemont ML, Sanlaville D, Redon R, Raoul O, Cormier-Daire V, et al.(2006) Array-based comparative genomic hybridisation identifies high frequency of cryptic chromosomal rearrangements in patients with syndromic autism spectrum disorders. J Med Genet 43: 843-849.

10. Kumar RA, Karamohamed S, Sudi J, Conrad DF, Brune C, et al. (2007) Recurrent 16p11.2 microdeletions in autism. Hum Mol Genet.

11. Weiss LA, Shen Y, Korn JM, Arking DE, Miller DT, et al. (2008) Association between Microdeletion and Microduplication at 16p11.2 and Autism. N Engl J Med.

12. Sharp AJ, Mefford HC, Li K, Baker C, Skinner C, et al. (2008) A recurrent 15q13.3 microdeletion syndrome associated with mental retardation and seizures. Nat Genet 40: 322-328.

13. Mefford HC, Sharp AJ, Baker C, Itsara A, Jiang Z, et al. (2008) Recurrent rearrangements of chromosome 1q21.1 and variable pediatric phenotypes. N Engl J Med 359: 1685-1699.

14. Alarcon M, Abrahams BS, Stone JL, Duvall JA, Perederiy JV, et al. (2008) Linkage, Association, and Gene-Expression Analyses Identify CNTNAP2 as an Autism-Susceptibility Gene. Am J Hum Genet 82: 150-159.

15. Kim HG, Kishikawa S, Higgins AW, Seong IS, Donovan DJ, et al. (2008) Disruption of neurexin 1 associated with autism spectrum disorder. Am J Hum Genet 82: 199-207.

16. Morrow EM, Yoo SY, Flavell SW, Kim TK, Lin Y, et al. (2008) Identifying autism loci and genes by tracing recent shared ancestry. Science 321: 218-223.

17. Glessner JT, Wang K, Cai G, Korvatska O, Kim CE, et al. (2009) Autism genome-wide copy number variation reveals ubiquitin and neuronal genes. Nature.

18. Geschwind DH, Sowinski J, Lord C, Iversen P, Shestack J, et al. (2001) The autism genetic resource exchange: a resource for the study of autism and related neuropsychiatric conditions. Am J Hum Genet 69: 463-466.

19. Nalls MA, Simon-Sanchez J, Gibbs JR, Paisan-Ruiz C, Bras JT, et al. (2009) Measures of autozygosity in decline: globalization, urbanization, and its implications for medical genetics. PLoS Genet 5: e1000415. doi:10.1371/joumal.pgen.1000415.

20. Wang K, Li M, Hadley D, Liu R, Glessner J, et al. (2007) PennCNV: an integrated hidden Markov model designed for high-resolution copy number variation detection in whole-genome SNP genotyping data. Genome Res 17: 1665-1674.

21. Christian SL, Brune CW, Sudi J, Kumar RA, Liu S, et al. (2008) Novel submicroscopic chromosomal abnormalities detected in autism spectrum disorder. Biol Psychiatry 63: 1111-1117.

22. Cai G, Edelmann L, Goldsmith JE, Cohen N, Nakamine A, et al. (2008) Multiplex ligation-dependent probe amplification for genetic screening in autism spectrum disorders: Efficient identification of known microduplications and identification of a novel microduplication in ASMT. BMC Med Genomics 1: 50.

23. Martin CL, Duvall JA, Ilkin Y, Simon JS, Arreaza MG, et al. (2007) Cytogenetic and molecular characterization of A2BP1/FOX1 as a candidate gene for autism. Am J Med Genet B Neuropsychiatr Genet 144: 869-876.

24. Bond J, Roberts E, Mochida GH, Hampshire DJ, Scott S, et al. (2002) ASPM is a major determinant of cerebral cortical size. Nat Genet 32: 316-320.

25. Bakkaloglu B, O'Roak BJ, Louvi A, Gupta AR, Abelson JF, et al. (2008) Molecular Cytogenetic Analysis and Resequencing of Contactin Associated Protein-Like 2 in Autism Spectrum Disorders. Am J Hum Genet 82: 165- 173.

26. Strauss KA, Puffenberger EG, Huentelman MJ, Gottlieb S, Dobrin SE, et al. (2006) Recessive symptomatic focal epilepsy and mutant contactin-associated protein-like 2. N Engl J Med 354: 1370-1377.

27. Feng J, Schroer R, Yan J, Song W, Yang C, et al. (2006) High frequency of neurexin lbeta signal peptide structural variants in patients with autism. Neurosci Lett 409: 10-13.

28. Yan J, Noltner K, Feng J, Li W, Schroer R, et al. (2008) Neurexin 1alpha structural variants associated with autism. Neurosci Lett 438: 368-370.

29. Jamain S, Quach H, Betancur C, Rastam M, Colineaux C, et al. (2003) Mutations of the X-linked genes encoding neuroligins NLGN3 and NLGN4 are associated with autism. Nat Genet 34: 27-29.

30. Comoletti D, De Jaco A, Jennings LL, Flynn RE, Gaietta G, et al. (2004) The Arg451Cys-neuroligin-3 mutation associated with autism reveals a defect in protein processing. J Neurosci 24: 4889-4893.

31. Laumonnier F, Bonnet-Brilhault F, Gomot M, Blanc R, David A, et al. (2004) X-linked mental retardation and autism are associated with a mutation in the NLGN4 gene, a member of the neuroligin family. Am J Hum Genet 74:552-557.

32. Yan J, Oliveira G, Coutinho A, Yang C, Feng J, et al. (2005) Analysis of the neuroligin 3 and 4 genes in autism and other neuropsychiatric patients. Mol Psychiatry 10: 329-332.

33. Scheiffele P, Fan J, Choih J, Fetter R, Serafini T (2000) Neuroligin expressed in nonneuronal cells triggers presynaptic development in contacting axons. Cell 101: 657-669.

34. Graf ER, Zhang X, Jin SX, Linhoff MW, Craig AM (2004) Neurexins induce differentiation of GABA and glutamate postsynaptic specializations via neuroligins. Cell 119: 1013-1026.

35. Sadakata T, Washida M, Iwayama Y, Shoji S, Sato Y, et al. (2007) Autistic-like phenotypes in Cadps2-knockout mice and aberrant CADPS2 splicing in autistic patients. J Clin Invest 117: 931-943.

36. Walsh T, McClellan JM, McCarthy SE, Addington AM, Pierce SB, et al. (2008) Rare structural variants disrupt multiple genes in neurodevelopmental pathways in schizophrenia. Science 320: 539-543.

37. Wang Y, Sugita S, Sudhof TC (2000) The RIM/NIM family of neuronal C2 domain proteins. Interactions with Rab3 and a new class of Src homology 3 domain proteins. J Biol Chem 275: 20033-20044.

38. Zoghbi HY (2003) Postnatal neurodevelopmental disorders: meeting at the synapse? Science 302: 826-830.

39. Litwack ED, Babey R, Buser R, Gesemann M, O'Leary DD (2004) Identification and characterization of two novel brain-derived immunoglobulin superfamily members with a unique structural organization. Mol Cell Neurosci 25: 263-274.

40. Fernandez T, Morgan T, Davis N, Klin A, Morris A, et al. (2004) Disruption of contactin 4 (CNTN4) results in developmental delay and other features of 3p deletion syndrome. Am J Hum Genet 74: 1286-1293.

41. Roohi J, Montagna C, Tegay DH, Palmer LE, DeVincent C, et al. (2009) Disruption of contactin 4 in three subjects with autism spectrum disorder. J Med Genet 46: 176-182.

42. Wang K, Zhang H, Ma D, Bucan M, Glessner JT, et al. (2009) Common genetic variants on 5p14.1 associate with autism spectrum disorders. Nature.

43. Cook EH Jr, Lindgren V, Leventhal BL, Courchesne R, Lincoln A, et al. (1997) Autism or atypical autism in maternally but not paternally derived proximal 15q duplication. Am J Hum Genet 60: 928-934.

44. Potocki L, Bi W, Treadwell-Deering D, Carvalho CM, Eifert A, et al. (2007) Characterization of Potocki-Lupski syndrome (dup(17)(p11.2p11.2)) and delineation of a dosage-sensitive critical interval that can convey an autism phenotype. Am J Hum Genet 80: 633-649.

45. Identification and characterization of the tuberous sclerosis gene on chromosome 16. Cell 75: 1305-1315.

46. Slager RE, Newton TL, Vlangos CN, Finucane B, Elsea SH (2003) Mutations in RAI1 associated with Smith-Magenis syndrome. Nat Genet 33: 466-468.

47. Bolton P, Macdonald H, Pickles A, Rios P, Goode S, et al. (1994) A case-control family history study of autism. J Child Psychol Psychiatry 35: 877-900.

48. Bishop DV, Maybery M, Maley A, Wong D, Hill W, et al. (2004) Using selfreport to identify the broad phenotype in parents of children with autistic spectrum disorders: a study using the Autism-Spectrum Quotient. J Child Psychol Psychiatry 45: 1431-1436.

49. Millar JK, Wilson-Annan JC, Anderson S, Christie S, Taylor MS, et al. (2000) Disruption of two novel genes by a translocation co-segregating with schizophrenia. Hum Mol Genet 9: 1415-1423.

50. Sachs NA, Sawa A, Holmes SE, Ross CA, DeLisi LE, et al. (2005) A frameshift mutation in Disrupted in Schizophrenia 1 in an American family with schizophrenia and schizoaffective disorder. Mol Psychiatry 10: 758-764.

51. Risch N, Spiker D, Lotspeich L, Nouri N, Hinds D, et al. (1999) A genomic screen of autism: evidence for a multilocus etiology. Am J Hum Genet 65: 493-507.

52. Rzhetsky A, Wajngurt D, Park N, Zheng T (2007) Probing genetic overlap among complex human phenotypes. Proc Natl Acad Sci U S A 104:11694-11699.

53. Bodmer W, Bonilla C (2008) Common and rare variants in multifactorial susceptibility to common diseases. Nat Genet 40: 695-701.

54. Yang S, Wang K, Gregory B, Berrettini W, Wang LS, et al. (2009) Genomic landscape of a three-generation pedigree segregating affective disorder. PLoS ONE 4: e4474. doi: 10.1371/journal.pone.0004474.

55. Ji W, Foo JN, O'Roak BJ, Zhao H, Larson MG, et al. (2008) Rare independent mutations in renal salt handling genes contribute to blood pressure variation. Nat Genet 40: 592-599.

56. Dennis G Jr, Sherman BT, Hosack DA, Yang J, Gao W, et al. (2003) DAVID: Database for Annotation, Visualization, and Integrated Discovery. Genome Biol 4: P3.

**Table 1**

| gene | class | AGRE. Cases. Unrelated | Screening. Controls | ACC. Cases | ACC. Controls | AGRE. Cases. Total | RE.Family.ID |
|---|---|---|---|---|---|---|---|
| ABCB9 | gdups | 3 | 0 | 0 | 0 | 5 | AU1378, AU1289, AU0899, AU0836, AU0688, AU0001 |
| ABCC1 | edups | 3 | 0 | 0 | 0 | 4 | AU1326, AU0301, AU1534 |
| ABHD8 | gdups | 2 | 0 | 0 | 0 | 2 | AU2005, AU1963, AU0806 |
| ACAT1 | gdups | 1 | 0 | 0 | 0 | 2 | AU0022 |
| ACP1 | edels | 1 | 0 | 0 | 0 | 2 | AU0385 |
| ACP6 | gdups | 3 | 0 | 3 | 0 | 4 | AU1688, AU1610, AU1163 |
| ACTRT1 | gdups | 1 | 0 | 0 | 0 | 2 | AU1764 |
| ACYP2 | edels | 2 | 0 | 0 | 1 | 2 | AU0930, AU0381 |
| ADAM10 | gdups | 1 | 0 | 0 | 0 | 2 | AU0467 |
| ADAM22 | edels | 1 | 0 | 0 | 0 | 2 | AU1221 |
| ADAMTS5 | gdups | 2 | 0 | 0 | 0 | 3 | AU1416, AU1227, AU0753, AU0158 |
| ADAMTS8 | edels | 2 | 0 | 0 | 0 | 2 | AU0939, AU0821 |
| ADAMTSL1 | edups | 2 | 0 | 0 | 0 | 3 | AU1496, AU0899, AU1594 |
| ADAMTSL2 | gdups | 2 | 0 | 0 | 0 | 2 | AU1273, AU0897, AU0520, AU1650, AU0806 |
| ADCK1 | gdups | 1 | 0 | 0 | 0 | 2 | AU0755 |
| ADCY1 | edups | 3 | 0 | 0 | 0 | 3 | AU1486, AU1331, AU1047, AU0828, AU0196, AU0168, AU0012 |
| ADCYAP1 | gdups | 1 | 0 | 0 | 0 | 2 | AU0827 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| ADM2 | gdups | 4 | 0 | 0 | 0 | 4 | AU1764, AU1212, AU1174, AU1164, AU0974, AU0899, AU0616, AU1944, AU1216 |
| ADPRHL1 | edels | 1 | 0 | 0 | 0 | 2 | AU1327 |
| AFMID | gdups | 2 | 0 | 0 | 0 | 2 | AU1174, AU0947, AU0991, AU0835 |
| AGL | edups | 2 | 0 | 0 | 0 | 2 | AU1833, AU0799, AU0771, AU0411 |
| AHCTF1 | edups | 1 | 0 | 0 | 0 | 2 | AU1668 |
| AHR | gdups | 2 | 0 | 0 | 1 | 3 | AU0440, AU0386 |
| AK7 | edels | 1 | 0 | 0 | 0 | 2 | AU0934 |
| AKR1B10 | edels | 2 | 0 | 0 | 0 | 2 | AU1145, AU0714 |
| AKT1S1 | gdups | 2 | 0 | 1 | 0 | 2 | AU1273, AU1072, AU0880, AU0698, AU0520, AU0068 |
| ALDH3B2 | gdups | 1 | 0 | 0 | 0 | 3 | AU1414, AU1228 |
| ALKBH1 | gdups | 1 | 0 | 0 | 0 | 2 | AU0755 |
| ALPK3 | edels | 1 | 0 | 0 | 0 | 3 | AU0561 |
| AMBP | gdups | 1 | 0 | 0 | 0 | 2 | AU0227 |
| ANGPTL4 | gdups | 2 | 0 | 0 | 0 | 2 | AU1592, AU1209, AU1189, AU1174, AU0899, AU0806 |
| ANKRD41 | gdups | 2 | 0 | 0 | 0 | 2 | AU2005, AU1963, AU0806 |
| ANTXR2 | edels | 2 | 0 | 0 | 11 | 2 | AU0753, AU0388, AU0114 |
| APBA3 | gdups | 3 | 0 | 0 | 0 | 3 | AU0520, AU0991, AU0866, AU0806 |
| APLP1 | gdups | 3 | 0 | 0 | 0 | 4 | AU1136, AU0599, AU0507 |
| APOBEC3C | gdups | 1 | 0 | 0 | 0 | 2 | AU1072, AU0550 |
| APOBEC3D | gdups | 1 | 0 | 0 | 0 | 2 | AU1072, AU0550 |
| APOBEC3F | gdups | 1 | 0 | 0 | 1 | 2 | AU1535, AU1072, AU0932, AU0550 |
| ARHGEF16 | edups | 3 | 0 | 0 | 0 | 5 | AU1830, AU1465, AU1412, AU1078 |
| ARHGEF4 | edels | 2 | 0 | 0 | 0 | 2 | AU1612, AU0991 |
| ARID3A | edups | 4 | 0 | 0 | 0 | 7 | AU0772, AU0725, AU0565, AU0308, AU0301, AU0139 |
| ARIH1 | edels | 1 | 0 | 0 | 1 | 2 | AU1309 |
| ARL11 | gdups | 4 | 0 | 0 | 0 | 5 | AU1056, AU0689, AU0325, AU0168, AU0055 |
| ARRB1 | edups | 2 | 0 | 0 | 1 | 2 | AU0430, AU0025, AU0556 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| ARRDC5 | gdups | 2 | 0 | 0 | 0 | 2 | AU1912, AU1742, AU1728, AU1273, AU1193, AU1190, AU1174, AU0007, AU1527, AU0481 |
| ARSA | gdups | 4 | 0 | 0 | 1 | 5 | AU0794, AU0772, AU0616, AU0145, AU0051 |
| ARSD | edels | 2 | 0 | 0 | 0 | 3 | AU1212, AU0338 |
| ARSD | gdups | 2 | 0 | 0 | 0 | 3 | AU1212, AU0338, AU1625, AU0361 |
| ARVCF | gdups | 4 | 0 | 4 | 0 | 4 | AU1189, AU1174, AU0018, AU0991, AU0688, AU0049 |
| ASCC3 | edups | 3 | 0 | 0 | 0 | 3 | AU0489, AU1533, AU0982, AU0301 |
| ASPM | edels | 2 | 0 | 0 | 1 | 2 | AU0662, AU0176, AU0102 |
| ATCAY | gdups | 3 | 0 | 0 | 0 | 3 | AU0520, AU0991, AU0866, AU0806 |
| ATP10A | gdups | 6 | 0 | 7 | 0 | 8 | AU1331, AU0106, AU0065, AU1135, AU0385, AU0233 |
| ATP11C | edels | 1 | 0 | 0 | 0 | 2 | AU1823 |
| ATP6VOD1 | edups | 1 | 0 | 0 | 0 | 2 | AU1482, AU0700 |
| ATP6VOD1 | gdups | 3 | 0 | 0 | 0 | 3 | AU1207, AU0962, AU1368, AU0991, AU0835 |
| AXUD1 | gdups | 1 | 0 | 0 | 0 | 2 | AU1424 |
| BAG2 | edels | 1 | 0 | 0 | 0 | 2 | AU1215 |
| BAI1 | edups | 2 | 0 | 0 | 0 | 2 | AU0616, AU0568 |
| BAIAP3 | edups | 1 | 0 | 0 | 0 | 2 | AU1172 |
| BBS2 | gdups | 1 | 0 | 0 | 0 | 2 | AU1197 |
| BC002942 | gdups | 5 | 0 | 0 | 0 | 6 | AU1912, AU1764, AU1212, AU1174, AU1164, AU1158, AU0899, AU0616, AU1944, AU1216, AU0806 |
| BCL9 | gdups | 3 | 0 | 3 | 0 | 4 | AU1688, AU1610, AU1163 |
| BCMO1 | gdups | 1 | 0 | 0 | 1 | 2 | AU1019, AU0708 |
| BDH1 | gdups | 1 | 0 | 0 | 0 | 2 | AU1407, AU1087, AU0477 |
| BGN | gdups | 2 | 0 | 0 | 0 | 2 | AU0947, AU0897, AU0562, AU0122, AU0866 |
| BIRC5 | gdups | 2 | 0 | 0 | 1 | 2 | AU1174, AU0947, AU0991, AU0835 |
| BIRC7 | edups | 2 | 0 | 0 | 0 | 2 | AU0106, AU1915 |

**23**

(continued)

| gene | class | AGRE. Cases. Unrelated | Screening. Controls | ACC. Cases | ACC. Controls | AGRE. Cases. Total | RE.Family.ID |
|------|-------|------|------|------|------|------|------|
| BLOC1S2 | edels | 1 | 0 | 0 | 0 | 2 | AU1016 |
| BMP2K | edups | 1 | 0 | 0 | 0 | 2 | AU1698 |
| BNIP2 | gdups | 1 | 0 | 0 | 0 | 2 | AU0467 |
| BTBD2 | edels | 1 | 0 | 0 | 1 | 2 | AU1806, AU1753 |
| BTBD4 | edups | 3 | 0 | 0 | 0 | 6 | AU1397, AU1273, AU0920, AU0307, AU0215 |
| BTBD4 | gdups | 2 | 0 | 0 | 0 | 2 | AU0939, AU0934, AU0543, AU0109 |
| BTN2A1 | edels | 2 | 0 | 0 | 1 | 4 | AU0561, AU0215 |
| BTN2A3 | edels | 1 | 0 | 0 | 1 | 3 | AU0561 |
| BTN3A3 | edels | 1 | 0 | 0 | 1 | 3 | AU0561 |
| BXDC1 | edels | 1 | 0 | 0 | 1 | 4 | AU0001 |
| BXDC1 | edups | 2 | 0 | 0 | 0 | 3 | AU1423, AU1341 |
| BZRAP1 | edels | 6 | 0 | 2 | 0 | 8 | AU1921, AU1286, AU1171, AU1105, AU0948, AU0897, AU0831, AU0803 |
| BZRAP1 | edups | 4 | 0 | 0 | 0 | 4 | AU1813, AU1341, AU1226, AU0899, AU0880, AU0616, AU0540, AU0085 |
| C10orf49 | edels | 1 | 0 | 0 | 0 | 2 | AU0305 |
| C10orf53 | gdups | 2 | 0 | 0 | 0 | 2 | AU0845, AU0329 |
| C10orf72 | edups | 9 | 0 | 0 | 1 | 12 | AU1282, AU1078, AU0994, AU0971, AU0952, AU0802, AU0698, AU0696, AU0616, AU0277, AU0134, AU0063, AU1691, AU1065 |
| C11orf72 | gdups | 3 | 0 | 0 | 0 | 5 | AU1414, AU1228, AU1527, AU0806, AU1007, AU0346, AU0152 |
| C12orf38 | gdups | 2 | 0 | 0 | 0 | 3 | AU0152 |
| C12orf49 | edels | 1 | 0 | 0 | 0 | 2 | AU1228 |
| C14orf151 | edups | 2 | 0 | 0 | 0 | 2 | AU0084, AU1315 |
| C14orf156 | gdups | 1 | 0 | 0 | 0 | 2 | AU0755 |
| C14orf173 | edups | 2 | 0 | 0 | 0 | 2 | AU0084, AU1315 |
| C15orf2 | gdups | 6 | 0 | 8 | 0 | 10 | AU1875, AU1331, AU0744, AU0106, AU0065, AU1135, AU0233 |
| C16orf30 | gdups | 2 | 0 | 0 | 0 | 2 | AU0932, AU0616, AU0008, AU0688 |
| C17orf58 | gdups | 1 | 0 | 0 | 0 | 2 | AU1685 |
| C19orf10 | gdups | 2 | 0 | 0 | 0 | 2 | AU1164, AU1099, AU0947, AU0616, AU0866 |
| C19orf15 | edels | 1 | 0 | 0 | 0 | 2 | AU1685 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| C19orf19 | edels | 3 | 0 | 7 | 0 | 3 | AU1286, AU1102, AU0995, AU1301, AU0194 |
| C19orf20 | edels | 2 | 0 | 7 | 0 | 2 | AU1286, AU1301, AU0194 |
| C19orf21 | edups | 1 | 0 | 0 | 0 | 2 | AU0022 |
| C1GALT1 | edels | 1 | 0 | 0 | 0 | 2 | AU0487 |
| C1orf101 | edels | 1 | 0 | 0 | 0 | 2 | AU0955 |
| C1orf171 | gdups | 1 | 0 | 0 | 0 | 2 | AU1285, AU0110 |
| C1orf192 | gdups | 2 | 0 | 0 | 0 | 2 | AU1875, AU1614 |
| C1orf93 | gdups | 4 | 0 | 0 | 0 | 4 | AU0934, AU0899, AU0880, AU0841, AU0816, AU0693, AU0520, AU0161, AU1409, AU0866, AU0481 |
| C1QTNF1 | edels | 8 | 0 | 0 | 6 | 11 | AU1779, AU1650, AU1338, AU1301, AU1292, AU1286, AU0951, AU0932, AU0598, AU1332, AU1107, AU0903, AU0803 |
| C20orf141 | gdups | 2 | 0 | 0 | 0 | 2 | AU1391, AU0758, AU0752, AU0509, AU0111, AU0049, AU0790 |
| C20orf151 | edels | 2 | 0 | 0 | 20 | 2 | AU1231, AU1318, AU0803 |
| C20orf72 | gdups | 1 | 0 | 0 | 0 | 2 | AU1520 |
| C21orf34 | edups | 1 | 0 | 0 | 0 | 2 | AU0799 |
| C21orf51 | gdups | 3 | 0 | 0 | 2 | 4 | AU1510, AU1233, AU1227, AU1213, AU0753, AU0747, AU0661, AU1213, AU0158 |
| C21orf70 | gdups | 2 | 0 | 0 | 0 | 2 | AU1227, AU1039, AU0753, AU0158 |
| C22orf25 | gdups | 3 | 0 | 4 | 0 | 3 | AU0520, AU0018, AU0991, AU0049 |
| C22orf29 | gdups | 3 | 0 | 4 | 0 | 3 | AU0018, AU0991, AU0049 |
| C6orf107 | gdups | 2 | 0 | 0 | 1 | 2 | AU1575, AU1412, AU0668 |
| C6orf213 | edels | 1 | 0 | 0 | 0 | 2 | AU0880 |
| C6orf65 | edels | 1 | 0 | 0 | 0 | 3 | AU1533 |
| C7orf20 | gdups | 1 | 0 | 0 | 0 | 2 | AU0385 |
| C7orf27 | gdups | 2 | 0 | 0 | 0 | 2 | AU0555, AU0806 |
| C8orf74 | edels | 1 | 0 | 0 | 0 | 2 | AU1171 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| C9orf28 | edups | 2 | 0 | 0 | 0 | 4 | AU1255, AU0780, AU0352 |
| C9orf48 | edels | 1 | 0 | 0 | 1 | 2 | AU0535 |
| C9orf7 | gdups | 2 | 0 | 0 | 0 | 2 | AU1273, AU0897, AU0520, AU1650, AU0806 |
| C9orf90 | gdups | 2 | 0 | 0 | 0 | 2 | AU1174, AU0866, AU0835 |
| CA5A | edups | 3 | 0 | 0 | 0 | 3 | AU0565, AU0308, AU0820 |
| CA6 | edels | 3 | 0 | 1 | 0 | 3 | AU1907, AU1226, AU0314 |
| CABLES2 | edels | 2 | 0 | 0 | 19 | 2 | AU1231, AU1318, AU0803 |
| CACHD1 | edups | 3 | 0 | 0 | 0 | 5 | AU0285, AU0029, AU1224 |
| CACNA2D2 | edups | 1 | 0 | 0 | 0 | 2 | AU1189, AU0178 |
| CACNA2D4 | edups | 5 | 0 | 0 | 0 | 5 | AU1551, AU1234, AU1072, AU0947, AU0263, AU0991 |
| CALB2 | gdups | 1 | 0 | 0 | 0 | 2 | AU1551 |
| CALCR | edels | 2 | 0 | 0 | 0 | 2 | AU1212, AU0049 |
| CAND2 | edels | 2 | 0 | 0 | 0 | 3 | AU1377, AU0025 |
| CARD11 | edups | 3 | 0 | 0 | 1 | 4 | AU1427, AU1328, AU1298 |
| CARD9 | gdups | 5 | 0 | 1 | 0 | 5 | AU1197, AU1072, AU0947, AU0934, AU0897, AU1283, AU1216, AU1033, AU0991, AU0068 |
| CASQ2 | gdups | 1 | 0 | 0 | 0 | 2 | AU0651 |
| CBLN3 | gdups | 4 | 0 | 0 | 0 | 5 | AU0980, AU0974, AU0742, AU0568, AU0551, AU0399 |
| CBR1 | gdups | 2 | 0 | 0 | 0 | 3 | AU1227, AU0753, AU0316, AU0158 |
| CCDC3 | edels | 1 | 0 | 0 | 0 | 2 | AU0305 |
| CCDC46 | edels | 2 | 0 | 0 | 1 | 2 | AU0742, AU0452, AU0121, AU0051 |
| CCDC65 | edups | 1 | 0 | 0 | 0 | 2 | AU1353 |
| CCDC67 | edels | 1 | 0 | 0 | 0 | 2 | AU1261 |
| CCDC94 | gdups | 2 | 0 | 0 | 0 | 2 | AU1164, AU1099, AU0947, AU0934, AU0991 |
| CCL1 | gdups | 1 | 0 | 0 | 1 | 2 | AU1559, AU0018 |
| CCL11 | gdups | 1 | 0 | 0 | 1 | 2 | AU1559, AU0018 |
| CCL13 | gdups | 2 | 0 | 0 | 3 | 3 | AU1559, AU0450, AU0018 |
| CCL14 | gdups | 1 | 0 | 0 | 0 | 2 | AU0806 |
| CCL15 | gdups | 1 | 0 | 0 | 0 | 2 | AU0806 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| CCL18 | gdups | 1 | 0 | 0 | 0 | 2 | AU0806 |
| CCL2 | gdups | 1 | 0 | 0 | 1 | 2 | AU1559, AU0018 |
| CCL23 | gdups | 1 | 0 | 0 | 0 | 2 | AU0806 |
| CCL3 | edels | 1 | 0 | 0 | 0 | 2 | AU1551 |
| CCL7 | gdups | 1 | 0 | 0 | 1 | 2 | AU1559, AU0018 |
| CCL8 | gdups | 1 | 0 | 0 | 1 | 2 | AU1559, AU0018 |
| CCNB2 | gdups | 1 | 0 | 0 | 0 | 2 | AU0467 |
| CCRK | edels | 1 | 0 | 0 | 0 | 2 | AU1516 |
| CD8A | gdups | 2 | 0 | 1 | 0 | 3 | AU1338, AU0600 |
| CDC42EP4 | edels | 1 | 0 | 0 | 0 | 2 | AU1921, AU1301 |
| CDC45L | gdups | 3 | 0 | 4 | 0 | 3 | AU0018, AU0991, AU0049 |
| CDC5L | edups | 1 | 0 | 0 | 0 | 3 | AU1867 |
| CDH17 | edels | 2 | 0 | 0 | 0 | 3 | AU0938, AU0355 |
| CDK5RAP2 | edups | 1 | 0 | 0 | 0 | 2 | AU0993, AU0076 |
| CDK9 | gdups | 2 | 0 | 0 | 0 | 2 | AU1174, AU1164, AU0932, AU0298, AU1650 |
| CDR1 | edels | 1 | 0 | 0 | 0 | 2 | AU1823 |
| CDRT15 | edels | 1 | 0 | 0 | 1 | 2 | AU0149 |
| CDRT15 | gdups | 1 | 0 | 0 | 0 | 2 | AU0707 |
| CDRT4 | edels | 1 | 0 | 0 | 1 | 2 | AU0149 |
| CDRT4 | gdups | 1 | 0 | 0 | 0 | 2 | AU0707 |
| CEBPA | gdups | 3 | 0 | 0 | 1 | 3 | AU1273, AU0934, AU0897, AU1963, AU1216, AU0991 |
| CEL | gdups | 2 | 0 | 0 | 0 | 2 | AU0698, AU0866 |
| CELSR1 | edups | 6 | 0 | 0 | 0 | 7 | AU1778, AU0688, AU0627, AU0540, AU0371, AU0307, AU1728, AU1527 |
| CENPT | gdups | 3 | 0 | 0 | 0 | 4 | AU0647, AU1368, AU1055 |
| CENTA1 | gdups | 1 | 0 | 0 | 0 | 2 | AU0385 |
| CERK | edups | 3 | 0 | 0 | 0 | 5 | AU0932, AU0816, AU0467, AU1610, AU0068 |
| CERK | gdups | 5 | 0 | 0 | 0 | 5 | AU1963, AU1527, AU1216, AU0806, AU0481 |
| CGB | gdups | 1 | 0 | 0 | 3 | 2 | AU1088 |
| CGB1 | gdups | 2 | 0 | 0 | 2 | 3 | AU1088, AU0698 |
| CGB2 | gdups | 2 | 0 | 0 | 2 | 3 | AU1088, AU0698 |
| CGB5 | gdups | 2 | 0 | 0 | 2 | 3 | AU1088, AU0698 |
| CGB8 | gdups | 2 | 0 | 0 | 2 | 3 | AU1088, AU0698 |
| CGI-38 | gdups | 3 | 0 | 0 | 0 | 3 | AU1207, AU0962, AU1368, AU0991, AU0835 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| CHD1L | gdups | 2 | 0 | 3 | 0 | 3 | AU1610, AU1163, AU1688 |
| CHD9 | edups | 2 | 0 | 1 | 0 | 3 | AU1558, AU1511 |
| CHIC2 | edels | 2 | 0 | 0 | 0 | 2 | AU0533, AU1333, AU0779 |
| CHODL | edels | 1 | 0 | 0 | 0 | 2 | AU0276 |
| CHRNA4 | edels | 1 | 0 | 0 | 0 | 2 | AU1921, AU0033 |
| CHRNA4 | gdups | 2 | 0 | 0 | 0 | 2 | AU1174, AU0932, AU0693, AU0678, AU0520, AU0991, AU0806 |
| CHRNG | gdups | 1 | 0 | 0 | 0 | 2 | AU1213, AU0520 |
| CHST3 | gdups | 1 | 0 | 0 | 0 | 2 | AU0862 |
| CLCN7 | edups | 1 | 0 | 0 | 0 | 2 | AU1990, AU1806 |
| CLCNKA | edels | 4 | 0 | 0 | 0 | 4 | AU1875, AU1048, AU0106, AU1944 |
| CLDN17 | gdups | 2 | 0 | 0 | 0 | 3 | AU1227, AU0816, AU0753, AU0158 |
| CLDN5 | gdups | 3 | 0 | 4 | 0 | 3 | AU0018, AU0991, AU0049 |
| CLDN6 | edels | 2 | 0 | 4 | 0 | 2 | AU1085, AU1338, AU1107 |
| CLDN8 | gdups | 2 | 0 | 0 | 0 | 3 | AU1227, AU0816, AU0753, AU0158 |
| CLDN9 | edels | 2 | 0 | 4 | 0 | 2 | AU1085, AU1338, AU1107 |
| CLEC2D | edups | 1 | 0 | 0 | 0 | 2 | AU1444 |
| CLEC4G | gdups | 1 | 0 | 0 | 0 | 2 | AU1730 |
| CLTCL1 | gdups | 3 | 0 | 4 | 0 | 3 | AU0018, AU0991, AU0049 |
| CNNM1 | edels | 1 | 0 | 0 | 0 | 2 | AU0325 |
| CNTNAP2 | edels | 2 | 0 | 0 | 0 | 2 | AU0880, AU0193 |
| COG4 | gdups | 1 | 0 | 0 | 0 | 2 | AU1551, AU0686 |
| COL16A1 | edups | 5 | 0 | 0 | 1 | 6 | AU1594, AU1158, AU0980, AU0763, AU0689, AU0258, AU0110, AU1283, AU0835 |
| COL20A1 | edels | 1 | 0 | 0 | 3 | 2 | AU1921, AU0033 |
| COL20A1 | edups | 1 | 0 | 0 | 0 | 2 | AU0325 |
| COL20A1 | gdups | 2 | 0 | 0 | 0 | 2 | AU1174, AU0932, AU0693, AU0678, AU0520, AU0991, AU0806 |
| COL22A1 | edups | 1 | 0 | 0 | 0 | 3 | AU0430 |
| COL27A1 | edels | 2 | 0 | 0 | 1 | 4 | AU1301, AU0489 |
| COL6A1 | edups | 2 | 0 | 0 | 0 | 2 | AU1764, AU0991 |
| COMT | gdups | 3 | 0 | 4 | 0 | 3 | AU0018, AU0991, AU0049 |
| COQ7 | gdups | 1 | 0 | 0 | 0 | 2 | AU0993 |

(continued)

| gene | class | AGRE. Cases. Unrelated | Screening. Controls | ACC. Cases | ACC. Controls | AGRE. Cases. Total | RE.Family.ID |
|------|-------|------------------------|---------------------|------------|---------------|--------------------|--------------|
| COR07 | gdups | 4 | 0 | 0 | 0 | 5 | AU1742, AU1212, AU1207, AU1174, AU1164, AU0971, AU0947, AU0899, AU0830, AU0688, AU0678, AU0081, AU1963 |
| COX10 | edels | 1 | 0 | 0 | 1 | 2 | AU0149 |
| COX411 | gdups | 3 | 0 | 0 | 0 | 4 | AU0920, AU0298, AU0179 |
| CPNE5 | edups | 2 | 0 | 0 | 0 | 2 | AU0905, AU1230 |
| CPNE7 | edups | 3 | 0 | 0 | 0 | 4 | AU1417, AU0795, AU0777, AU1921 |
| CPS1 | edels | 1 | 0 | 0 | 0 | 2 | AU0717 |
| CPXM2 | edups | 2 | 0 | 0 | 0 | 2 | AU1215, AU0053 |
| CREB3L3 | gdups | 9 | 0 | 0 | 0 | 9 | AU1099, AU0991, AU0947, AU0934, AU0911, AU0897, AU0241, AU0206, AU1527, AU0866, AU0742, AU0688, AU0676 |
| CRELD2 | gdups | 3 | 0 | 0 | 0 | 3 | AU1742, AU1368, AU1174, AU1055, AU0932, AU0916, AU0520, AU0991 |
| CRYGA | gdups | 1 | 0 | 0 | 0 | 2 | AU1060 |
| CRYZ | gdups | 1 | 0 | 0 | 0 | 2 | AU1285, AU0110 |
| CSAG2 | gdups | 1 | 0 | 0 | 0 | 2 | AU1439, AU0254 |
| CSAG3B | gdups | 1 | 0 | 0 | 0 | 2 | AU1439, AU0254 |
| CSTF2T | gdups | 2 | 0 | 0 | 0 | 3 | AU0640, AU0329 |
| CT45-5 | gdups | 2 | 0 | 0 | 0 | 2 | AU0542, AU0080 |
| CT45-6 | gdups | 2 | 0 | 0 | 0 | 2 | AU0542, AU0080 |
| CWF19L1 | edels | 1 | 0 | 0 | 0 | 2 | AU1016 |
| CXorf40A | gdups | 1 | 0 | 0 | 0 | 2 | AU0308 |
| CYB5R2 | gdups | 1 | 0 | 0 | 0 | 2 | AU 1309 |
| CYBASC3 | gdups | 2 | 0 | 0 | 0 | 3 | AU1323, AU0918 |
| CYP2B6 | edels | 2 | 0 | 0 | 1 | 2 | AU1486, AU1317 |
| CYP4A11 | gdups | 1 | 0 | 0 | 1 | 3 | AU0052 |
| CYP4A22 | gdups | 2 | 0 | 0 | 0 | 4 | AU1550, AU0052 |
| CYP4F22 | edels | 3 | 0 | 0 | 2 | 4 | AU1685, AU1411, AU1171, AU1157, AU1069 |
| CYP4X1 | gdups | 2 | 0 | 0 | 0 | 4 | AU1550, AU0052 |
| CYP4Z1 | gdups | 2 | 0 | 0 | 0 | 4 | AU1550, AU0052 |
| DAB1 | edels | 1 | 0 | 0 | 0 | 2 | AU0325 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| DACH1 | edels | 8 | 0 | 0 | 7 | 10 | AU0265, AU0250, AU0210, AU0208, AU0203, AU0179, AU0173, AU0108, AU0102, AU0098, AU0056, AU0820, AU0134, AU0123 |
| DACT2 | gdups | 1 | 0 | 0 | 0 | 2 | AU1409 |
| DAK | gdups | 4 | 0 | 0 | 0 | 6 | AU1728, AU1323, AU1215, AU1009, AU0995, AU0918, AU0658, AU0262 |
| DAPK3 | gdups | 7 | 0 | 0 | 0 | 7 | AU1273, AU1193, AU1189, AU1164, AU1137, AU1072, AU0947, AU0932, AU0520, AU1216, AU0991, AU0866, AU0806, AU0688 |
| DAZAP1 | gdups | 8 | 0 | 0 | 0 | 8 | AU1778, AU1632, AU1368, AU1353, AU1277, AU1212, AU1207, AU1174, AU1164, AU0974, AU0939, AU0934, AU0924, AU0899, AU0883, AU0795, AU0753, AU0081, AU1944, AU1527, AU1033, AU0866, AU0835 |
| DBH | edups | 2 | 0 | 0 | 0 | 3 | AU1695, AU1536, AU1289 |
| DCUN1D2 | edels | 1 | 0 | 0 | 0 | 2 | AU1327 |
| DDB1 | gdups | 3 | 0 | 0 | 0 | 4 | AU1728, AU1323, AU1215, AU1009, AU0995, AU0658, AU0262 |
| DDX19A | gdups | 1 | 0 | 0 | 0 | 2 | AU1551, AU1445, AU0686 |
| DDX19B | gdups | 1 | 0 | 0 | 0 | 2 | AU1551, AU1445, AU0686 |
| DEFB125 | gdups | 2 | 0 | 0 | 0 | 2 | AU1534, AU1322 |
| DGCR14 | gdups | 3 | 0 | 4 | 0 | 3 | AU0018, AU0991, AU0049 |
| DGCR2 | gdups | 3 | 0 | 4 | 0 | 3 | AU0018, AU0991, AU0049 |
| DGCR6L | gdups | 2 | 0 | 3 | 0 | 2 | AU0018, AU0049 |
| DGCR8 | gdups | 3 | 0 | 4 | 0 | 3 | AU0520, AU0018, AU0991, AU0049 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| DGKB | edels | 8 | 0 | 0 | 0 | 10 | AU1400, AU1185, AU1074, AU0953, AU0767, AU0688, AU0399, AU0178, AU0110, AU1368, AU0289 |
| DHTKD1 | gdups | 1 | 0 | 0 | 3 | 2 | AU0032 |
| DHX29 | edels | 3 | 0 | 0 | 1 | 3 | AU1823, AU1616, AU1054, AU0242 |
| DHX34 | edels | 1 | 0 | 0 | 1 | 2 | AU1685 |
| DID01 | edups | 2 | 0 | 0 | 0 | 2 | AU0506, AU0099 |
| DID01 | gdups | 4 | 0 | 0 | 0 | 4 | AU0467, AU0099, AU0835, AU0676, AU0210 |
| DKFZP686A10121 | edels | 2 | 0 | 0 | 3 | 3 | AU0049, AU0030 |
| DKFZP686E2158 | gdups | 1 | 0 | 0 | 0 | 2 | AU0293 |
| DLGAP1 | edels | 4 | 0 | 0 | 0 | 5 | AU1069, AU0952, AU0509, AU0453, AU0055, AU0052, AU0043 |
| DNAJC10 | edels | 2 | 0 | 0 | 1 | 2 | AU1798, AU0839, AU0134 |
| DNAJC15 | edups | 1 | 0 | 0 | 1 | 2 | AU0043 |
| DNAJC17 | edups | 7 | 0 | 0 | 0 | 13 | AU1587, AU1399, AU1377, AU1211, AU1178, AU0958, AU0165 |
| DOCK6 | gdups | 4 | 0 | 0 | 0 | 4 | AU1193, AU1174, AU1164, AU0962, AU0934, AU0899, AU0520, AU0122, AU0991, AU0796 |
| DOT1L | gdups | 2 | 0 | 0 | 0 | 2 | AU0991, AU0947, AU0298, AU0806, AU0676 |
| DPP10 | edels | 2 | 0 | 0 | 0 | 3 | AU0543, AU0465 |
| DSCR1 | gdups | 3 | 0 | 0 | 2 | 4 | AU1510, AU1233, AU1227, AU1213, AU0753, AU0747, AU0661, AU1213, AU0158 |
| DTX1 | edels | 1 | 0 | 0 | 1 | 2 | AU1171 |
| DTX2 | gdups | 2 | 0 | 0 | 2 | 2 | AU1632, AU0352, AU0314, AU0085 |
| DUSP13 | edups | 2 | 0 | 0 | 0 | 3 | AU1327, AU1289, AU0085 |
| DUSP7 | gdups | 1 | 0 | 0 | 0 | 2 | AU1190 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| E2F4 | gdups | 12 | 0 | 0 | 0 | 16 | AU0939, AU0899, AU0610, AU0509, AU0450, AU0210, AU0028, AU1368, AU0991, AU0835 |
| EBI3 | gdups | 2 | 0 | 0 | 0 | 2 | AU1164, AU1099, AU0947, AU0934, AU0991 |
| EDG5 | gdups | 3 | 0 | 0 | 0 | 3 | AU1764, AU1174, AU1164, AU1963, AU0796 |
| EDG6 | gdups | 2 | 0 | 0 | 0 | 2 | AU1277, AU1174, AU0947, AU0622, AU0081, AU1534 |
| EDG8 | gdups | 2 | 0 | 1 | 0 | 2 | AU1273, AU1174, AU1164, AU0701, AU0866 |
| EEF2 | gdups | 7 | 0 | 0 | 0 | 7 | AU1273, AU1193, AU1189, AU1164, AU1137, AU1072, AU0947, AU0932, AU0520, AU1216, AU0991, AU0866, AU0806, AU0688 |
| EEFSEC | gdups | 1 | 0 | 0 | 0 | 2 | AU0835 |
| EFHA2 | edels | 2 | 0 | 0 | 0 | 3 | AU1470, AU0477 |
| EFHB | edups | 1 | 0 | 0 | 0 | 2 | AU0897 |
| ELM03 | gdups | 12 | 0 | 0 | 0 | 16 | AU0939, AU0899, AU0610, AU0509, AU0450, AU0210, AU0028, AU1368, AU0991, AU0835 |
| ELP4 | edels | 2 | 0 | 0 | 7 | 3 | AU0905, AU0117, AU0290 |
| EPHA10 | edups | 1 | 0 | 0 | 0 | 2 | AU0616 |
| EPRS | edels | 2 | 0 | 0 | 0 | 3 | AU1344, AU0053 |
| EPX | edups | 1 | 0 | 0 | 0 | 2 | AU0257 |
| ERAS | gdups | 1 | 0 | 0 | 0 | 2 | AU1465 |
| ERGIC1 | edups | 4 | 0 | 0 | 0 | 5 | AU1368, AU1289, AU0899, AU0781 |
| ESPN | edels | 1 | 0 | 0 | 8 | 2 | AU1612, AU1301 |
| EYA4 | edups | 1 | 0 | 0 | 0 | 2 | AU1324 |
| F2RL2 | edels | 1 | 0 | 0 | 0 | 3 | AU0980, AU0301 |
| F9 | edels | 1 | 0 | 0 | 0 | 2 | AU1823 |
| FABP3 | edels | 1 | 0 | 0 | 0 | 3 | AU0700 |
| FAM102A | gdups | 2 | 0 | 0 | 0 | 2 | AU1174, AU0934, AU0866, AU0835 |
| FAM110C | edels | 1 | 0 | 0 | 0 | 2 | AU0385 |
| FAM18B2 | edels | 1 | 0 | 0 | 1 | 2 | AU0149 |
| FAM18B2 | gdups | 1 | 0 | 0 | 0 | 2 | AU0707 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| FAM19A1 | edels | 1 | 0 | 1 | 0 | 2 | AU0034 |
| FAM19A4 | edels | 1 | 0 | 1 | 0 | 2 | AU0034 |
| FAM20C | gdups | 1 | 0 | 0 | 0 | 2 | AU0385 |
| FAM21B | gdups | 2 | 0 | 0 | 1 | 2 | AU1713, AU0845, AU0329 |
| FAM43A | gdups | 1 | 0 | 0 | 0 | 2 | AU0568 |
| FAM81A | gdups | 1 | 0 | 0 | 0 | 2 | AU0467, AU0236 |
| FAM89B | edels | 3 | 0 | 1 | 0 | 4 | AU1520, AU1439, AU1102 |
| FANCL | edups | 1 | 0 | 0 | 0 | 2 | AU1299, AU1296 |
| FARSA | edups | 1 | 0 | 0 | 0 | 2 | AU0599 |
| FBXL8 | gdups | 1 | 0 | 0 | 0 | 2 | AU0962, AU0210 |
| FCER2 | gdups | 1 | 0 | 0 | 0 | 2 | AU1730 |
| FGF13 | edels | 1 | 0 | 0 | 0 | 2 | AU1823 |
| FGR | edels | 1 | 0 | 0 | 0 | 2 | AU1798 |
| FGR | gdups | 2 | 0 | 0 | 0 | 2 | AU0783, AU0450, AU0085, AU0043, AU0008, AU1284 |
| FHOD1 | gdups | 13 | 0 | 0 | 0 | 18 | AU0939, AU0899, AU0722, AU0610, AU0509, AU0450, AU0210, AU0028, AU1368, AU0991, AU0899, AU0835 |
| FKSG24 | gdups | 3 | 0 | 0 | 0 | 3 | AU1277, AU1174, AU0947, AU0520, AU0991, AU0866 |
| FLJ10379 | gdups | 2 | 0 | 0 | 2 | 3 | AU1067, AU0821, AU0705, AU0640 |
| FLJ11171 | gdups | 1 | 0 | 0 | 0 | 2 | AU1551 |
| FLJ11331 | edels | 1 | 0 | 0 | 6 | 2 | AU1275, AU0603, AU0556 |
| FLJ12529 | edups | 6 | 0 | 0 | 0 | 12 | AU1909, AU1640, AU1563, AU1327, AU1261, AU1072, AU0922, AU0199, AU0917 |
| FLJ12949 | edups | 9 | 0 | 0 | 0 | 13 | AU1406, AU1172, AU0920, AU0722, AU0689, AU0648, AU0629, AU0310, AU1187, AU1033, AU0806 |
| FLJ14668 | gdups | 3 | 0 | 0 | 0 | 4 | AU0722, AU0465, AU0149, AU0051 |
| FLJ20323 | edels | 1 | 0 | 0 | 0 | 2 | AU1105, AU0150 |
| FLJ20487 | gdups | 1 | 0 | 0 | 0 | 2 | AU0786 |
| FLJ21865 | edels | 3 | 0 | 0 | 9 | 3 | AU1779, AU1650, AU1286, AU1240, AU1332, AU0803 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| FLJ22671 | edups | 1 | 0 | 0 | 0 | 2 | AU1244 |
| FLJ22688 | gdups | 2 | 0 | 1 | 0 | 2 | AU1273, AU1072, AU0980, AU0880, AU0830, AU0753, AU0520, AU0068 |
| FLJ25416 | edels | 1 | 0 | 0 | 0 | 2 | AU1523, AU0246 |
| FLJ25976 | gdups | 1 | 0 | 0 | 0 | 2 | AU0755 |
| FLJ37440 | edups | 1 | 0 | 0 | 0 | 3 | AU1536, AU1163 |
| FLJ38991 | gdups | 3 | 0 | 0 | 0 | 5 | AU1462, AU1039, AU0551 |
| FLJ41603 | edups | 3 | 0 | 0 | 0 | 5 | AU1652, AU1486, AU1443, AU1389, AU0028 |
| FLJ41993 | gdups | 3 | 0 | 0 | 0 | 3 | AU1742, AU1368, AU1174, AU1055, AU0932, AU0916, AU0520, AU0991 |
| FLJ43860 | edups | 3 | 0 | 0 | 0 | 3 | AU1695, AU1374, AU1342, AU1102, AU0903, AU0763 |
| FLJ44815 | gdups | 1 | 0 | 0 | 1 | 2 | AU1559, AU0018 |
| FLJ44894 | edels | 2 | 0 | 0 | 7 | 4 | AU1559, AU1266, AU0686, AU0493, AU0208, AU0030 |
| FLJ45831 | edels | 1 | 0 | 0 | 1 | 2 | AU0149 |
| FLJ45831 | gdups | 1 | 0 | 0 | 0 | 2 | AU0707 |
| FLJ45850 | gdups | 2 | 0 | 0 | 0 | 2 | AU1174, AU0934, AU0816, AU1527, AU0481 |
| FLRT1 | gdups | 6 | 0 | 0 | 0 | 6 | AU0962, AU0947, AU0934, AU1164, AU1033, AU0866, AU0835, AU0806, AU0796 |
| FLYWCH1 | edels | 3 | 0 | 3 | 0 | 3 | AU1601, AU1338, AU1107 |
| FMO5 | gdups | 2 | 0 | 3 | 0 | 3 | AU1688, AU1610, AU1163 |
| FRMD3 | edups | 2 | 0 | 0 | 0 | 2 | AU1043, AU0897 |
| FRMD4A | edels | 1 | 0 | 0 | 0 | 2 | AU0241 |
| FRMD4A | edups | 1 | 0 | 0 | 0 | 2 | AU1806 |
| FUK | gdups | 1 | 0 | 0 | 0 | 2 | AU1551, AU0686 |
| FUT10 | edups | 3 | 0 | 0 | 0 | 4 | AU0788, AU0752, AU0618, AU0800 |
| GABRA5 | gdups | 5 | 0 | 7 | 0 | 8 | AU1331, AU0106, AU0065, AU1135, AU0233 |
| GABRB3 | gdups | 6 | 0 | 8 | 0 | 9 | AU1331, AU0106, AU0065, AU1135, AU0385, AU0233 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| GABRG3 | gdups | 5 | 0 | 6 | 0 | 8 | AU1331, AU0106, AU0065, AU1135, AU0233 |
| GAK | gdups | 2 | 0 | 1 | 0 | 2 | AU1164, AU0947, AU0806 |
| GALNT13 | edels | 4 | 0 | 1 | 0 | 6 | AU1509, AU1185, AU0781, AU0692, AU0653, AU0481, AU0481, AU0125 |
| GAMT | gdups | 8 | 0 | 0 | 0 | 8 | AU1778, AU1632, AU1368, AU1353, AU1277, AU1212, AU1207, AU1174, AU1164, AU0974, AU0939, AU0934, AU0924, AU0899, AU0883, AU0795, AU0753, AU0081, AU1944, AU1527, AU1368, AU1212, AU1164, AU1033, AU0866, AU0835 |
| GATA1 | gdups | 1 | 0 | 0 | 0 | 2 | AU1465 |
| GBGT1 | gdups | 2 | 0 | 0 | 0 | 2 | AU1164, AU0916, AU0899, AU0520, AU1368 |
| GCNT3 | gdups | 1 | 0 | 0 | 0 | 2 | AU0467 |
| GDPD4 | edups | 1 | 0 | 0 | 0 | 2 | AU1525 |
| GEMIN4 | edups | 2 | 0 | 0 | 0 | 3 | AU0662, AU0164 |
| GGN | edels | 1 | 0 | 0 | 0 | 2 | AU1685 |
| GGN | gdups | 4 | 0 | 0 | 0 | 5 | AU1685, AU1273, AU0680, AU0618, AU0379, AU0325, AU0001, AU0796 |
| GIMAP4 | edels | 2 | 0 | 0 | 0 | 2 | AU0257, AU0162 |
| GIMAP6 | edels | 2 | 0 | 0 | 0 | 2 | AU0257, AU0162 |
| GJA8 | gdups | 3 | 0 | 1 | 0 | 4 | AU1688, AU1610, AU1163 |
| GLT25D1 | edups | 1 | 0 | 0 | 0 | 2 | AU1429, AU1075 |
| GMPS | edels | 3 | 0 | 0 | 0 | 4 | AU1221, AU0714, AU0825 |
| GNA11 | gdups | 6 | 0 | 0 | 0 | 6 | AU2005, AU1652, AU1174, AU1164, AU1072, AU0081, AU1963, AU1944, AU0481 |
| GNB1L | gdups | 3 | 0 | 4 | 0 | 3 | AU0018, AU0991, AU0049 |
| GNG7 | edups | 2 | 0 | 1 | 0 | 3 | AU1632, AU0289, AU0109, AU0796 |
| GOLGA8B | gdups | 1 | 0 | 0 | 0 | 2 | AU1031, AU0656 |

(continued)

| gene | class | AGRE. Cases. Unrelated | Screening. Controls | ACC. Cases | ACC. Controls | AGRE. Cases. Total | RE.Family.ID |
|------|-------|------------|----------|----------|----------|------------|--------------|
| GOLGA8E | gdups | 2 | 0 | 0 | 0 | 3 | AU1331, AU0106 |
| GPR146 | gdups | 2 | 0 | 0 | 1 | 3 | AU1212, AU1174, AU0385, AU0806 |
| GPR17 | gdups | 1 | 0 | 0 | 0 | 2 | AU1327, AU1174, AU1099, AU0920, AU0836 |
| GPR30 | gdups | 1 | 0 | 0 | 0 | 2 | AU1174, AU0385 |
| GPR89A | gdups | 3 | 0 | 1 | 0 | 4 | AU1688, AU1610, AU1163 |
| GRIK5 | edels | 3 | 0 | 0 | 5 | 4 | AU0862, AU1305, AU1286 |
| GRIP2 | edups | 2 | 0 | 0 | 0 | 2 | AU1190, AU1072 |
| GSCL | gdups | 3 | 0 | 4 | 0 | 3 | AU0018, AU0991, AU0049 |
| GSTP1 | gdups | 1 | 0 | 0 | 0 | 3 | AU1414, AU1228 |
| GTF2A2 | gdups | 1 | 0 | 0 | 0 | 2 | AU0467 |
| GUCA1C | edups | 1 | 0 | 0 | 0 | 2 | AU0238 |
| GYG2 | edels | 2 | 0 | 0 | 0 | 4 | AU1059, AU0338 |
| GYG2 | gdups | 2 | 0 | 0 | 0 | 3 | AU1212, AU0338, AU1625, AU0361 |
| GYPE | edels | 1 | 0 | 0 | 1 | 2 | AU1822 |
| HAMP | edels | 1 | 0 | 0 | 0 | 2 | AU1553, AU1301 |
| HCFC1R1 | edels | 2 | 0 | 1 | 0 | 2 | AU1085, AU1338, AU1107 |
| HDAC6 | gdups | 1 | 0 | 0 | 0 | 2 | AU1465 |
| HDCMA18P | edels | 1 | 0 | 0 | 4 | 2 | AU1275, AU0603, AU0556 |
| HEATR2 | gdups | 1 | 0 | 0 | 0 | 2 | AU0385 |
| HECA | edups | 1 | 0 | 0 | 0 | 3 | AU1830 |
| HES7 | gdups | 11 | 0 | 0 | 0 | 17 | AU0991, AU0947, AU0939, AU0883, AU0880, AU0616, AU0520, AU0399, AU1283, AU0250, AU0169 |
| HEXA | edels | 1 | 0 | 0 | 0 | 2 | AU1427 |
| HFM1 | edels | 2 | 0 | 0 | 0 | 2 | AU0991, AU1619 |
| HIRA | gdups | 3 | 0 | 4 | 0 | 3 | AU0018, AU0991, AU0049 |
| HM13 | gdups | 2 | 0 | 0 | 0 | 2 | AU1694, AU0952 |
| HMGN3 | edels | 1 | 0 | 0 | 0 | 2 | AU1009 |
| HNF4G | edels | 2 | 0 | 0 | 0 | 3 | AU0745, AU0604, AU1212 |
| HOXA1 | gdups | 1 | 0 | 0 | 0 | 2 | AU0325 |
| HOXA2 | gdups | 1 | 0 | 0 | 0 | 2 | AU0325 |
| HPCAL1 | edups | 4 | 0 | 1 | 0 | 4 | AU1138, AU1055, AU0830, AU0773, AU0161, AU0102, AU0866, AU0134 |
| HRC | gdups | 2 | 0 | 0 | 2 | 2 | AU0919, AU0319 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| HS3ST3B1 | edels | 1 | 0 | 0 | 1 | 2 | AU0149 |
| HS3ST3B1 | gdups | 1 | 0 | 0 | 0 | 2 | AU0707 |
| HS6ST3 | edels | 1 | 0 | 0 | 1 | 2 | AU0700 |
| HSD11B2 | gdups | 3 | 0 | 0 | 0 | 3 | AU1207, AU0962, AU1368, AU0991, AU0835 |
| HSD3B2 | gdups | 2 | 0 | 0 | 1 | 2 | AU0875, AU1332 |
| HSF4 | gdups | 1 | 0 | 0 | 0 | 2 | AU0962, AU0210 |
| HSPC171 | gdups | 13 | 0 | 0 | 0 | 18 | AU0939, AU0899, AU0722, AU0610, AU0509, AU0450, AU0210, AU0028, AU1368, AU0991, AU0835 |
| HTF9C | gdups | 3 | 0 | 4 | 0 | 3 | AU0520, AU0018, AU0991, AU0049 |
| HYDIN | gdups | 1 | 0 | 0 | 0 | 2 | AU1551 |
| IAPP | edels | 2 | 0 | 0 | 1 | 2 | AU1368, AU1098, AU0753, AU0653 |
| IFI30 | gdups | 4 | 0 | 0 | 0 | 4 | AU1277, AU1174, AU0947, AU0520, AU0991, AU0866, AU0795 |
| INHBB | gdups | 3 | 0 | 0 | 0 | 3 | AU0257, AU1527, AU1164 |
| INSRR | edels | 1 | 0 | 0 | 0 | 2 | AU1171 |
| IQCE | gdups | 2 | 0 | 0 | 0 | 2 | AU0555, AU0806A |
| IQGAP2 | edels | 1 | 0 | 0 | 0 | 3 | AU0980, AU0301 |
| ITGA2 | edups | 1 | 0 | 0 | 0 | 2 | AU1764 |
| ITGAE | edels | 1 | 0 | 0 | 2 | 2 | AU1334, AU0246, AU0204 |
| ITGB1BP3 | gdups | 7 | 0 | 0 | 0 | 7 | AU1273, AU1193, AU1189, AU1164, AU1137, AU1072, AU0947, AU0932, AU0520, AU1216, AU0991, AU0866, AU0806, AU0688 |
| ITGB2 | gdups | 2 | 0 | 0 | 0 | 2 | AU1227, AU1039, AU0753, AU0158 |
| ITK | edups | 1 | 0 | 0 | 0 | 2 | AU0717 |
| KATNAL1 | edups | 1 | 0 | 0 | 0 | 2 | AU1551 |
| KCNAB2 | edups | 5 | 0 | 1 | 0 | 7 | AU0752, AU0722, AU0356, AU0017, AU1048 |
| KCND1 | gdups | 1 | 0 | 0 | 0 | 2 | AU1465, AU0346, AU0048 |

(continued)

| gene | class | AGRE. Cases. Unrelated | Screening. Controls | ACC. Cases | ACC. Controls | AGRE. Cases. Total | RE.Family.ID |
|------|-------|------------------------|---------------------|------------|---------------|--------------------|--------------|
| KCNE1 | gdups | 3 | 0 | 0 | 2 | 4 | AU1510, AU1233, AU1227, AU1213, AU0753, AU0747, AU0661, AU1213, AU0158 |
| KCNE2 | gdups | 3 | 0 | 0 | 2 | 4 | AU1510, AU1233, AU1227, AU1213, AU0753, AU0747, AU0661, AU0158 |
| KCNH7 | edups | 2 | 0 | 0 | 0 | 4 | AU1813, AU1492, AU1060, AU1492 |
| KCNJ14 | gdups | 3 | 0 | 0 | 0 | 3 | AU1610, AU1527, AU0795 |
| KCNQ1 | edels | 3 | 0 | 0 | 2 | 3 | AU1171, AU1157, AU0753, AU0276, AU1105 |
| KCNQ2 | gdups | 2 | 0 | 0 | 0 | 2 | AU1174, AU0932, AU0693, AU0678, AU0520, AU0991, AU0806 |
| KCTD19 | gdups | 3 | 0 | 0 | 0 | 3 | AU1207, AU0962, AU1368, AU0991, AU0835 |
| KCTD5 | edups | 5 | 0 | 0 | 0 | 7 | AU1620, AU1190, AU0938, AU0419, AU0307, AU0145 |
| KEAP1 | gdups | 2 | 0 | 1 | 0 | 2 | AU1273, AU1174, AU1164, AU0701, AU1164, AU0866 |
| KHDRBS2 | edels | 2 | 0 | 0 | 0 | 2 | AU0385, AU0308, AU0063, AU0538 |
| KIAA0195 | gdups | 9 | 0 | 0 | 0 | 9 | AU0897, AU0852, AU0836, AU0725, AU0712, AU0662, AU0340, AU0206, AU1283, AU0481 |
| KIAA0284 | gdups | 2 | 0 | 0 | 0 | 2 | AU0008, AU1055 |
| KIAA0319 | edups | 5 | 0 | 0 | 0 | 8 | AU1944, AU1798, AU1396, AU1137, AU0995, AU0933, AU0207 |
| KIAA0528 | edels | 2 | 0 | 0 | 4 | 3 | AU1533, AU0729, AU0399, AU0196 |
| KIAA0556 | edups | 1 | 0 | 0 | 0 | 2 | AU1953, AU0540 |
| KIAA0701 | edels | 1 | 0 | 0 | 0 | 2 | AU0076 |
| KIAA1086 | gdups | 3 | 0 | 0 | 0 | 3 | AU0520, AU0991, AU0866, AU0806 |
| KIAA1414 | edups | 1 | 0 | 0 | 0 | 3 | AU1650 |
| KIAA1576 | edups | 1 | 0 | 0 | 1 | 2 | AU0385, AU0033 |
| KIAA1586 | edels | 3 | 0 | 2 | 0 | 7 | AU1559, AU1533, AU1215 |

(continued)

| gene | class | AGRE. Cases. Unrelated | Screening. Controls | ACC. Cases | ACC. Controls | AGRE. Cases. Total | RE.Family.ID |
|------|-------|------------------------|---------------------|------------|---------------|--------------------|--------------|
| KIAA1666 | gdups | 2 | 0 | 3 | 0 | 2 | AU0018, AU0049 |
| KIAA1838 | edups | 1 | 0 | 0 | 0 | 2 | AU0264 |
| KIAA1856 | edups | 4 | 0 | 0 | 1 | 4 | AU1189, AU0971, AU0752, AU0680, AU0438, AU0481, AU0068 |
| KIF12 | gdups | 1 | 0 | 0 | 0 | 2 | AU0227 |
| KIF1A | edups | 1 | 0 | 0 | 0 | 2 | AU1505, AU0907 |
| KIF26B | edups | 2 | 0 | 0 | 0 | 2 | AU1889, AU0062 |
| KLF6 | edels | 1 | 0 | 0 | 0 | 2 | AU0263 |
| KLHDC6 | gdups | 1 | 0 | 0 | 0 | 2 | AU0835 |
| KLHL21 | edups | 1 | 0 | 0 | 0 | 2 | AU1172, AU0231 |
| KLHL22 | gdups | 3 | 0 | 3 | 1 | 3 | AU1334, AU0018, AU0049 |
| KLHL8 | edels | 1 | 0 | 0 | 0 | 2 | AU0781 |
| KREMEN2 | edels | 3 | 0 | 4 | 0 | 3 | AU1921, AU1601, AU1085, AU1338, AU1107 |
| KRT3 | edels | 1 | 0 | 0 | 0 | 4 | AU0700 |
| KRTAP13-2 | gdups | 2 | 0 | 0 | 0 | 3 | AU1227, AU0816, AU0753, AU0158 |
| KRTAP23-1 | gdups | 2 | 0 | 0 | 0 | 3 | AU1227, AU0816, AU0753, AU0158 |
| KRTAP24-1 | gdups | 2 | 0 | 0 | 0 | 3 | AU1227, AU0816, AU0753, AU0158 |
| KRTAP26-1 | gdups | 2 | 0 | 0 | 0 | 3 | AU1227, AU0816, AU0753, AU0158 |
| KRTAP27-1 | gdups | 2 | 0 | 0 | 0 | 3 | AU1227, AU0816, AU0753, AU0158 |
| KRTHB1 | gdups | 3 | 0 | 0 | 1 | 3 | AU0971, AU0504, AU0005 |
| LAMA1 | gdups | 1 | 0 | 0 | 0 | 2 | AU1527 |
| LAMA2 | edels | 2 | 0 | 0 | 0 | 4 | AU0831, AU0169, AU1619 |
| LDHAL6B | gdups | 1 | 0 | 0 | 0 | 2 | AU0467 |
| LENG12 | edels | 1 | 0 | 0 | 0 | 2 | AU0450 |
| LFNG | gdups | 6 | 0 | 0 | 0 | 6 | AU2005, AU1174, AU1072, AU0922, AU0832, AU0555, AU1944, AU1527, AU0806 |
| LHB | gdups | 1 | 0 | 0 | 3 | 2 | AU1088 |
| LIAS | gdups | 1 | 0 | 0 | 0 | 2 | AU0158 |
| LILRA3 | gdups | 4 | 0 | 0 | 6 | 6 | AU0648, AU0614, AU0607, AU0235, AU0624, AU0290 |
| LILRA5 | gdups | 4 | 0 | 0 | 6 | 6 | AU0648, AU0614, AU0607, AU0235, AU0624, AU0290 |
| LLGL2 | edups | 1 | 0 | 0 | 0 | 2 | AU0722 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| LMTK3 | gdups | 4 | 0 | 0 | 0 | 5 | AU0542, AU1610, AU1527, AU0795 |
| LOC128977 | gdups | 3 | 0 | 4 | 0 | 3 | AU0018, AU0991, AU0049 |
| LOC144404 | edups | 2 | 0 | 0 | 0 | 2 | AU1289, AU0068 |
| LOC148198 | edels | 1 | 0 | 0 | 0 | 2 | AU0145 |
| LOC150383 | gdups | 3 | 0 | 0 | 0 | 3 | AU0899, AU1292, AU0835 |
| LOC162073 | edels | 3 | 0 | 0 | 0 | 4 | AU1549, AU1691, AU1437 |
| LOC200810 | gdups | 1 | 0 | 0 | 0 | 2 | AU0911 |
| LOC283849 | gdups | 12 | 0 | 0 | 0 | 16 | AU0939, AU0899, AU0610, AU0509, AU0450, AU0210, AU0028, AU1368, AU0991, AU0835 |
| LOC284434 | edups | 1 | 0 | 0 | 0 | 2 | AU1626, AU1582 |
| LOC285016 | edels | 1 | 0 | 0 | 0 | 2 | AU0385 |
| LOC285498 | gdups | 3 | 0 | 1 | 0 | 3 | AU1273, AU0947, AU1283, AU0866, AU0806 |
| LOC285501 | edups | 1 | 0 | 0 | 0 | 2 | AU1688, AU1396, AU1091 |
| LOC340061 | edels | 1 | 0 | 0 | 1 | 2 | AU1240 |
| LOC342994 | edels | 1 | 0 | 0 | 0 | 2 | AU0145 |
| LOC347487 | edels | 1 | 0 | 0 | 0 | 2 | AU1823 |
| LOC387680 | gdups | 2 | 0 | 0 | 1 | 2 | AU1713, AU0845, AU0329 |
| LOC388910 | gdups | 2 | 0 | 0 | 0 | 3 | AU1912, AU1300, AU0932 |
| LOC389827 | gdups | 2 | 0 | 0 | 0 | 2 | AU1273, AU0897, AU0520, AU1650, AU0806 |
| LOC389852 | gdups | 3 | 0 | 0 | 0 | 3 | AU1083, AU0133, AU0034 |
| LOC392465 | gdups | 1 | 0 | 0 | 0 | 2 | AU1465 |
| LOC400451 | edels | 1 | 0 | 0 | 0 | 2 | AU0201 |
| LOC402057 | gdups | 2 | 0 | 0 | 0 | 2 | AU1620, AU0714 |
| LOC402635 | gdups | 2 | 0 | 0 | 0 | 2 | AU0555, AU1944 |
| LOC642968 | gdups | 2 | 0 | 0 | 0 | 2 | AU1273, AU0897, AU0520, AU1650, AU0806 |
| LOC645993 | edels | 1 | 0 | 0 | 0 | 2 | AU0729 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| LOC650137 | edels | 26 | 0 | 1 | 0 | 37 | AU1916, AU1742, AU1698, AU1497, AU1469, AU1427, AU1391, AU1344, AU1299, AU1245, AU1197, AU1195, AU1159, AU1091, AU1088, AU1010, AU0953, AU0933, AU0895, AU0883, AU0868, AU0823, AU0812, AU0802, AU0792, AU0756, AU0752, AU0718, AU0698, AU0687, AU0686, AU0664, AU0654, AU0603, AU0542, AU0187, AU0168, AU0102, AU0039, AU0029, AU0021, AU0015, AU1809, AU0768, AU0665, AU0531 |
| LOC653319 | gdups | 10 | 0 | 0 | 0 | 14 | AU0939, AU0610, AU0509, AU0450, AU0210, AU1368, AU0991, AU0835 |
| LOC728489 | gdups | 5 | 0 | 1 | 0 | 5 | AU1197, AU1072, AU0947, AU0934, AU0897, AU1283, AU1216, AU1033, AU0991, AU0068 |
| LOC728912 | gdups | 3 | 0 | 2 | 0 | 4 | AU1688, AU1610, AU1163 |
| LOC728932 | gdups | 3 | 0 | 2 | 0 | 4 | AU1688, AU1610, AU1163 |
| LOC730112 | gdups | 1 | 0 | 0 | 0 | 2 | AU0509 |
| LOC92017 | edels | 1 | 0 | 0 | 0 | 2 | AU0430 |
| LOC92154 | edels | 2 | 0 | 0 | 1 | 2 | AU1338, AU1286, AU1285, AU1213, AU0948, AU0598 |
| LOC93343 | gdups | 5 | 0 | 0 | 0 | 7 | AU0947, AU0818, AU0501, AU0109, AU0633 |
| LRBA | edels | 2 | 0 | 0 | 3 | 4 | AU0868, AU0800, AU0664 |
| LRP3 | gdups | 3 | 0 | 0 | 0 | 3 | AU1033, AU0991, AU0806 |
| LRP5 | edups | 3 | 0 | 0 | 0 | 4 | AU0483, AU0259, AU0196, AU1534 |
| LRRC27 | edups | 2 | 0 | 0 | 0 | 5 | AU1544, AU1038 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| LRRC29 | gdups | 12 | 0 | 0 | 0 | 16 | AU0939, AU0899, AU0610, AU0509, AU0450, AU0210, AU0028, AU1368, AU0991, AU0835 |
| LRRC36 | gdups | 3 | 0 | 0 | 0 | 3 | AU1207, AU0962, AU1368, AU0991, AU0835 |
| LRRIQ1 | edels | 2 | 0 | 0 | 0 | 2 | AU1963, AU0001 |
| LRTM2 | gdups | 3 | 0 | 0 | 0 | 3 | AU1072, AU0947, AU1072, AU0991 |
| LYG1 | gdups | 3 | 0 | 0 | 1 | 5 | AU1650, AU1639, AU1088, AU1006, AU0314 |
| LYG2 | gdups | 3 | 0 | 0 | 1 | 5 | AU1650, AU1639, AU1088, AU1006, AU0314 |
| MADCAM1 | edels | 3 | 0 | 8 | 0 | 3 | AU1286, AU1102, AU1301, AU0194 |
| MAG | edels | 1 | 0 | 0 | 0 | 2 | AU1301 |
| MAGEA1 | gdups | 1 | 0 | 0 | 0 | 2 | AU1400, AU0684 |
| MAGEA11 | gdups | 2 | 0 | 0 | 0 | 3 | AU1791, AU1242 |
| MAGEA2 | gdups | 1 | 0 | 0 | 0 | 2 | AU1439, AU0254, AU0254 |
| MAGEA2B | gdups | 1 | 0 | 0 | 0 | 2 | AU1439, AU0254 |
| MAGEA3 | gdups | 1 | 0 | 0 | 0 | 2 | AU1439, AU0254 |
| MAGEL2 | gdups | 5 | 0 | 8 | 0 | 8 | AU1875, AU1331, AU0106, AU0065, AU1135, AU0233 |
| MAP2K2 | gdups | 7 | 0 | 0 | 0 | 7 | AU0947, AU0934, AU0911, AU0897, AU0206, AU1527, AU0991, AU0866, AU0688, AU0676 |
| MAP3K4 | edels | 2 | 0 | 0 | 1 | 2 | AU0604, AU0453 |
| MAP4K2 | gdups | 1 | 0 | 0 | 0 | 2 | AU0647 |
| MAPK8IP1 | gdups | 5 | 0 | 0 | 0 | 6 | AU1189, AU0947, AU0932, AU0899, AU0662, AU0289, AU0099, AU0481 |
| MAST3 | gdups | 2 | 0 | 0 | 0 | 2 | AU1174, AU0866, AU0795 |
| MAST4 | edels | 2 | 0 | 0 | 0 | 3 | AU1145, AU1138, AU1198 |
| MATK | gdups | 3 | 0 | 0 | 0 | 3 | AU0520, AU0991, AU0866, AU0806 |
| MCEMP1 | gdups | 1 | 0 | 0 | 0 | 2 | AU1730 |
| MCF2 | edels | 1 | 0 | 0 | 0 | 2 | AU1823 |
| MCM10 | edels | 1 | 0 | 0 | 0 | 2 | AU0305 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| MCM5 | edups | 1 | 0 | 0 | 0 | 2 | AU1207, AU0934, AU0159 |
| MDGA2 | edels | 8 | 0 | 2 | 0 | 8 | AU1368, AU1242, AU1065, AU0915, AU0819, AU0781, AU0729, AU0696, AU0653, AU0399, AU0981, AU0290, AU0134, AU0063 |
| MED25 | gdups | 2 | 0 | 1 | 0 | 2 | AU1273, AU1072, AU0980, AU0880, AU0830, AU0753, AU0520, AU0068 |
| MEGF6 | edups | 1 | 0 | 0 | 0 | 2 | AU0356, AU0307 |
| MEIS3 | edels | 1 | 0 | 0 | 0 | 2 | AU1685 |
| MEN1 | gdups | 1 | 0 | 0 | 0 | 2 | AU0647 |
| METAP2 | edups | 2 | 0 | 0 | 0 | 3 | AU0799, AU0331 |
| MGAT4C | edels | 2 | 0 | 0 | 1 | 2 | AU0629, AU0093 |
| MGAT5 | edups | 1 | 0 | 0 | 0 | 2 | AU0179, AU0056 |
| MGC10992 | edups | 3 | 0 | 0 | 0 | 3 | AU0809, AU0501, AU0482 |
| MGC11257 | gdups | 1 | 0 | 0 | 0 | 2 | AU1174, AU0385 |
| MGC11335 | gdups | 3 | 0 | 0 | 0 | 4 | AU0647, AU1368, AU1055 |
| MGC23244 | gdups | 2 | 0 | 0 | 0 | 2 | AU1164, AU0947, AU0934, AU0922, AU0991 |
| MGC26733 | edels | 1 | 0 | 0 | 1 | 2 | AU1210 |
| MGC34647 | gdups | 1 | 0 | 0 | 0 | 2 | AU1551, AU0686 |
| MGC4266 | edups | 1 | 0 | 0 | 0 | 4 | AU0700 |
| MGC4618 | gdups | 2 | 0 | 1 | 0 | 2 | AU1164, AU0947, AU0806 |
| MGC4655 | gdups | 1 | 0 | 0 | 0 | 2 | AU0962, AU0210 |
| MGMT | edups | 4 | 0 | 0 | 0 | 8 | AU1411, AU1280, AU0895, AU0596 |
| MIOX | gdups | 4 | 0 | 0 | 0 | 4 | AU1764, AU1212, AU1174, AU1164, AU0974, AU0899, AU0616, AU1944, AU1216 |
| MKRN3 | gdups | 5 | 0 | 8 | 0 | 8 | AU1875, AU1331, AU0106, AU0065, AU1135, AU0233 |
| MKS1 | edels | 2 | 0 | 0 | 3 | 2 | AU1305, AU1539 |
| MLSTD1 | edups | 1 | 0 | 0 | 0 | 2 | AU1081 |
| MMP16 | edups | 1 | 0 | 0 | 0 | 2 | AU0862 |
| MOCOS | edups | 3 | 0 | 0 | 0 | 7 | AU1589, AU1453, AU1423, AU1088 |
| MON2 | edels | 1 | 0 | 0 | 1 | 2 | AU0700, AU0361 |

(continued)

| gene | class | AGRE. Cases. Unrelated | Screening. Controls | ACC. Cases | ACC. Controls | AGRE. Cases. Total | RE.Family.ID |
|------|-------|------------------------|---------------------|------------|---------------|--------------------|--------------| 
| MPDZ | edels | 2 | 0 | 0 | 1 | 3 | AU0788, AU0780, AU0767, AU0477, AU0329 |
| M-RIP | edups | 2 | 0 | 0 | 0 | 2 | AU0729, AU0254 |
| MRPL34 | gdups | 2 | 0 | 0 | 0 | 2 | AU2005, AU1963, AU0806 |
| MRPL40 | gdups | 3 | 0 | 4 | 0 | 3 | AU0018, AU0991, AU0049 |
| MRPL54 | gdups | 3 | 0 | 0 | 0 | 3 | AU0520, AU0991, AU0866, AU0806 |
| MSMB | gdups | 2 | 0 | 0 | 0 | 3 | AU1272, AU0845 |
| MT2A | gdups | 1 | 0 | 0 | 0 | 2 | AU1197 |
| MT3 | gdups | 1 | 0 | 0 | 0 | 2 | AU1197 |
| MT4 | gdups | 1 | 0 | 0 | 0 | 2 | AU1197 |
| MUC13 | gdups | 1 | 0 | 0 | 0 | 2 | AU0911 |
| MUM1 | gdups | 4 | 0 | 0 | 0 | 4 | AU1778, AU1632, AU1277, AU1164, AU0934, AU0924, AU0899, AU0753, AU0081, AU1944, AU1527, AU0866 |
| MUM1L1 | gdups | 1 | 0 | 0 | 0 | 2 | AU1466 |
| MYH6 | edels | 1 | 0 | 0 | 0 | 3 | AU0028 |
| MYH7 | edels | 1 | 0 | 0 | 0 | 3 | AU0028 |
| MYLK2 | gdups | 2 | 0 | 0 | 0 | 3 | AU1764, AU1072, AU0939, AU0934, AU1289 |
| MYO1E | gdups | 1 | 0 | 0 | 0 | 2 | AU0467 |
| MYOM2 | gdups | 1 | 0 | 0 | 0 | 2 | AU0109 |
| NAT2 | gdups | 1 | 0 | 0 | 0 | 2 | AU0948 |
| NBPF11 | gdups | 3 | 0 | 2 | 0 | 4 | AU1688, AU1610, AU1163 |
| NCAM2 | edels | 2 | 0 | 0 | 1 | 2 | AU1607, AU0836, AU0753, AU0729, AU0477, AU0081, AU1619 |
| NCLN | gdups | 2 | 0 | 0 | 0 | 2 | AU1277, AU1174, AU0947, AU0622, AU0081, AU1534 |
| NCOA4 | gdups | 2 | 0 | 0 | 0 | 3 | AU1272, AU0845 |
| NCR2 | edels | 2 | 0 | 0 | 0 | 2 | AU0509, AU1798, AU0627 |
| NDN | gdups | 5 | 0 | 7 | 0 | 8 | AU1875, AU1331, AU0106, AU0065, AU1135, AU0233 |
| NDUFA12L | gdups | 1 | 0 | 0 | 0 | 2 | AU0293 |

44

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| NDUFS7 | gdups | 4 | 0 | 0 | 0 | 4 | AU1778, AU1632, AU1277, AU1164, AU0934, AU0924, AU0899, AU0753, AU0081, AU1944, AU1527, AU0866 |
| NEK3 | edups | 2 | 0 | 0 | 0 | 4 | AU1753, AU1644, AU0090 |
| NFIC | edups | 3 | 0 | 0 | 0 | 4 | AU1318, AU1189, AU0920, AU0264 |
| NHLH2 | gdups | 1 | 0 | 0 | 0 | 2 | AU0651 |
| NIBP | edups | 2 | 0 | 0 | 2 | 2 | AU1912, AU1585, AU0594, AU0154, AU0520 |
| NLGN1 | edups | 1 | 0 | 0 | 0 | 2 | AU0816 |
| NLRP14 | gdups | 1 | 0 | 0 | 0 | 2 | AU1309 |
| NMB | edels | 1 | 0 | 0 | 0 | 3 | AU0561 |
| NOL3 | gdups | 1 | 0 | 0 | 0 | 2 | AU0962, AU0210 |
| NOMO3 | edels | 1 | 0 | 0 | 0 | 2 | AU0791 |
| NPFFR1 | gdups | 1 | 0 | 0 | 1 | 2 | AU1399 |
| NPNT | edels | 1 | 0 | 0 | 0 | 2 | AU1185, AU0275, AU1185 |
| NRBP2 | gdups | 2 | 0 | 0 | 1 | 2 | AU0773, AU1944, AU0796 |
| NRD1 | edups | 1 | 0 | 0 | 0 | 2 | AU1060 |
| NRXN1 | edels | 5 | 0 | 4 | 0 | 7 | AU1495, AU1210, AU0918, AU0515, AU0411 |
| NTN1 | edups | 1 | 0 | 0 | 0 | 3 | AU1650 |
| NTRK1 | edels | 1 | 0 | 0 | 0 | 2 | AU1171 |
| NUDT14 | edels | 2 | 0 | 0 | 6 | 2 | AU1921, AU1553, AU1307, AU0803, AU0493 |
| NULP1 | edups | 1 | 0 | 0 | 0 | 2 | AU0899 |
| NUP210 | edups | 2 | 0 | 0 | 0 | 3 | AU0599, AU0056 |
| NUTF2 | gdups | 3 | 0 | 0 | 0 | 4 | AU0647, AU1368, AU1055, AU0647 |
| OBSCN | edels | 2 | 0 | 0 | 2 | 3 | AU1685, AU0803, AU0029 |
| OCA2 | gdups | 5 | 0 | 7 | 0 | 8 | AU0106, AU0065, AU1331, AU1135, AU0233 |
| ODZ3 | edups | 1 | 0 | 0 | 0 | 2 | AU1493 |
| OGDHL | gdups | 2 | 0 | 0 | 0 | 2 | AU0845, AU0329 |
| OGFOD1 | gdups | 1 | 0 | 0 | 0 | 2 | AU1197 |
| OLFML1 | gdups | 1 | 0 | 0 | 0 | 2 | AU1309 |
| OLIG2 | gdups | 2 | 0 | 0 | 0 | 2 | AU1227, AU0753, AU0165, AU0158 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| OPRD1 | edups | 5 | 0 | 0 | 0 | 7 | AU1289, AU1211, AU1166, AU1099, AU1030, AU0765, AU0165, AU0157 |
| OPTN | edels | 1 | 0 | 0 | 0 | 2 | AU0305 |
| OR10A2 | gdups | 1 | 0 | 0 | 0 | 2 | AU1309 |
| OR10A4 | gdups | 1 | 0 | 0 | 0 | 2 | AU1309 |
| OR10A5 | gdups | 1 | 0 | 0 | 0 | 2 | AU1309 |
| OR11L1 | edels | 2 | 0 | 0 | 0 | 2 | AU1368, AU0951 |
| OR1C1 | edels | 3 | 0 | 1 | 0 | 3 | AU1368, AU1208, AU0951 |
| OR2AG1 | edels | 3 | 0 | 0 | 0 | 5 | AU1190, AU0965, AU0654 |
| OR2AG2 | edels | 3 | 0 | 0 | 0 | 5 | AU1190, AU0965, AU0654 |
| OR2D2 | gdups | 1 | 0 | 0 | 0 | 2 | AU1309 |
| OR2D3 | gdups | 1 | 0 | 0 | 0 | 2 | AU1309 |
| OR2L13 | edels | 2 | 0 | 0 | 0 | 2 | AU1368, AU1059, AU1961 |
| OR2M2 | edels | 2 | 0 | 0 | 1 | 2 | AU1368, AU1961 |
| OR2M3 | edels | 2 | 0 | 0 | 1 | 2 | AU1368, AU1961 |
| OR2M4 | edels | 2 | 0 | 0 | 1 | 2 | AU1368, AU1961 |
| OR2M5 | edels | 2 | 0 | 0 | 0 | 2 | AU1368, AU1961 |
| OR2W3 | edels | 2 | 0 | 0 | 0 | 2 | AU1368, AU0951 |
| OR4C6 | gdups | 4 | 0 | 0 | 5 | 5 | AU1458, AU1414, AU1350, AU1209, AU1187, AU1074, AU1000, AU0959, AU0911, AU0819, AU0747, AU0620, AU0521 AU0489 AU0465, AU0356, AU0158, AU0148, AU0139 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| OR4M2 | edels | 26 | 0 | 1 | 0 | 37 | AU1916, AU1742, AU1698, AU1497, AU1469, AU1427, AU1391, AU1344, AU1299, AU1245, AU1197, AU1195, AU1159, AU1091, AU1088, AU1010, AU0953, AU0933, AU0895, AU0883, AU0868, AU0823, AU0812, AU0802, AU0792, AU0756, AU0752, AU0718, AU0698, AU0687, AU0686, AU0664, AU0654, AU0603, AU0542, AU0187, AU0168, AU0102, AU0039, AU0029, AU0021, AU0015, AU1809, AU0768, AU0665, AU0531 |
| OR4N4 | edels | 26 | 0 | 2 | 0 | 37 | AU1916, AU1742, AU1698, AU1497, AU1469, AU1427, AU1391, AU1344, AU1299, AU1245, AU1197, AU1195, AU1159, AU1091, AU1088, AU1010, AU0953, AU0933, AU0895, AU0883, AU0868, AU0823, AU0812, AU0802, AU0792, AU0756, AU0752, AU0718, AU0698, AU0687, AU0686, AU0664, AU0654, AU0603, AU0542, AU0187, AU0168, AU0102, AU0039, AU0029, AU0021, AU0015, AU1809, AU0768, AU0665, AU0531 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| | | | | | | | AU1806, AU1695, AU1612, AU1578, AU1565, AU1559, AU1521, AU1520, AU1498, AU1465, AU1458, AU1414, AU1350, AU1274, AU1209, AU1187, AU1163, AU1074, AU1056, AU1024, AU1000, AU0965, AU0959, AU0911, AU0819, AU0747, AU0620, AU0521, AU0489, AU0465, AU0358, AU0356, AU0167, AU0158, AU0148, |
| OR4S2 | gdups | 9 | 0 | 0 | 5 | 12 | AU0139, AU0108 |
| OR51I1 | edels | 1 | 0 | 0 | 0 | 2 | AU0254 |
| OR51I2 | edels | 1 | 0 | 0 | 0 | 2 | AU0254 |
| OR51Q1 | edels | 1 | 0 | 0 | 0 | 2 | AU0254 |
| OR52E4 | gdups | 2 | 0 | 0 | 2 | 2 | AU1589, AU1301, AU0477, AU0134, AU0052 |
| OR5AT1 | edels | 2 | 0 | 0 | 0 | 2 | AU1368, AU0951 |
| OR5D13 | edels | 1 | 0 | 0 | 2 | 2 | AU1240, AU1137 |
| OR5D14 | edels | 1 | 0 | 0 | 2 | 2 | AU1240, AU1137 |
| OR5D18 | edels | 1 | 0 | 0 | 2 | 2 | AU1240, AU1137 |
| OR5H6 | gdups | 1 | 0 | 0 | 1 | 2 | AU1622 |
| OR5L1 | edels | 1 | 0 | 0 | 2 | 2 | AU1240, AU1137 |
| OR5L2 | edels | 1 | 0 | 0 | 2 | 2 | AU1240, AU1137 |
| OR6F1 | edels | 2 | 0 | 0 | 0 | 2 | AU1368, AU0951 |
| OSBPL11 | gdups | 1 | 0 | 0 | 0 | 2 | AU0911 |
| OSBPL5 | edups | 4 | 0 | 0 | 0 | 4 | AU1764, AU1277, AU1072, AU1944, AU1368, AU1216 |
| OTOP2 | gdups | 2 | 0 | 0 | 0 | 2 | AU0298, AU0806 |
| OTOR | gdups | 2 | 0 | 1 | 0 | 2 | AU1158, AU1143 |
| OTUD5 | gdups | 1 | 0 | 0 | 0 | 2 | AU1465 |
| OVCH2 | gdups | 1 | 0 | 0 | 0 | 2 | AU1309 |
| OVOL2 | gdups | 1 | 0 | 0 | 0 | 2 | AU1520 |
| OXSR1 | edups | 2 | 0 | 0 | 0 | 2 | AU1512, AU1274, AU1094, AU0991 |
| PAMCI | edels | 2 | 0 | 0 | 0 | 3 | AU0802, AU0325, AU0028 |
| PAQR4 | edels | 3 | 0 | 4 | 0 | 3 | AU 1921, AU1601, AU10851, AU 1338, AU1107 |
| PARD3B | edels | 1 | 0 | 0 | 2 | 2 | AU0733 |

(continued)

| gene | class | AGRE. Cases. Unrelated | Screening. Controls | ACC. Cases | ACC. Controls | AGRE. Cases. Total | RE.Family.ID |
|------|-------|------------------------|---------------------|------------|---------------|--------------------|--------------|
| PCDH15 | edels | 2 | 0 | 0 | 0 | 2 | AU0783, AU0465, AU0043, AU0599 |
| PCDH9 | edels | 2 | 0 | 0 | 0 | 4 | AU0753, AU0482, AU0109 |
| PCMTD2 | edels | 1 | 0 | 0 | 0 | 2 | AU0729 |
| PCQAP | gdups | 3 | 0 | 3 | 0 | 3 | AU1334, AU0018, AU1334, AU0049, AU0018 |
| PCSK1N | gdups | 1 | 0 | 0 | 0 | 2 | AU1465 |
| PDE4A | edups | 2 | 0 | 0 | 0 | 2 | AU1067, AU0467 |
| PDE4A | gdups | 2 | 0 | 1 | 0 | 2 | AU1663, AU1273, AU1174, AU1164, AU0701, AU0866 |
| PDE4C | gdups | 2 | 0 | 0 | 0 | 2 | AU1277, AU1174, AU0947, AU0520, AU0991 |
| PDE4DIP | gdups | 1 | 0 | 0 | 2 | 4 | AU0700, AU0167 |
| PDE5A | edups | 2 | 0 | 0 | 0 | 2 | AU1172, AU0803, AU1105 |
| PDE8A | edels | 1 | 0 | 0 | 0 | 3 | AU0561 |
| PDGFA | gdups | 1 | 0 | 0 | 0 | 2 | AU0385 |
| PDGFD | edels | 1 | 0 | 0 | 0 | 2 | AU1211 |
| PEMT | gdups | 2 | 0 | 0 | 1 | 2 | AU1164, AU0806 |
| PFN2 | edels | 2 | 0 | 0 | 0 | 2 | AU0707, AU0686, AU0548 |
| PHF2 | edups | 2 | 0 | 0 | 0 | 2 | AU1368, AU1650 |
| PHKB | edels | 2 | 0 | 0 | 0 | 2 | AU1713, AU1171, AU0687 |
| PHYH | edels | 1 | 0 | 0 | 0 | 2 | AU0305 |
| PI4KA | gdups | 3 | 0 | 4 | 0 | 3 | AU1334, AU0049, AU0018 |
| PIK3C2G | edups | 1 | 0 | 0 | 2 | 2 | AU1798 |
| PIK3R2 | gdups | 5 | 0 | 0 | 0 | 5 | AU1277, AU1197, AU1174, AU0947, AU0520, AU0991, AU0866, AU0795 |
| PIM2 | gdups | 1 | 0 | 0 | 0 | 2 | AU1465 |
| PIM3 | gdups | 3 | 0 | 0 | 0 | 3 | AU1742, AU1368, AU1174, AU1055, AU0932, AU0916, AU0520, AU0991 |
| PIP5K1C | gdups | 7 | 0 | 0 | 0 | 7 | AU1189, AU1164, AU1072, AU0897, AU0614, AU0520, AU1944, AU1527, AU0991, AU0866, AU0806 |
| PIWIL2 | edups | 1 | 0 | 0 | 0 | 2 | AU0199 |
| PKD1L2 | gdups | 1 | 0 | 0 | 1 | 2 | AU1019, AU0708 |
| PKD2L1 | edels | 1 | 0 | 0 | 0 | 2 | AU1016 |

(continued)

| gene | class | AGRE. Cases. Unrelated | Screening. Controls | ACC. Cases | ACC. Controls | AGRE. Cases. Total | RE.Family.ID |
|------|-------|------------------------|---------------------|------------|---------------|--------------------|--------------|
| PKIB | edels | 1 | 0 | 0 | 0 | 2 | AU1031 |
| PKMYT1 | edels | 3 | 0 | 4 | 0 | 3 | AU1921, AU1085, AU1601, AU1338, AU1107 |
| PLA2G4C | edups | 2 | 0 | 0 | 0 | 3 | AU1088, AU0393 |
| PLCB1 | edels | 1 | 0 | 0 | 0 | 2 | AU1368 |
| PLD4 | gdups | 2 | 0 | 0 | 0 | 2 | AU0008, AU1055 |
| PLEKHA9 | edels | 1 | 0 | 0 | 0 | 2 | AU0088 |
| PLEKHG4 | gdups | 12 | 0 | 0 | 0 | 16 | AU0939, AU0899, AU0875, AU0610, AU0450, AU0210, AU0028, AU1368, AU0991, AU0835 |
| PLEKHG5 | edels | 1 | 0 | 0 | 8 | 2 | AU1301 |
| PLEKHG5 | edups | 4 | 0 | 0 | 0 | 7 | AU1587, AU0725, AU0455, AU0020 |
| PLEKHM2 | edels | 3 | 0 | 0 | 2 | 3 | AU1185, AU0932, AU0063, AU1030, AU0319 |
| PLVAP | gdups | 2 | 0 | 0 | 0 | 2 | AU2005, AU1626, AU1963, AU0481 |
| PMP22 | edels | 1 | 0 | 0 | 1 | 2 | AU0149 |
| PMP22 | gdups | 1 | 0 | 0 | 0 | 2 | AU0707 |
| PNKP | gdups | 2 | 0 | 1 | 0 | 2 | AU1273, AU1072, AU0880, AU0698, AU0520, AU0068 |
| PNLIPRP1 | edels | 2 | 0 | 0 | 0 | 2 | AU0977, AU0819 |
| PNPLA7 | gdups | 1 | 0 | 0 | 0 | 2 | AU1650 |
| PODN | gdups | 1 | 0 | 0 | 0 | 2 | AU1811 |
| POSTN | edels | 3 | 0 | 0 | 0 | 4 | AU1409, AU0875, AU0600, AU0246 |
| PP2447 | edups | 5 | 0 | 0 | 0 | 7 | AU1833, AU1822, AU1582, AU1494, AU1424, AU1364, AU1348, AU1300, AU0231, AU0169, AU1861 |
| PP2447 | gdups | 8 | 0 | 0 | 0 | 8 | AU0897, AU0693, AU0688, AU0520, AU1216, AU0991, AU0866, AU0722 |
| PPFIBP2 | gdups | 1 | 0 | 0 | 0 | 2 | AU1309 |
| PPME1 | edups | 4 | 0 | 0 | 0 | 6 | AU1939, AU1862, AU1713, AU1532, AU1389, AU1271 |
| PPP1R12C | edels | 1 | 0 | 0 | 1 | 2 | AU1301 |
| PQBP1 | gdups | 1 | 0 | 0 | 0 | 2 | AU1465 |
| PRB3 | edels | 3 | 0 | 0 | 0 | 7 | AU1516, AU1423, AU1008, AU0951, AU0828 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| PRDM10 | edups | 3 | 0 | 0 | 0 | 4 | AU1532, AU1419, AU1315, AU0640, AU0607, AU0506, AU0230 |
| PRH1 | edels | 1 | 0 | 0 | 0 | 3 | AU1791 |
| PRH1 | edups | 1 | 0 | 0 | 0 | 2 | AU0001 |
| PRIC285 | edups | 3 | 0 | 1 | 0 | 5 | AU1417, AU1210, AU0253, AU009 |
| PRKAB2 | gdups | 2 | 0 | 3 | 0 | 3 | AU1688, AU1610, AU1163 |
| PRKAR1B | gdups | 1 | 0 | 0 | 0 | 2 | AU0385 |
| PRKG1 | edels | 2 | 0 | 0 | 0 | 3 | AU0688, AU1619 |
| PROP1 | gdups | 3 | 0 | 0 | 0 | 3 | AU0718, AU1298, AU1231 |
| PRPF18 | edups | 2 | 0 | 0 | 0 | 2 | AU1699, AU0880 |
| PRR4 | edels | 1 | 0 | 0 | 0 | 3 | AU1791 |
| PRR4 | edups | 1 | 0 | 0 | 0 | 2 | AU0001 |
| PRR5 | edups | 3 | 0 | 0 | 0 | 3 | AU2005, AU1273, AU1055, AU1963, AU0481 |
| PSCD2 | gdups | 4 | 0 | 0 | 0 | 5 | AU0542, AU1610, AU1527, AU0795 |
| PSG3 | edels | 1 | 0 | 0 | 1 | 2 | AU1073 |
| PSG8 | edels | 1 | 0 | 0 | 1 | 2 | AU1073 |
| PSKH1 | gdups | 4 | 0 | 0 | 0 | 5 | AU0647, AU1368, AU1055, AU0305 |
| PSMD8 | edels | 1 | 0 | 0 | 0 | 2 | AU1685 |
| PSMD8 | gdups | 2 | 0 | 0 | 0 | 3 | AU1685, AU1273, AU0618, AU0379, AU0796 |
| PTOV1 | gdups | 2 | 0 | 1 | 0 | 2 | AU1273, AU1072, AU0880, AU0698, AU0520, AU0068 |
| PTRH1 | gdups | 2 | 0 | 0 | 0 | 2 | AU1174, AU1164, AU1650 |
| PTTG1IP | gdups | 2 | 0 | 0 | 0 | 2 | AU1227, AU1039, AU0753, AU0158 |
| PWWP2 | edups | 1 | 0 | 0 | 0 | 2 | AU1047, AU0786 |
| QSER1 | edels | 1 | 0 | 0 | 3 | 2 | AU0361 |
| QSOX2 | edups | 2 | 0 | 1 | 0 | 3 | AU1055, AU0247, AU0288 |
| RAB11B | gdups | 2 | 0 | 0 | 0 | 2 | AU1592, AU1209, AU1189, AU1174, AU0899, AU0806 |
| RAB23 | edels | 1 | 0 | 0 | 0 | 2 | AU1215, AU0455 |
| RAB35 | edups | 3 | 0 | 0 | 0 | 3 | AU0563, AU0411, AU0386 |
| RAB39 | gdups | 2 | 0 | 0 | 1 | 3 | AU0521, AU0352 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| RAB3A | gdups | 2 | 0 | 0 | 0 | 2 | AU1277, AU1174, AU0947, AU0520, AU0991 |
| RABGAP1L | edels | 2 | 0 | 0 | 12 | 3 | AU1016, AU0616 |
| RAFTLIN | edels | 1 | 0 | 0 | 1 | 2 | AU0862 |
| RAI1 | edups | 5 | 0 | 0 | 0 | 8 | AU1713, AU1607, AU0993, AU0932, AU0922, AU0920, AU0585, AU0504, AU0483, AU0455, AU0289, AU0236, AU0186, AU0173 |
| RALBP1 | edels | 1 | 0 | 0 | 0 | 2 | AU1301 |
| RANBP1 | gdups | 3 | 0 | 4 | 0 | 3 | AU1174, AU0520, AU0018, AU0991, AU0049 |
| RANBP10 | gdups | 3 | 0 | 0 | 0 | 4 | AU0647, AU1368, AU1055 |
| RANBP6 | gdups | 1 | 0 | 0 | 0 | 2 | AU1699 |
| RARG | edups | 1 | 0 | 0 | 0 | 2 | AU0506 |
| RAX2 | gdups | 3 | 0 | 0 | 0 | 3 | AU0520, AU0991, AU0866, AU0806 |
| RBMS3 | edels | 1 | 0 | 0 | 0 | 2 | AU0254 |
| RBMXL2 | gdups | 1 | 0 | 0 | 0 | 2 | AU1309 |
| RCD-8 | gdups | 4 | 0 | 0 | 0 | 5 | AU1368, AU1055, AU0647, AU0305 |
| RDH13 | edels | 1 | 0 | 0 | 0 | 2 | AU1880 |
| RNF111 | edups | 3 | 0 | 0 | 0 | 3 | AU0763, AU1312, AU0039 |
| RNF111 | gdups | 1 | 0 | 0 | 0 | 2 | AU0467 |
| RNF126 | edups | 1 | 0 | 0 | 0 | 2 | AU1054 |
| RNF133 | edels | 3 | 0 | 1 | 0 | 4 | AU0733, AU0109, AU0029 |
| RNF148 | edels | 3 | 0 | 1 | 0 | 4 | AU0733, AU0109, AU0029 |
| RNF44 | edups | 5 | 0 | 0 | 1 | 8 | AU0629, AU0620, AU0034, AU0603, AU0563 |
| ROCK1 | edels | 2 | 0 | 0 | 0 | 2 | AU0190, AU0125 |
| ROPN1B | gdups | 1 | 0 | 0 | 0 | 2 | AU0911 |
| RPL9 | gdups | 1 | 0 | 0 | 0 | 2 | AU0158 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| RPS15 | gdups | 8 | 0 | 0 | 0 | 8 | AU1778, AU1632, AU1368, AU1353, AU1277, AU1212, AU1207, AU1174, AU1164, AU1099, AU0974, AU0939, AU0934, AU0924, AU0899, AU0883, AU0795, AU0753, AU0081, AU1944, AU1527, AU1033, AU0866, AU0835 |
| RPS19 | edups | 3 | 0 | 0 | 0 | 4 | AU0712, AU0515, AU0025 |
| RUVBL1 | gdups | 1 | 0 | 0 | 0 | 2 | AU0835 |
| RYR2 | edups | 3 | 0 | 0 | 2 | 3 | AU1344, AU1285, AU1257 |
| SACS | gdups | 2 | 0 | 0 | 1 | 2 | AU1347, AU0965 |
| SBF1 | gdups | 4 | 0 | 0 | 0 | 4 | AU1764, AU1212, AU1174, AU1164, AU0974, AU0899, AU0616, AU1944, AU1216 |
| SCP2 | gdups | 1 | 0 | 0 | 0 | 2 | AU1811 |
| SEC11L1 | edels | 1 | 0 | 0 | 0 | 3 | AU0561 |
| SEC61A1 | gdups | 1 | 0 | 0 | 0 | 2 | AU0835 |
| SEMA6B | gdups | 2 | 0 | 0 | 0 | 2 | AU1164, AU1099, AU0947, AU0934, AU0616, AU0866 |
| SEMA7A | edups | 2 | 0 | 0 | 0 | 2 | AU1193, AU1055, AU0125 |
| SETD4 | gdups | 2 | 0 | 0 | 0 | 3 | AU1227, AU0753, AU0316, AU0158 |
| SF3B3 | gdups | 1 | 0 | 0 | 0 | 2 | AU1551, AU0686 |
| SH2D3C | gdups | 2 | 0 | 0 | 0 | 2 | AU1174, AU1164, AU0932, AU0298, AU1650 |
| SH3TC1 | edups | 2 | 0 | 1 | 0 | 4 | AU1650, AU1226, AU1137, AU0897, AU0773, AU0481 |
| SH3YL1 | edels | 1 | 0 | 0 | 0 | 2 | AU0385 |
| SHB | edups | 2 | 0 | 0 | 0 | 2 | AU1002, AU0640, AU0991, AU0796 |
| SHD | gdups | 2 | 0 | 0 | 0 | 2 | AU1164, AU1099, AU0947, AU0934, AU0991 |
| SIAHBP1 | gdups | 2 | 0 | 0 | 1 | 2 | AU0773, AU1944, AU0796 |
| SIRT4 | edups | 2 | 0 | 0 | 0 | 4 | AU1301, AU1194, AU0756 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| SKIV2L2 | edels | 6 | 0 | 0 | 1 | 6 | AU1907, AU1823, AU1511, AU1616, AU1448, AU1407, AU1054, AU0242 |
| SLC12A8 | gdups | 1 | 0 | 0 | 0 | 2 | AU0911 |
| SLC16A5 | edups | 3 | 0 | 0 | 0 | 7 | AU1038, AU0307, AU0932 |
| SLC17A3 | edels | 1 | 0 | 0 | 0 | 2 | AU0208 |
| SLC18A1 | edels | 2 | 0 | 0 | 0 | 4 | AU1072, AU0043 |
| SLC22A18 | edups | 3 | 0 | 1 | 0 | 3 | AU1323, AU1072, AU0974, AU0816, AU0481 |
| SLC24A3 | edups | 1 | 0 | 0 | 0 | 2 | AU1321 |
| SLC25A1 | gdups | 3 | 0 | 4 | 0 | 3 | AU0018, AU0991, AU0049 |
| SLC25A34 | edels | 3 | 0 | 0 | 2 | 3 | AU1185, AU0932, AU1030, AU0319, AU0063 |
| SLC26A11 | gdups | 3 | 0 | 0 | 0 | 3 | AU2005, AU1277, AU1164, AU1072, AU0947 |
| SLC27A5 | gdups | 2 | 0 | 0 | 0 | 2 | AU1164, AU0934, AU0816, AU0786 |
| SLC28A1 | edups | 2 | 0 | 0 | 0 | 3 | AU0827, AU0109, AU0056 |
| SLC2A4RG | gdups | 3 | 0 | 0 | 0 | 3 | AU1912, AU1368, AU1277, AU1207, AU0947, AU0939, AU0934, AU0922, AU0543, AU0520, AU0109 |
| SLC2A6 | gdups | 2 | 0 | 0 | 0 | 2 | AU1273, AU0897, AU0520, AU1650, AU0806 |
| SLC35A2 | gdups | 1 | 0 | 0 | 0 | 2 | AU1465 |
| SLC41A3 | gdups | 1 | 0 | 0 | 0 | 2 | AU0911 |
| SLC43A2 | edups | 1 | 0 | 0 | 0 | 3 | AU0034 |
| SLC45A1 | edups | 2 | 0 | 0 | 0 | 3 | AU1486, AU1416, AU0253 |
| SLC5A10 | edups | 2 | 0 | 0 | 1 | 2 | AU1197, AU0665 |
| SLC5A8 | gdups | 1 | 0 | 0 | 0 | 2 | AU1317 |
| SLC6A15 | edels | 3 | 0 | 0 | 1 | 4 | AU1482, AU0993, AU0599, AU0180, AU0043, AU0800 |
| SLC6A7 | edels | 1 | 0 | 0 | 0 | 2 | AU1171 |
| SLC7A10 | gdups | 3 | 0 | 0 | 0 | 3 | AU0816, AU1033, AU0991, AU0806 |
| SLC7A9 | edups | 1 | 0 | 0 | 0 | 2 | AU1289 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| SLC9A5 | gdups | 12 | 0 | 0 | 0 | 16 | AU0939, AU0899, AU0722, AU0610, AU0450, AU0210, AU0028, AU1368, AU0991, AU0835 |
| SLCO1A2 | edels | 3 | 0 | 0 | 12 | 3 | AU1368, AU1098, AU0753, AU0681, AU0653, AU0134 |
| SLCO1B3 | edups | 2 | 0 | 0 | 0 | 3 | AU1953, AU1916 |
| SMARCA4 | edups | 2 | 0 | 0 | 0 | 3 | AU1289, AU1190 |
| SMU1 | edels | 1 | 0 | 0 | 0 | 2 | AU1544 |
| SMU1 | edups | 1 | 0 | 0 | 0 | 2 | AU0822 |
| SNRPB2 | gdups | 2 | 0 | 1 | 0 | 2 | AU1158, AU1143 |
| SNRPN | gdups | 5 | 0 | 8 | 0 | 8 | AU1331, AU0106, AU0065, AU1135, AU0233 |
| SNURF | gdups | 5 | 0 | 8 | 0 | 8 | AU1331, AU0106, AU0065, AU1135, AU0233 |
| SNW1 | gdups | 1 | 0 | 0 | 0 | 2 | AU0755 |
| SNX14 | edels | 2 | 0 | 0 | 5 | 2 | AU0780, AU0622, AU1048, AU0139 |
| SNX25 | edups | 2 | 0 | 0 | 0 | 3 | AU1730, AU1143, AU1010 |
| SNX4 | gdups | 1 | 0 | 0 | 0 | 2 | AU0911 |
| SNX5 | gdups | 1 | 0 | 0 | 0 | 2 | AU1520, AU0752 |
| SNX9 | edups | 1 | 0 | 0 | 0 | 2 | AU1227 |
| SOX3 | edels | 1 | 0 | 0 | 0 | 2 | AU1823 |
| SPACA5B | gdups | 3 | 0 | 0 | 0 | 3 | AU1083, AU0133, AU0034 |
| SPANXB1 | edels | 1 | 0 | 0 | 0 | 2 | AU1823 |
| SPANXB2 | edels | 1 | 0 | 0 | 0 | 2 | AU1823 |
| SPATA21 | edups | 1 | 0 | 0 | 0 | 2 | AU1344, AU0081 |
| SPG7 | gdups | 1 | 0 | 0 | 0 | 2 | AU0907 |
| SPON2 | edels | 5 | 0 | 0 | 2 | 6 | AU1318, AU1231, AU1185, AU1085, AU1083, AU0782 |
| SPRED3 | edels | 1 | 0 | 0 | 0 | 2 | AU1685 |
| SPRED3 | gdups | 4 | 0 | 0 | 0 | 5 | AU1273, AU0680, AU0618, AU0379, AU0325, AU0001, AU0796 |
| SPRN | gdups | 2 | 0 | 0 | 1 | 3 | AU1482, AU1342, AU0653, AU1165 |
| SRL | edups | 3 | 0 | 0 | 0 | 3 | AU1193, AU0767, AU1762, AU0633, AU0066 |
| SSSCA1 | edels | 3 | 0 | 1 | 0 | 4 | AU1520, AU1439, AU1102 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| SSU72 | edups | 2 | 0 | 0 | 0 | 2 | AU1010, AU0275, AU0268 |
| SSX5 | gdups | 3 | 0 | 0 | 0 | 3 | AU1083, AU0133, AU0034 |
| SSX6 | gdups | 2 | 0 | 0 | 0 | 2 | AU0034, AU0133 |
| ST3GAL2 | gdups | 1 | 0 | 0 | 0 | 2 | AU1551, AU1445, AU0686 |
| STAM2 | edups | 1 | 0 | 0 | 0 | 3 | AU1525, AU1454, AU0958 |
| STEAP3 | edups | 2 | 0 | 0 | 1 | 3 | AU1622, AU1444, AU1684 |
| STIP1 | gdups | 5 | 0 | 0 | 0 | 5 | AU1207, AU0947, AU0934, AU1164, AU1033, AU0866, AU0835, AU0806 |
| SUCLG2 | edels | 2 | 0 | 1 | 0 | 3 | AU0034, AU0122 |
| SULT2A1 | edels | 2 | 0 | 0 | 1 | 2 | AU1424, AU1222, AU1373 |
| SUPT4H1 | gdups | 2 | 0 | 0 | 0 | 2 | AU1189, AU0991, AU0806 |
| SYNGR2 | gdups | 3 | 0 | 0 | 0 | 3 | AU1174, AU0947, AU0991, AU0835, AU0806 |
| SYT9 | gdups | 1 | 0 | 0 | 0 | 2 | AU1309 |
| TAF11 | gdups | 2 | 0 | 0 | 1 | 2 | AU1575, AU1412, AU0668 |
| TANC1 | edels | 2 | 0 | 0 | 0 | 2 | AU1334, AU0378, AU0381 |
| TAS2R44 | edels | 1 | 0 | 0 | 0 | 3 | AU1791 |
| TAS2R44 | gdups | 1 | 0 | 0 | 0 | 2 | AU0001 |
| TAS2R48 | edels | 1 | 0 | 0 | 0 | 3 | AU1791 |
| TAS2R49 | edels | 1 | 0 | 0 | 1 | 3 | AU1791 |
| TBC1D4 | edels | 1 | 0 | 0 | 0 | 2 | AU1565 |
| TCERG1 | edels | 2 | 0 | 0 | 0 | 2 | AU0808, AU0679, AU0453 |
| TCP10L | gdups | 3 | 0 | 0 | 0 | 3 | AU1227, AU0753, AU0180, AU0910, AU0158 |
| TDP1 | edups | 2 | 0 | 0 | 0 | 2 | AU1579, AU0980, AU0923, AU0314, AU1313 |
| TEKT3 | edels | 1 | 0 | 0 | 1 | 2 | AU0149 |
| TEKT3 | gdups | 1 | 0 | 0 | 0 | 2 | AU0707 |
| TESK2 | edels | 1 | 0 | 0 | 1 | 2 | AU0029 |
| TF | edups | 2 | 0 | 0 | 0 | 2 | AU0771, AU0752 |
| TH | edels | 2 | 0 | 0 | 0 | 2 | AU1231, AU1102, AU1098 |
| THAP11 | gdups | 3 | 0 | 0 | 0 | 4 | AU0647, AU1368, AU1055 |
| TIGD1 | gdups | 1 | 0 | 0 | 0 | 2 | AU1213 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| TIMM17B | gdups | 1 | 0 | 0 | 0 | 2 | AU1465 |
| TJP3 | gdups | 5 | 0 | 0 | 0 | 5 | AU1411, AU1072, AU0520, AU1944, AU0991, AU0866, AU0806 |
| TK1 | gdups | 2 | 0 | 0 | 0 | 2 | AU1174, AU0947, AU0991, AU0835 |
| TLL1 | edups | 1 | 0 | 0 | 0 | 2 | AU1379, AU1352, AU1234 |
| TLN2 | edels | 1 | 0 | 0 | 0 | 2 | AU1261 |
| TMC7 | gdups | 1 | 0 | 0 | 0 | 2 | AU0993 |
| TMC03 | edels | 1 | 0 | 0 | 0 | 2 | AU1327 |
| TMC07 | edups | 2 | 0 | 0 | 0 | 2 | AU1353, AU1220, AU0312 |
| TMEM104 | edups | 1 | 0 | 0 | 0 | 2 | AU0254 |
| TMEM112 | gdups | 3 | 0 | 0 | 0 | 3 | AU1348, AU1174, AU0947, AU0932, AU0678, AU0520, AU1216, AU1159, AU0796 |
| TMEM138 | gdups | 2 | 0 | 0 | 0 | 3 | AU1323, AU0918, AU0246 |
| TMEM146 | edels | 1 | 0 | 0 | 0 | 2 | AU1135 |
| TMEM16E | edels | 2 | 0 | 0 | 0 | 2 | AU0604, AU0180 |
| TMEM18 | edels | 1 | 0 | 0 | 0 | 2 | AU0385 |
| TMEM56 | edels | 1 | 0 | 0 | 0 | 2 | AU0028 |
| TNFAIP8L1 | gdups | 2 | 0 | 0 | 0 | 2 | AU1164, AU1099, AU0947, AU0934, AU0616 |
| TNFRSF10D | edups | 1 | 0 | 0 | 1 | 2 | AU0745, AU0267 |
| TNFRSF12A | edels | 2 | 0 | 1 | 0 | 2 | AU1085, AU1338, AU1107 |
| TNFRSF14 | gdups | 4 | 0 | 0 | 0 | 4 | AU0934, AU0899, AU0880, AU0841, AU0816, AU0693, AU0520, AU0161, AU1409, AU0866 |
| TNFRSF19 | gdups | 2 | 0 | 0 | 1 | 2 | AU1347, AU0965 |
| TNFRSF21 | edups | 1 | 0 | 0 | 0 | 3 | AU0379 |
| TNFRSF25 | edels | 1 | 0 | 0 | 8 | 2 | AU1301 |
| TNFRSF8 | edups | 3 | 0 | 0 | 0 | 5 | AU1344, AU1244, AU0241 |
| TNIP2 | gdups | 2 | 0 | 1 | 0 | 2 | AU1338, AU0947, AU0866 |
| TNNT1 | edels | 1 | 0 | 0 | 0 | 2 | AU1301 |
| TNS3 | edups | 1 | 0 | 0 | 0 | 2 | AU0780 |
| TOR2A | gdups | 2 | 0 | 0 | 0 | 2 | AU1174, AU1164, AU1650 |

(continued)

| gene | class | AGRE. Cases. Unrelated | Screening. Controls | ACC. Cases | ACC. Controls | AGRE. Cases. Total | RE.Family.ID |
|------|-------|------------------------|---------------------|------------|---------------|--------------------|--------------|
| TP73 | edels | 2 | 0 | 0 | 1 | 2 | AU1171, AU1157, AU1098, AU0809, AU0614, AU1231 |
| TPPP | edels | 8 | 0 | 0 | 1 | 9 | AU1867, AU1243, AU1235, AU1231, AU1213, AU1185, AU1157, AU0951, AU0802, AU1373, AU1333, AU1105 |
| TPPP | gdups | 1 | 0 | 0 | 0 | 2 | AU1172, AU0934 |
| TRADD | gdups | 1 | 0 | 0 | 0 | 2 | AU0962, AU0210 |
| TRAPPC5 | gdups | 1 | 0 | 0 | 0 | 2 | AU1730 |
| TRDN | edups | 1 | 0 | 0 | 0 | 2 | AU0977 |
| TRHDE | edels | 2 | 0 | 0 | 0 | 2 | AU1215, AU0752, AU0736, AU0668, AU0465, AU0972 |
| TRIM58 | edels | 2 | 0 | 0 | 0 | 2 | AU1368, AU0951 |
| TRPM1 | gdups | 2 | 0 | 0 | 1 | 3 | AU1208, AU0520 |
| TRPM5 | edels | 1 | 0 | 0 | 0 | 2 | AU1612 |
| TRPS1 | edels | 1 | 0 | 0 | 0 | 2 | AU0063 |
| TRPT1 | gdups | 5 | 0 | 0 | 0 | 5 | AU1207, AU0947, AU0934, AU1164, AU1033, AU0866, AU0835, AU0806 |
| TSC2 | edups | 2 | 0 | 0 | 0 | 3 | AU0482, AU0056 |
| TSNAXIP1 | gdups | 3 | 0 | 0 | 0 | 4 | AU0647, AU1368. AU1055 |
| TSPAN32 | edels | 1 | 0 | 0 | 0 | 2 | AU1921, AU1798 |
| TSSK2 | gdups | 3 | 0 | 4 | 0 | 3 | AU0018, AU0991, AU0049 |
| TTC16 | gdups | 2 | 0 | 0 | 0 | 2 | AU1174, AU1164, AU1650 |
| TTC9B | gdups | 1 | 0 | 0 | 0 | 2 | AU1054 |
| TTYH3 | edels | 2 | 0 | 0 | 11 | 2 | AU1437, AU1285, AU0782 |
| TUSC3 | edels | 2 | 0 | 0 | 0 | 2 | AU0241, AU0827 |
| TUSC5 | gdups | 1 | 0 | 0 | 1 | 2 | AU1575 |
| TXNRD2 | gdups | 3 | 0 | 4 | 0 | 3 | AU0018, AU0991, AU0049 |
| UBE1L2 | edups | 1 | 0 | 0 | 0 | 2 | AU1688 |
| UBE20 | edups | 4 | 0 | 0 | 0 | 7 | AU0736, AU0696, AU0325, AU0289, AU0215 |
| UBE3A | gdups | 5 | 0 | 8 | 0 | 8 | AU1331, AU0106, AU0065, AU1135, AU0233 |
| UBR1 | edups | 2 | 0 | 0 | 1 | 3 | AU1644, AU0809, AU1254 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| UBXD1 | gdups | 2 | 0 | 0 | 0 | 2 | AU1273, AU1174, AU1099, AU1072, AU1944, AU1216 |
| UFD1L | gdups | 3 | 0 | 4 | 0 | 3 | AU0018, AU0991, AU0049 |
| UFM1 | gdups | 1 | 0 | 0 | 0 | 2 | AU1403 |
| UGCGL2 | edels | 1 | 0 | 0 | 0 | 2 | AU0700, AU0696 |
| UGDH | gdups | 1 | 0 | 0 | 0 | 2 | AU0158 |
| UGT1A5 | edels | 2 | 0 | 0 | 0 | 3 | AU1535, AU1458, AU1277, AU1059, AU1535, AU1059 |
| UNC13C | edels | 1 | 0 | 0 | 1 | 2 | AU1589, AU0729 |
| UNC84A | gdups | 1 | 0 | 0 | 0 | 2 | AU0385 |
| UNC93B1 | edels | 3 | 0 | 1 | 0 | 3 | AU1553, AU0803, AU0746 |
| UNCX4.1 | gdups | 3 | 0 | 0 | 0 | 4 | AU1632, AU1348, AU1174, AU0922, AU0899, AU0385, AU0340, AU1527 |
| UNQ2446 | gdups | 4 | 0 | 0 | 0 | 5 | AU0647, AU1368, AU1055, AU0305 |
| URP2 | gdups | 5 | 0 | 0 | 0 | 5 | AU1207, AU0947, AU0934, AU1164, AU1033, AU0866, AU0835, AU0806 |
| USF2 | edels | 1 | 0 | 0 | 0 | 2 | AU1553, AU1301 |
| USH1G | gdups | 2 | 0 | 0 | 0 | 2 | AU0298, AU0806 |
| USH2A | edels | 3 | 0 | 0 | 0 | 4 | AU0920, AU0622, AU0352, AU0137 |
| USP20 | edups | 2 | 0 | 0 | 0 | 2 | AU0076, AU0008 |
| UTRN | edups | 2 | 0 | 0 | 0 | 3 | AU1496, AU1524 |
| VAC14 | gdups | 1 | 0 | 0 | 0 | 2 | AU1551 |
| VANGL1 | gdups | 1 | 0 | 0 | 0 | 2 | AU0651 |
| VCX2 | gdups | 3 | 0 | 0 | 0 | 5 | AU0661, AU0268, AU0165, AU1798 |
| VCX3A | gdups | 2 | 0 | 0 | 0 | 2 | AU0943, AU0842, AU0708, AU0264 |
| VDP | edels | 1 | 0 | 0 | 0 | 2 | AU0915 |
| VIPR2 | edups | 2 | 0 | 0 | 0 | 2 | AU0159, AU1283 |
| VPS37B | gdups | 4 | 0 | 0 | 0 | 6 | AU1378, AU1289, AU0899, AU0836, AU0688, AU0001, AU0382 |
| VPS4A | edups | 1 | 0 | 0 | 0 | 3 | AU0733 |
| WASF3 | edups | 5 | 0 | 0 | 1 | 8 | AU1565, AU1054, AU1000, AU0610, AU0509, AU0138, AU0362 |
| WDR18 | edels | 2 | 0 | 0 | 9 | 2 | AU1231, AU0809, AU1171 |

(continued)

| gene | class | AGRE. Cases. Unrelated | Screening. Controls | ACC. Cases | ACC. Controls | AGRE. Cases. Total | RE.Family.ID |
|---|---|---|---|---|---|---|---|
| WDR71 | edups | 2 | 0 | 0 | 0 | 2 | AU0997, AU0290 |
| WDR73 | edels | 1 | 0 | 0 | 0 | 3 | AU0561 |
| WDR78 | edups | 3 | 0 | 0 | 0 | 7 | AU1916, AU1880, AU1791, AU1368 |
| WNT7A | edups | 3 | 0 | 0 | 0 | 4 | AU0633, AU0121, AU0068 |
| WWP2 | edups | 1 | 0 | 0 | 0 | 2 | AU0718 |
| XG | edels | 2 | 0 | 0 | 0 | 4 | AU1059, AU0338 |
| XYLT1 | edels | 1 | 0 | 0 | 0 | 2 | AU0791 |
| YTHDF1 | edels | 1 | 0 | 0 | 0 | 2 | AU0746 |
| ZBTB20 | edels | 1 | 0 | 0 | 0 | 2 | AU1055 |
| ZC3H7B | edups | 5 | 0 | 0 | 0 | 7 | AU1582, AU1226, AU1158, AU1030, AU0947, AU0346, AU0017, AU1359 |
| ZCCHC14 | edups | 1 | 0 | 0 | 0 | 2 | AU0771, AU0575 |
| ZDHHC1 | gdups | 3 | 0 | 0 | 0 | 3 | AU1207, AU0962, AU1368, AU0991, AU0835 |
| ZDHHC11 | edels | 2 | 0 | 0 | 0 | 2 | AU1185, AU1056, AU0951, AU1105 |
| ZFAND2A | gdups | 1 | 0 | 0 | 1 | 2 | AU1174, AU0922, AU0385 |
| ZFP37 | edels | 1 | 0 | 0 | 1 | 2 | AU1247 |
| ZFPM2 | edels | 1 | 0 | 0 | 0 | 2 | AU0725, AU0452, AU0259 |
| ZIC3 | edels | 1 | 0 | 0 | 0 | 2 | AU1823 |
| ZMYND19 | gdups | 2 | 0 | 0 | 0 | 2 | AU0767, AU0622 |
| ZNF135 | edels | 1 | 0 | 0 | 0 | 2 | AU0179 |
| ZNF141 | edels | 2 | 0 | 0 | 0 | 2 | AU1251, AU1251, AU0032 |
| ZNF148 | gdups | 1 | 0 | 0 | 0 | 2 | AU0911 |
| ZNF208 | edels | 1 | 0 | 0 | 0 | 2 | AU0145 |
| ZNF208 | gdups | 2 | 0 | 0 | 2 | 3 | AU1338, AU1292 |
| ZNF214 | gdups | 1 | 0 | 0 | 0 | 2 | AU1309 |
| ZNF215 | gdups | 1 | 0 | 0 | 0 | 2 | AU1309 |
| ZNF257 | edels | 2 | 0 | 0 | 1 | 3 | AU1277, AU0145 |
| ZNF324 | gdups | 2 | 0 | 0 | 0 | 2 | AU1174, AU0934, AU0816, AU1527, AU0481 |
| ZNF37A | gdups | 2 | 0 | 0 | 0 | 3 | AU0257, AU1403 |
| ZNF446 | gdups | 2 | 0 | 0 | 0 | 2 | AU1174, AU0934, AU0816, AU1527, AU0481 |
| ZNF451 | edels | 1 | 0 | 1 | 0 | 2 | AU1215 |
| ZNF467 | edups | 2 | 0 | 0 | 0 | 2 | AU0412, AU1048 |
| ZNF492 | edels | 1 | 0 | 0 | 0 | 2 | AU0145 |
| ZNF499 | gdups | 2 | 0 | 0 | 0 | 2 | AU1164, AU0934, AU0816, AU0786 |

(continued)

| gene | class | AGRE. Cases. Unrelated | Screening. Controls | ACC. Cases | ACC. Controls | AGRE. Cases. Total | RE.Family.ID |
|------|-------|----|----|----|----|----|----|
| ZNF574 | edels | 2 | 0 | 0 | 5 | 2 | AU1305, AU1286 |
| ZNF592 | edels | 1 | 0 | 0 | 0 | 3 | AU0561 |
| ZNF650 | edels | 2 | 0 | 0 | 2 | 2 | AU0767, AU0419, AU0264 |
| ZNF676 | edels | 2 | 0 | 0 | 0 | 3 | AU1277, AU0145 |
| ZNF74 | gdups | 3 | 0 | 3 | 1 | 3 | AU1334, AU0049, AU0018 |
| ZNF85 | edels | 2 | 0 | 0 | 1 | 2 | AU0911, AU0809, AU0677, AU0201, AU0980 |
| ZNF99 | edels | 1 | 0 | 0 | 0 | 2 | AU0145 |
| ZSCAN2 | edels | 1 | 0 | 0 | 0 | 3 | AU0561 |

**Table 2**

Description of AGRE sample used in the analysis.

*CHOP Control Cohort:*
    1110 samples genotyped
    1070 retained after QC (96% pass rate)

*NINDS Control Cohort:*
    540 samples genotyped
    418 retained after QC (77% pass rate)

*AGRE Family Cohort:*
    4163 samples genotyped on v3 arrays
    3832 retained after QC (92% pass rate)

*ACC Cases & Controls:*
    see Glessner *et al., 2009, Nature* for a full description

**Table 3**

| Summary of CNVs in AGRE cases, first-degree relatives, and unrelated controls. | | | | |
|------|------|------|------|------|
| | AGRE affected | AGRE unaffected (siblinqs/parents) | NINDS controls | CHOP controls |
| N= | 1673 | 2159 | 418 | 1070 |
| Mean # CNV | 24.7 | 25.2 | 20.5 | 23.3 |
| Mean # eDels | 2.0 | 2.1 | 2.3 | 2.6 |
| Mean # eDups | 6.0 | 6.3 | 2.2 | 4.2 |
| Mean # gDups | 4.0 | 4.1 | 1.0 | 2.5 |

# Table 4

| Region | #SNP | Length (bp) | Type | AGRE ID | Scored status | Inheritance status | Shared by affected sibling? | Previous reports |
|---|---|---|---|---|---|---|---|---|
| 15q11-13 | 1246 | 5,902,313 | dup | AU010601 | | parent | | [22] |
| 15q11-13 | 1246 | 5,902,313 | dup | AU010604 | Autism | inherited | No | [22] |
| 15q11-13 | 1246 | 5,902,313 | dup | AU1331202 | | parent | | |
| 15q11-13 | 1246 | 5,902,313 | dup | AU1331302 | Autism | inherited | Yes | |
| 15q11-13 | 1246 | 5,902,313 | dup | AU1331303 | Autism | inherited | Yes | |
| 15q11-13 | 1130 | 5,008,629 | dup | AU006501 | | parent | | |
| 15q11-13 | 1130 | 5,008,629 | dup | AU006503 | Spectrum | inherited | Yes | AGRE cytogenetic annotation |
| 15q11-13 | 1130 | 5,008,629 | dup | AU006504 | Autism | inherited | Yes | AGRE cytogenetic annotation, [21] |
| 15q11-13 | 1130 | 5,008,629 | dup | AU1135202 | Autism | de novo | NA | |
| 15q11-13 | 1127 | 4,993,869 | dup | AU023303 | Spectrum | NA | Yes | [22] |
| 15q11-13 | 1127 | 4,993,869 | dup | AU023304 | Autism | NA | Yes | [21,22] |
| 15q11-13 | 1127 | 4,993,869 | dup | AU1607307 | Autism | de novo | No | |
| 15q11-13 | 569 | 3,540,078 | del | AU1024202 | | parent | | |
| 15q11-13 | 569 | 3,540,078 | del | AU1024301 | Autism | inherited | NA | |
| 15q11-13 | 437 | 1,347,744 | dup | AU038504 | Autism | de novo | No | [22] |
| 15q11-13 | 287 | 1,578,642 | dup | AU1208301 | Autism | de novo | No | |
| 15q11-13 | 273 | 1,517,841 | dup | AU1875202 | | parent | | |
| 15q11-13 | 98 | 572,462 | dup | AU052003 | Autism | NA | Yes | [21] |
| 15q11-13 | 98 | 572,462 | dup | AU052004 | Autism | NA | Yes | |
| 16p11.2 | 47 | 530,466 | del | AU0154302 | Autism | de novo | Yes | [10,11] |
| 16p11.2 | 47 | 530,466 | del | AU0154303 | Autism | de novo | Yes | [10,11,21] |
| 16p11.2 | 47 | 530,466 | del | AU029803 | Autism | de novo | No | [10,11,21] |
| 16p11.2 | 47 | 530,466 | del | AU041905 | Autism | de novo | No | [10,11,21] |
| 16p11.2 | 47 | 530,466 | del | AU0938301 | Autism | de novo | No | [10,11,21] |
| 16p11.2 | 47 | 530,466 | dup | AU002901 | | parent | | [11] |
| 16p11.2 | 47 | 530,466 | dup | AU002903 | Autism | inherited | Yes | [11] |
| 16p11.2 | 47 | 530,466 | dup | AU002904 | None | inherited | | [11] |
| 16p11.2 | 47 | 530,466 | dup | AU002905 | | inherited | Yes | [10,11] |
| 22q11.21 | 512 | 2,534,567 | dup | AU001802 | | parent | | [22] |
| 22q11.21 | 512 | 2,534,567 | dup | AU001804 | Autism | inherited | No | [21,22] |
| 22q11.21 | 512 | 2,534,567 | dup | AU004903 | Autism | de novo | No | [21,22] |
| 22q11.21 | 335 | 1,429,207 | dup | AU0991301 | Autism | NA | No | |
| 22q11.21 | 177 | 728,859 | dup | AU1334201 | | parent | | |
| 22q11.21 | 177 | 728,859 | dup | AU1334302 | Spectrum | inherited | No | |
| 22q11.21 | 149 | 601,423 | del | AU1555302 | Autism | NA | NA | |

doi:10.1371/journal.pgen.1000536.t001

## Table 5

TaqMan primers and probes used in CNV validation.

*Reporter and reporter quencher are FAM and NFQ, respectively, unless noted*

**AssaBSA15**

Target = human BCL9

forward primer = CTGAGTTGATTTTTGGTTAAGTTGATTCCTT

(continued)

**AssaBSA15**
reverse primer = GGACCTGAAATTCGAGGATTCTGT
reporter sequence = TAGGAATGGGCATTAATAC

**AssaBSA16**
Target = human NLRP3
forward primer = AGTGCAACCCAGGCTTTCTATTT
reverse primer = GTGTTTCTAACGCACTTTTTGTCTCA
reporter sequence = CAGACAACCTGTAAAAGC

**AssaBSA20**
Target = human NKX3-2
forward primer = TGGAAGCTCTATTCGCTGTATTTTTTCT
reverse primer = CCAAAAGTCGGGAAAAGACAGTTT
reporter sequence = CATGCCCTCCTGGACGC

**AssaBSA21**
Target = human HHIP-itg
forward primer = TCATCTCAGTTGTGATCGTTCTGTTTT
reverse primer = AGGGTGTGCAGAAATGGTACTTAATT
reporter sequence = TCTACATCGTGAAATTAC

**AssaBSA22**
Target = human 4q32.1
forward primer = TGAGTAACAGCATTTATCATGGCTTGA
reverse primer = GGAAAAGGTTTTGAAAACATTGTTATCACAGT
reporter sequence = CCTAAGATCAGGCAATTAG

**AssaBSA23**
Target = human 6q16.1
forward primer = AGTGACAGTACATGCAACAGTTCAT
reverse primer = GCTCCTCTGTAGCTGTCAGTTC
reporter sequence = CTGTGCCAAACTTCA

**AssaBSA25**
Target = human 8q21.2
forward primer = AGTGTAGGTGCAATCAAAGAGAATGA
reverseprimer = CTCAATTGTTTTAAAATATTGGGCAAAGTTCA
reporter sequence = ATAAGTGGTTTAGCATTTCTG

**AssaBSA26**
Target = human HPSE2-in
forward primer = TCAGTGAGGTCTGGGTTCAATATCT
reverse primer = TGCTGCTCATATGTTATCAAAGCATTATATCA
reporter sequence = TTGGCTGTCCGCCTTGT

**AssaBSA27**
Target = human TAT
forward primer = GCTTCTTGGAGGCTGCTTTCT
reverse primer = CACCACTGCCTGATCAGCTT
reporter sequence = TTGGAAGGTAAAAATCTC

(continued)

**AssaBSA27**

**AssaBSA28**
Target = human PPP1R16B
forward primer = CCAGCTGGTAATGTTGTCCTTCT
reverse primer = GAGAGTAGCACGGGCTTCT
reporter sequence = CACTCGCAGAACCCCA

**AssaBSA29**
Target = human BHLHB4
forward primer = GCGTAGCCGTGGCTTAGT
reverse primer = CCATGGCCGAGCTCAAGT
reporter sequence = CAGGTACGCGTCCCC

**AssaBSA30**
Target = human DMD
forward primer = GATGGACTTCTTATCTGGATAGGTGGTA
reverse primer = GAGTCTCAAATATAGAAACCAAAAATTGATGTGT
reporter sequence = CAACATCTGTAAGCACATTAA

**AssaBSA32**
Target = RNaseP endogenous control
reporter = VIC; quencher = TAMRA; primer limited
Part Number 4316844 (applied biosystems)

**Table 6**

| gene | class | locus | AGRE.Cases.Unrelated | Map.Position.March.2006. | Chr.Band | ACRD? | Combined.P |
|---|---|---|---|---|---|---|---|
| ABCB9 | gdups | 120 | 3 | chr12:121979494-122025705 | 12q24.31 | Yes | 0.07 |
| ABCC1 | edups | 144 | 3 | chr16:15950935-16144432 | 16p13.11 | No | 0.07 |
| ACP6 | gdups | 15 | 3 | chr1:145585794-145608988 | 1q21.1 | Yes | 4.79E-03 |
| ADAMTS5 | gdups | 198 | 2 | chr21:27212112-27260703 | 21q21.3 | No | 0.17 |
| ADAMTSL1 | edups | 76 | 2 | chr9:18464098-18900948 | 9p22.2 | No | 0.17 |
| ADCY1 | edups | 66 | 3 | chr7:45580646-45729237 | 7p13 | No | 0.07 |
| ADM2 | gdups | 208 | 4 | chr22:49266878-49271732 | 22q13.33 | Yes | 0.03 |
| AHR | gdups | 65 | 2 | chr7:17304832-17352294 | 7p21.1 | Yes | 0.37 |
| APBA3 | gdups | 173 | 3 | chr19:3701771-3712673 | 19p13.3 | No | 0.07 |
| APLP1 | gdups | 185 | 3 | chr19:41051241-41062539 | 19q13.12 | Yes | 0.07 |
| ARHGEF16 | edups | 2 | 3 | chr1:3361100-3387537 | 1p36.32 | No | 0.07 |
| ARID3A | edups | 168 | 4 | chr19:877037-923781 | 19p13.3 | Yes | 0.03 |
| ARL11 | gdups | 123 | 4 | chr13:49100436-49106009 | 13q14.3 | No | 0.03 |
| ARSA | gdups | 208 | 4 | chr22:49410316-49413473 | 22q13.33 | Yes | 0.10 |
| ARSD | edels | 209 | 2 | chrX:2832011-2857392 | Xp22.33 | Yes | 0.17 |
| ARSD | gdups | 209 | 2 | chrX:2832011-2857392 | Xp22.33 | Yes | 0.17 |
| ARVCF | gdups | 203 | 4 | chr22:18337421-18384309 | 22q11.21 | Yes | 8.05E-04 |
| ASCC3 | edups | 55 | 3 | chr6:101062791-101435961 | 6q16.3 | No | 0.07 |
| ATCAY | gdups | 173 | 3 | chr19:3831639-3873184 | 19p13.3 | No | 0.07 |
| ATP10A | gdups | 132 | 6 | chr15:23474952-23661412 | 15q12 | Yes | 9.28E-06 |
| ATP6V0D1 | gdups | 149 | 3 | chr16:66029418-66072590 | 16q22.1 | No | 0.07 |
| BC002942 | gdups | 208 | 5 | chr22:49288250-49292975 | 22q13.33 | Yes | 0.01 |
| BCL9 | gdups | 15 | 3 | chr1:145479806-145564641 | 1q21.1 | Yes | 4.79E-03 |
| BTBD4 | edups | 197 | 3 | chr20:61846322-61907300 | 20q13.33 | Yes | 0.07 |
| BTN2A1 | edels | 53 | 2 | chr6:26566168-26577844 | 6p22.1 | No | 0.37 |
| BXDC1 | edups | 56 | 2 | chr6:111409984-111453486 | 6q21 | Yes | 0.17 |
| BZRAP1 | edels | 158 | 6 | chr17:53733597-53760477 | 17q22 | No | 8.05E-04 |
| BZRAP1 | edups | 158 | 4 | chr17:53733597-53760477 | 17q22 | No | 0.03 |
| C10orf72 | edups | 83 | 9 | chr10:49896258-49993560 | 10q11.22 | Yes | 2.08E-03 |
| C11orf72 | gdups | 102 | 3 | chr11:67126927-67130753 | 11q13.2 | No | 0.07 |
| C12orf38 | gdups | 121 | 2 | chr12:122721644-122758901 | 12q24 | Yes | 0.17 |
| C15orf2 | gdups | 132 | 6 | chr15:22471634-22479686 | 15q11.2 | Yes | 3.79E-06 |
| C19orf19 | edels | 167 | 3 | chr19:414347-425983 | 19p13.3 | No | 1.35E-04 |

| gene | class | locus | AGRE.Cases.Unrelated | Map.Position.March.2006. | Chr.Band | ACRD? | Combined.P |
|------|-------|-------|----------------------|--------------------------|----------|-------|------------|
| C1orf93 | gdups | 1 | 4 | chr1:2508097-2512762 | 1p36.32 | Yes | 0.03 |
| C1QTNF1 | edels | 163 | 8 | chr17:74531846-74557465 | 17q25.3 | No | 0.17 |
| C21orf51 | gdups | 201 | 3 | chr21:34669619-34683322 | 21q22.11 | No | 0.34 |
| C22orf25 | gdups | 203 | 3 | chr22:18384537-18433449 | 22q11.21 | Yes | 1.96E-03 |
| C22orf29 | gdups | 203 | 3 | chr22:18213661-18222419 | 22q11.21 | Yes | 1.96E-03 |
| C9orf28 | edups | 78 | 2 | chr9:128128949-128309140 | 9q33.3 | No | 0.17 |
| CA5A | edups | 152 | 3 | chr16:86479126-86527613 | 16q24.2 | Yes | 0.07 |
| CA6 | edels | 6 | 3 | chr1:8928509-8957733 | 1p36.23 | No | 0.03 |
| CACHD1 | edups | 13 | 3 | chr1:64709063-64931329 | 1p31.3 | No | 0.07 |
| CACNA2D4 | edups | 108 | 5 | chr12:1771384-1898131 | 12p13.33 | Yes | 0.01 |
| CAND2 | edels | 34 | 2 | chr3:12813171-12851301 | 3p25.1 | No | 0.17 |
| CARD11 | edups | 60 | 3 | chr7:2912295-3050105 | 7p22.2 | No | 0.19 |
| CARD9 | gdups | 81 | 5 | chr9:138378229-138387954 | 9q34.3 | Yes | 4.79E-03 |
| CBLN3 | gdups | 127 | 4 | chr14:23965582-23968571 | 14q12 | No | 0.03 |
| CBR1 | gdups | 202 | 2 | chr21:36364155-36367332 | 21q22.12 | Yes | 0.17 |
| CCL13 | gdups | 157 | 2 | chr17:29707584-29709741 | 17q12 | Yes | 0.68 |
| CD8A | gdups | 26 | 2 | chr2:86865240-86889030 | 2p11.2 | No | 0.07 |
| CDC45L | gdups | 203 | 3 | chr22:17846982-17888135 | 22q11.21 | Yes | 1.96E-03 |
| CDH17 | edels | 73 | 2 | chr8:95208566-95289986 | 8q22.1 | No | 0.17 |
| CEBPA | gdups | 184 | 3 | chr19:38482543-38485460 | 19q13.11 | Yes | 0.19 |
| CELSR1 | edups | 207 | 6 | chr22:45134397-45311731 | 22q13.31 | Yes | 4.79E-03 |
| CENPT | gdups | 150 | 3 | chr16:66419565-66425300 | 16q22.1 | No | 0.07 |
| CERK | edups | 207 | 3 | chr22:45458984-45512833 | 22q13.31 | Yes | 0.07 |
| CERK | gdups | 207 | 5 | chr22:45458984-45512833 | 22q13.31 | Yes | 0.01 |
| CGB1 | gdups | 191 | 2 | chr19:54230638-54231885 | 19q13.33 | No | 0.54 |
| CGB2 | gdups | 191 | 2 | chr19:54226942-54228307 | 19q13.33 | No | 0.54 |
| CGB5 | gdups | 191 | 2 | chr19:54238875-54240378 | 19q13.33 | No | 0.54 |
| CGB8 | gdups | 191 | 2 | chr19:54242709-54244212 | 19q13.33 | No | 0.54 |
| CGI-38 | gdups | 149 | 3 | chr16:65981213-65984922 | 16q22.1 | No | 0.07 |
| CHD1L | gdups | 15 | 2 | chr1:145180915-145234065 | 1q21.1 | Yes | 0.01 |
| CHD9 | edups | 146 | 2 | chr16:51646446-51918914 | 16q12.2 | No | 0.07 |
| CLCNKA | edels | 9 | 4 | chr1:16220953-16233132 | 1p36.13 | No | 0.03 |
| CLDN17 | gdups | 199 | 2 | chr21:30459753-30460945 | 21q21.3 | No | 0.17 |

| gene | class | locus | AGRE.Cases.Unrelated | Map.Position.March.2006. | Chr.Band | ACRD? | Combined.P |
|---|---|---|---|---|---|---|---|
| CLDN5 | gdups | 203 | 3 | chr22:17890547-17895068 | 22q11.21 | Yes | 1.96E-03 |
| CLDN8 | gdups | 199 | 2 | chr21:30508195-30510262 | 21q22.11 | No | 0.17 |
| CLTCL1 | gdups | 203 | 3 | chr22:17546989-17659239 | 22q11.21 | Yes | 1.96E-03 |
| COL16A1 | edups | 11 | 5 | chr1:31890435-31942507 | 1p35.2 | No | 0.05 |
| COL27A1 | edels | 77 | 2 | chr9:115957661-116114612 | 9q32 | Yes | 0.37 |
| COMT | gdups | 203 | 3 | chr22:18309256-18336539 | 22q11.21 | Yes | 1.96E-03 |
| CORO7 | gdups | 143 | 4 | chr16:4344546-4406572 | 16p13.3 | No | 0.03 |
| COX4I1 | gdups | 151 | 3 | chr16:84390697-84398109 | 16q24.1 | No | 0.07 |
| CPNE7 | edups | 153 | 3 | chr16:88169677-88191155 | 16q24.3 | No | 0.07 |
| CREB3L3 | gdups | 173 | 9 | chr1 9:4104629-4124050 | 19p13.3 | No | 3.30E-04 |
| CRELD2 | gdups | 208 | 3 | chr22:48698287-48707192 | 22q13.33 | Yes | 0.07 |
| CSTF2T | gdups | 86 | 2 | chr10:53125253-53129357 | 10q11.23 | Yes | 0.17 |
| CYBASC3 | gdups | 98 | 2 | chr1 1:60872856-60886305 | 11q12.2 | No | 0.17 |
| CYP4A22 | gdups | 12 | 2 | chr1:47375694-47388000 | 1p33 | No | 0.17 |
| CYP4F22 | edels | 178 | 3 | chr19:15497144-15524127 | 19p13.12 | No | 0.34 |
| CYP4X1 | gdups | 12 | 2 | chr1:47261827-47289009 | 1p33 | No | 0.17 |
| CYP4Z1 | gdups | 12 | 2 | chr1:47305634-47356577 | 1p33 | No | 0.17 |
| DACH1 | edels | 126 | 8 | chr1 3:70910099-71339331 | 13q21.33 | No | 0.24 |
| DAK | gdups | 98 | 4 | chr11:60857230-60872806 | 11q12.2 | No | 0.03 |
| DAPK3 | gdups | 173 | 7 | chr19:3909452-3920826 | 19p13.3 | No | 1.96E-03 |
| DAZAP1 | gdups | 169 | 8 | chr19:1358584-1386683 | 19p13.3 | Yes | 8.05E-04 |
| DBH | edups | 79 | 2 | chr9:135491306-135514287 | 9q34.2 | Yes | 0.17 |
| DDB1 | gdups | 98 | 3 | chr11:60823510-60857143 | 11q12.2 | No | 0.07 |
| DGCR14 | gdups | 203 | 3 | chr22:17497793-17512190 | 22q11.21 | Yes | 1.96E-03 |
| DGCR2 | gdups | 203 | 3 | chr22:17403795-17489967 | 22q11.21 | Yes | 1.96E-03 |
| DGCR8 | gdups | 203 | 3 | chr22:18447814-18479395 | 22q11.21 | Yes | 1.96E-03 |
| DGKB | edels | 64 | 8 | chr7:14151199-14909359 | 7p21.2 | Yes | 8.05E-04 |
| DHX29 | edels | 46 | 3 | chr5:54587831-54639278 | 5q11.2 | No | 0.19 |
| DIDO1 | gdups | 194 | 4 | chr20:60979535-61039719 | 20q13.33 | Yes | 0.03 |
| DKFZP686A10121 | edels | 67 | 2 | chr7:89813926-89854586 | 7q21.13 | No | 0.68 |
| DLGAP1 | edels | 165 | 4 | chr18:3486030-3870135 | 18p11.31 | No | 0.03 |
| DNAJC17 | edups | 134 | 7 | chr15:38847363-38886954 | 15q15.1 | No | 1.96E-03 |
| DOCK6 | gdups | 177 | 4 | chr19:11170973-11234157 | 19p13.2 | No | 0.03 |

| gene | class | locus | AGRE.Cases.Unrelated | Map.Position.March.2006. | Chr.Band | ACRD? | Combined.P |
|------|-------|-------|----------------------|--------------------------|----------|-------|------------|
| DPP10 | edels | 28 | 2 | chr2:114916346-116319798 | 2q14.1 | Yes | 0.17 |
| DSCR1 | gdups | 201 | 3 | chr21:34810654-34908615 | 21q22.12 | No | 0.34 |
| DUSP13 | edups | 87 | 2 | chr10:76524196-76538976 | 10q22.2 | No | 0.17 |
| E2F4 | gdups | 148 | 12 | chr16:65783569-65790322 | 16q22.1 | No | 2.27E-05 |
| EDG5 | gdups | 174 | 3 | chr19:10195520-10196581 | 19p13.2 | No | 0.07 |
| EEF2 | gdups | 173 | 7 | chr19:3927055-3936461 | 19p13.3 | No | 1.96E-03 |
| EFHA2 | edels | 69 | 2 | chr8:16929119-17024516 | 8p22 | No | 0.17 |
| ELMO3 | gdups | 148 | 12 | chr16:65790515-65795433 | 16q22.1 | No | 2.27E-05 |
| ELP4 | edels | 95 | 2 | chr11:31487873-31761903 | 11p13 | No | 0.94 |
| EPRS | edels | 18 | 2 | chr1:218208567-218286623 | 1q41 | No | 0.17 |
| ERGIC1 | edups | 49 | 4 | chr5:172193928-172312287 | 5q35.1 | No | 0.03 |
| FAM89B | edels | 101 | 3 | chr11:65096396-65098245 | 11q13.1 | No | 0.03 |
| FHOD1 | gdups | 148 | 13 | chr16:65820795-65838926 | 16q22.1 | No | 9.28E-06 |
| FKSG24 | gdups | 180 | 3 | chr19:18165040-18168550 | 19p13.11 | No | 0.07 |
| FLJ10379 | gdups | 23 | 2 | chr2:45469324-45691937 | 2p21 | Yes | 0.54 |
| FLJ12529 | edups | 99 | 6 | chr11:60926697-60953959 | 11q12.2 | No | 4.79E-03 |
| FLJ12949 | edups | 175 | 9 | chr1 9:10525267-10536683 | 19p13.2 | No | 3.30E-04 |
| FLJ14668 | gdups | 25 | 3 | chr2:70376612-70382724 | 2p14 | Yes | 0.07 |
| FLJ21865 | edels | 163 | 3 | chr17:74582614-74596276 | 17q25.3 | No | 0.93 |
| FLJ38991 | gdups | 42 | 3 | chr4:74140668-74154286 | 4q13.3 | Yes | 0.07 |
| FLJ41603 | edups | 48 | 3 | chr5:148941328-148994720 | 5q33.1 | No | 0.07 |
| FLJ41993 | gdups | 208 | 3 | chr22:48775069-48793182 | 22q13.33 | Yes | 0.07 |
| FLJ43860 | edups | 74 | 3 | chr8:142513111-142586512 | 8q24.3 | No | 0.07 |
| FLJ44894 | edels | 181 | 2 | chr19:20366360-20399602 | 19p12 | No | 0.94 |
| FLRT1 | gdups | 100 | 6 | chr11:63627938-63643221 | 11q13.1 | No | 4.79E-03 |
| FLYWCH1 | edels | 141 | 3 | chr16:2901981-2941209 | 16p13.3 | No | 4.79E-03 |
| FMO5 | gdups | 15 | 2 | chr1:145124462-145163569 | 1q21.1 | Yes | 0.01 |
| FUT10 | edups | 71 | 3 | chr8:33347884-33450206 | 8p12 | No | 0.07 |
| GABRA5 | gdups | 132 | 5 | chr15:24663151-24777095 | 15q12 | Yes | 2.27E-05 |
| GABRB3 | gdups | 132 | 6 | chr15:24339786-24767329 | 15q12 | Yes | 3.79E-06 |
| GABRG3 | gdups | 132 | 5 | chr15:24799263-25451622 | 15q12 | Yes | 5.54E-05 |
| GALNT13 | edels | 31 | 4 | chr2:154436672-155018734 | 2q23.3 | Yes | 0.01 |
| GAMT | gdups | 169 | 8 | chr19:1348089-1352552 | 19p13.3 | Yes | 8.05E-04 |

| gene | class | locus | AGRE.Cases.Unrelated | Map.Position.March.2006. | Chr.Band | ACRD? | Combined.P |
|------|-------|-------|----------------------|--------------------------|----------|-------|------------|
| GEMIN4 | edups | 154 | 2 | chr17:594411-602251 | 17p13.3 | No | 0.17 |
| GGN | gdups | 186 | 4 | chr19:43566745-43570508 | 19q13.2 | No | 0.03 |
| GJA8 | gdups | 15 | 3 | chr1:145841560-145848017 | 1q21.1 | Yes | 0.03 |
| GMPS | edels | 38 | 3 | chr3:157071019-157138215 | 3q25.31 | No | 0.07 |
| GNA11 | gdups | 171 | 6 | chr19:3045408-3072452 | 19p13.3 | Yes | 4.79E-03 |
| GNB1L | gdups | 203 | 3 | chr22:18150747-18222462 | 22q11.21 | Yes | 1.96E-03 |
| GNG7 | edups | 170 | 2 | chr19:2465451-2506186 | 19p13.3 | No | 0.07 |
| GOLGA8E | gdups | 130 | 2 | chr15:20986537-20999858 | 15q11.2 | Yes | 0.17 |
| GPR146 | gdups | 62 | 2 | chr7:1061447-1065423 | 7p22.3 | Yes | 0.37 |
| GPR89A | gdups | 15 | 3 | chr1:144475774-144538430 | 1q21.1 | No | 0.03 |
| GRIK5 | edels | 188 | 3 | chr19:47194317-47261797 | 19q13.2 | No | 0.71 |
| GSCL | gdups | 203 | 3 | chr22:17516504-17517796 | 22q11.21 | Yes | 1.96E-03 |
| GYG2 | edels | 209 | 2 | chrX:2756859-2810858 | Xp22.33 | Yes | 0.17 |
| GYG2 | gdups | 209 | 2 | chrX:2756859-2810858 | Xp22.33 | Yes | 0.17 |
| HES7 | gdups | 155 | 11 | chr17:7965024-7968127 | 17p13.1 | No | 5.54E-05 |
| HIRA | gdups | 203 | 3 | chr22:17698224-17799219 | 22q11.21 | Yes | 1.96E-03 |
| HNF4G | edels | 72 | 2 | chr8:76482732-76641600 | 8q21.11 | No | 0.17 |
| HPCAL1 | edups | 22 | 4 | chr2:10360491-10485193 | 2p25.1 | No | 0.01 |
| HSD11B2 | gdups | 149 | 3 | chr16:66022537-66028953 | 16q22.1 | No | 0.07 |
| HSPC171 | gdups | 148 | 13 | chr16:65818517-65820683 | 16q22.1 | No | 9.28E-06 |
| HTF9C | gdups | 203 | 3 | chr22:18479398-18484768 | 22q11.21 | Yes | 1.96E-03 |
| IFI30 | gdups | 180 | 4 | chr19:18145579-18149927 | 19p13.11 | No | 0.03 |
| INHBB | gdups | 30 | 3 | chr2:120820189-120825853 | 2q14.2 | No | 0.07 |
| ITGB1BP3 | gdups | 173 | 7 | chr19:3884101-3893412 | 19p13.3 | No | 1.96E-03 |
| KCNAB2 | edups | 3 | 5 | chr1:5974113-6083840 | 1p36.31 | No | 4.79E-03 |
| KCNE1 | gdups | 201 | 3 | chr21:34740858-34806443 | 21q22.12 | No | 0.34 |
| KCNE2 | gdups | 201 | 3 | chr21:34658193-34665307 | 21q22.11 | No | 0.34 |
| KCNH7 | edups | 32 | 2 | chr2:162936163-163403274 | 2q24.2 | No | 0.17 |
| KCNJ14 | gdups | 190 | 3 | chr19:53650578-53661179 | 19q13.32 | No | 0.07 |
| KCNQ1 | edels | 91 | 3 | chr11:2422797-2826915 | 11p15.5 | No | 0.34 |
| KCTD19 | gdups | 149 | 3 | chr16:65880894-65918162 | 16q22.1 | No | 0.07 |
| KCTD5 | edups | 140 | 5 | chr16:2672499-2699030 | 16p13.3 | No | 0.01 |
| KIAA0195 | gdups | 160 | 9 | chr17:70964317-71007758 | 17q25.1 | No | 3.30E-04 |

| gene | class | locus | AGRE.Cases.Unrelated | Map.Position.March.2006. | Chr.Band | ACRD? | Combined.P |
|------|-------|-------|----------------------|--------------------------|----------|-------|------------|
| KIAA0319 | edups | 52 | 5 | chr6:24652311-24754362 | 6p22.2 | No | 0.01 |
| KIAA0528 | edels | 113 | 2 | chr12:22492808-22588719 | 12p12.1 | No | 0.78 |
| KIAA1086 | gdups | 173 | 3 | chr19:3755022-3820027 | 19p13.3 | No | 0.07 |
| KIAA1586 | edels | 54 | 3 | chr6:57019343-57027951 | 6p12.1 | No | 0.01 |
| KIAA1856 | edups | 61 | 4 | chr7:5312949-5429703 | 7p22.1 | No | 0.10 |
| KLHL22 | gdups | 203 | 3 | chr22:19125806-19180122 | 22q11.21 | Yes | 0.02 |
| KREMEN2 | edels | 141 | 3 | chr16:2954218-2958381 | 16p13.3 | No | 1.96E-03 |
| KRTAP13-2 | gdups | 199 | 2 | chr21:30665580-30666446 | 21q22.11 | Yes | 0.17 |
| KRTAP23-1 | gdups | 199 | 2 | chr21:30642598-30642795 | 21q22.11 | Yes | 0.17 |
| KRTAP24-1 | gdups | 199 | 2 | chr21:30575498-30577147 | 21q22.11 | Yes | 0.17 |
| KRTAP26-1 | gdups | 199 | 2 | chr21:30613313-30614505 | 21q22.11 | Yes | 0.17 |
| KRTAP27-1 | gdups | 199 | 2 | chr21:30631202-30631883 | 21q22.11 | Yes | 0.17 |
| KRTHB1 | gdups | 114 | 3 | chr12:50965964-50971566 | 12q13.13 | No | 0.19 |
| LAMA2 | edels | 57 | 2 | chr6:129245979-129879401 | 6q22.33 | Yes | 0.17 |
| LFNG | gdups | 59 | 6 | chr7:2518689-2535334 | 7p22.2 | No | 4.79E-03 |
| LILRA3 | gdups | 192 | 4 | chr19:59491666-59501764 | 19q13.42 | No | 0.64 |
| LILRA5 | gdups | 192 | 4 | chr19:59510165-59516221 | 19q13.42 | No | 0.64 |
| LMTK3 | gdups | 190 | 4 | chr19:53680340-53708258 | 19q13.32 | No | 0.03 |
| LOC128977 | gdups | 203 | 3 | chr22:17808417-17815220 | 22q11.21 | Yes | 1.96E-03 |
| LOC150383 | gdups | 207 | 3 | chr22:45018574-45024857 | 22q13.31 | No | 0.07 |
| LOC162073 | edels | 145 | 3 | chr16:19032783-19040453 | 16p12.3 | No | 0.07 |
| LOC283849 | gdups | 148 | 12 | chr16:65767006-65775384 | 16q22.1 | No | 2.27E-05 |
| LOC285498 | gdups | 39 | 3 | chr4:1056544-1097350 | 4p16.3 | Yes | 0.03 |
| LOC388910 | gdups | 205 | 2 | chr22:43343883-43346993 | 22q13.31 | No | 0.17 |
| LOC389852 | gdups | 211 | 3 | chrX:47871547-47876941 | Xp11.23 | Yes | 0.07 |
| LOC650137 | edels | 129 | 26 | chr15:19915066-19915749 | 15q11.2 | Yes | 3.31E-11 |
| LOC653319 | gdups | 148 | 10 | chr16:65775770-65781608 | 16q22.1 | No | 1.35E-04 |
| LOC728489 | gdups | 81 | 5 | chr9:138376173-138378062 | 9q34.3 | Yes | 4.79E-03 |
| LOC728912 | gdups | 15 | 3 | chr1:146040948-146076705 | 1q21.1 | Yes | 0.01 |
| LOC728932 | gdups | 15 | 3 | chr1:145907791-145932379 | 1q21.1 | Yes | 0.01 |
| LOC93343 | gdups | 179 | 5 | chr19:17393714-17397140 | 19p13.11 | No | 0.01 |
| LRBA | edels | 43 | 2 | chr4:151405044-152156329 | 4q31.3 | No | 0.68 |
| LRP3 | gdups | 184 | 3 | chr19:38377330-38390383 | 19q13.11 | Yes | 0.07 |

(continued)

| gene | class | locus | AGRE.Cases.Unrelated | Map.Position.March.2006. | Chr.Band | ACRD? | Combined.P |
|---|---|---|---|---|---|---|---|
| LRP5 | edups | 104 | 3 | chr11:67836684-67973315 | 11q13.2 | No | 0.07 |
| LRRC27 | edups | 89 | 2 | chr10:133995648-134109058 | 10q26.3 | No | 0.17 |
| LRRC29 | gdups | 148 | 12 | chr16:65798543-65818414 | 16q22.1 | No | 2.27E-05 |
| LRRC36 | gdups | 149 | 3 | chr16:65918248-65976604 | 16q22.1 | No | 0.07 |
| LRTM2 | gdups | 109 | 3 | chr12:1799956-1816179 | 12p13.33 | Yes | 0.07 |
| LYG1 | gdups | 27 | 3 | chr2:99267134-99287637 | 2q11.2 | Yes | 0.19 |
| LYG2 | gdups | 27 | 3 | chr2:99225141-99238034 | 2q11.2 | Yes | 0.19 |
| MADCAM1 | edels | 167 | 3 | chr19:447490-456340 | 19p13.3 | No | 5.54E-05 |
| MAGEA11 | gdups | 212 | 2 | chrX:148575479-148603920 | Xq28 | Yes | 0.17 |
| MAGEL2 | gdups | 131 | 5 | chr15:21439789-21442268 | 15q11.2 | Yes | 9.28E-06 |
| MAP2K2 | gdups | 173 | 7 | chr19:4041319-4075126 | 19p13.3 | No | 1.96E-03 |
| MAPK8IP1 | gdups | 96 | 5 | chr1 1:45863778-45884592 | 11p11.2 | No | 0.01 |
| MAST4 | edels | 47 | 2 | chr5:65927932-66501179 | 5q12.3 | No | 0.17 |
| MATK | gdups | 173 | 3 | chr19:3728968-3752810 | 19p13.3 | No | 0.07 |
| MDGA2 | edels | 128 | 8 | chr14:46379045-47213703 | 14q21.3 | No | 1.35E-04 |
| METAP2 | edups | 117 | 2 | chr12:94391953-94433746 | 12q22 | No | 0.17 |
| MGC10992 | edups | 147 | 3 | chr1 6:56103591-56127978 | 16q13 | No | 0.07 |
| MGC11335 | gdups | 150 | 3 | chrl 6:66265940-66310720 | 16q22.1 | No | 0.07 |
| MGMT | edups | 88 | 4 | chr10:131155510-131455356 | 10q26.3 | Yes | 0.03 |
| MIOX | gdups | 208 | 4 | chr22:49272079-49275943 | 22q13.33 | Yes | 0.03 |
| MKRN3 | gdups | 131 | 5 | chr15:21361547-21364653 | 15q11.2 | Yes | 9.28E-06 |
| MOCOS | edups | 166 | 3 | chr18:32021478-32102682 | 18q12.2 | No | 0.07 |
| MPDZ | edels | 75 | 2 | chr9:13095703-13269563 | 9p23 | Yes | 0.37 |
| MRPL40 | gdups | 203 | 3 | chr22:17800036-17803594 | 22q11.21 | Yes | 1.96E-03 |
| MRPL54 | gdups | 173 | 3 | chr19:3713665-3718562 | 19p13.3 | No | 0.07 |
| MSMB | gdups | 84 | 2 | chr10:51219559-51232596 | 10q11.23 | Yes | 0.17 |
| MUM1 | gdups | 169 | 4 | chr19:1300175-1329427 | 19p13.3 | Yes | 0.03 |
| MYLK2 | gdups | 193 | 2 | chr20:29870772-29886153 | 20q11.21 | No | 0.17 |
| NBPF11 | gdups | 15 | 3 | chr1:146040948-146076705 | 1q21.1 | Yes | 0.01 |
| NCOA4 | gdups | 84 | 2 | chr10:51235233-51260740 | 10q11.23 | Yes | 0.17 |
| NDN | gdups | 131 | 5 | chr15:21481916-21483570 | 15q11.2 | Yes | 2.27E-05 |
| NDUFS7 | gdups | 169 | 4 | chr19:1334906-1346583 | 19p13.3 | Yes | 0.03 |
| NEK3 | edups | 124 | 2 | chr13:51604780-51631997 | 13q14.3 | No | 0.17 |

| gene | class | locus | AGRE.Cases.Unrelated | Map.Position.March.2006. | Chr.Band | ACRD? | Combined.P |
|------|-------|-------|----------------------|--------------------------|----------|-------|------------|
| NFIC | edups | 172 | 3 | chr19:3310616-3414603 | 19p13.3 | Yes | 0.07 |
| NRXN1 | edels | 24 | 5 | chr2:50000992-51113178 | 2p16.3 | Yes | 3.30E-04 |
| NUP210 | edups | 35 | 2 | chr3:13332737-13436809 | 3p25.1 | No | 0.17 |
| NUTF2 | gdups | 150 | 3 | chr16:66438331-66462727 | 16q22.1 | No | 0.07 |
| OBSCN | edels | 19 | 2 | chr1:226462484-226633198 | 1q42.13 | No | 0.54 |
| OCA2 | gdups | 132 | 5 | chr15:25673622-26018053 | 15q13.1 | Yes | 2.27E-05 |
| OPRD1 | edups | 10 | 5 | chr1:29011241-29062795 | 1p35.3 | No | 0.01 |
| OR1C1 | edels | 21 | 3 | chr1:245987387-245988331 | 1q44 | No | 0.03 |
| OR2AG1 | edels | 94 | 3 | chr11:6762845-6763795 | 11p15.4 | No | 0.07 |
| OR2AG2 | edels | 94 | 3 | chr11:6745814-6746764 | 11p15.4 | No | 0.07 |
| OR4C6 | gdups | 97 | 4 | chr11:55186202-55190738 | 11q11 | No | 0.54 |
| OR4M2 | edels | 129 | 26 | chr15:19869940-19870881 | 15q11.2 | Yes | 3.31E-11 |
| OR4N4 | edels | 129 | 26 | chr15:19804548-19885172 | 15q11.2 | Yes | 1.35E-11 |
| OR4S2 | gdups | 97 | 9 | chr11:55174956-55175891 | 11q11 | No | 0.07 |
| OSBPL5 | edups | 93 | 4 | chr11:3064922-3143116 | 11p15.4 | No | 0.03 |
| PAMCI | edels | 116 | 2 | chr12:84722462-84754449 | 12q21.31 | No | 0.17 |
| PAQR4 | edels | 141 | 3 | chr16:2959343-2963484 | 16p13.3 | No | 1.96E-03 |
| PCDH9 | edels | 125 | 2 | chr13:65774970-66702578 | 13q21.32 | Yes | 0.17 |
| PCQAP | gdups | 203 | 3 | chr22:19191886-19248975 | 22q11.21 | Yes | 4.79E-03 |
| PI4KA | gdups | 203 | 3 | chr22:19391981-19543070 | 22q11.21 | Yes | 1.96E-03 |
| PIK3R2 | gdups | 180 | 5 | chr19:18125016-18142343 | 19p13.11 | No | 0.01 |
| PIM3 | gdups | 208 | 3 | chr22:48740165-48743721 | 22q13.33 | Yes | 0.07 |
| PIP5K1C | gdups | 173 | 7 | chr19:3581182-3651445 | 19p13.3 | No | 1.96E-03 |
| PKMYT1 | edels | 141 | 3 | chr16:2962793-2970506 | 16p13.3 | No | 1.96E-03 |
| PLA2G4C | edups | 189 | 2 | chr19:53242916-53305865 | 19q13.32 | No | 0.17 |
| PLEKHG4 | gdups | 148 | 12 | chr16:65868914-65880883 | 16q22.1 | No | 2.27E-05 |
| PLEKHG5 | edups | 4 | 4 | chr1:6448739-6502708 | 1p36.31 | No | 0.03 |
| PLEKHM2 | edels | 8 | 3 | chr1:15883414-15933849 | 1p36.21 | No | 0.34 |
| POSTN | edels | 122 | 3 | chr13:37034779-37070874 | 13q13.3 | No | 0.07 |
| PP2447 | edups | 208 | 5 | chr22:48966487-48980154 | 22q13.33 | Yes | 0.01 |
| PP2447 | gdups | 208 | 8 | chr22:48966487-48980154 | 22q13.33 | Yes | 8.05E-04 |
| PPME1 | edups | 105 | 4 | chr11:73619081-73643395 | 11q13.4 | No | 0.03 |
| PRB3 | edels | 110 | 3 | chr12:11311393-11313908 | 12p13.2 | No | 0.07 |

| gene | class | locus | AGRE.Cases.Unrelated | Map.Position.March.2006. | Chr.Band | ACRD? | Combined.P |
|------|-------|-------|----------------------|---------------------------|----------|-------|------------|
| PRDM10 | edups | 107 | 3 | chr11:129274817-129377940 | 11q24.3 | No | 0.07 |
| PRIC285 | edups | 195 | 3 | chr20:61659883-61676036 | 20q13.33 | Yes | 0.03 |
| PRKAB2 | gdups | 15 | 2 | chr1:145093314-145110753 | 1q21.1 | Yes | 0.01 |
| PRKG1 | edels | 85 | 2 | chr10:52421124-53728116 | 10q11.23 | Yes | 0.17 |
| PROP1 | gdups | 51 | 3 | chr5:177351842-177355849 | 5q35.3 | No | 0.07 |
| PRR5 | edups | 206 | 3 | chr22:43443257-43637329 | 22q13.31 | No | 0.07 |
| PSCD2 | gdups | 190 | 4 | chr19:53664424-53674457 | 19q13.32 | No | 0.03 |
| PSKH1 | gdups | 150 | 4 | chr16:66484705-66521078 | 16q22.1 | No | 0.03 |
| PSMD8 | gdups | 186 | 2 | chr19:43557016-43566304 | 19q13.2 | No | 0.17 |
| QSOX2 | edups | 80 | 2 | chr9:138238006-138277508 | 9q34.3 | Yes | 0.07 |
| RAB35 | edups | 118 | 3 | chr12:119017289-119038982 | 12q24.23 | Yes | 0.07 |
| RAB39 | gdups | 106 | 2 | chr11:107304487-107339416 | 11q22.3 | No | 0.37 |
| RABGAP1L | edels | 16 | 2 | chr1:172395171-173226353 | 1q25.1 | No | 0.99 |
| RAI1 | edups | 156 | 5 | chr17:17525512-17655492 | 17p11.2 | Yes | 0.01 |
| RANBP1 | gdups | 203 | 3 | chr22:18484947-18494878 | 22q11.21 | Yes | 1.96E-03 |
| RANBP10 | gdups | 150 | 3 | chr16:66314506-66398056 | 16q22.1 | No | 0.07 |
| RAX2 | gdups | 173 | 3 | chr19:3448813-3723219 | 19p13.3 | No | 0.07 |
| RCD-8 | gdups | 150 | 4 | chr16:66464500-66475907 | 16q22.1 | No | 0.03 |
| RNF111 | edups | 136 | 3 | chr15:57067157-57176541 | 15q22.1 | Yes | 0.07 |
| RNF133 | edels | 68 | 3 | chr7:122125078-122126208 | 7q31.32 | Yes | 0.03 |
| RNF148 | edels | 68 | 3 | chr7:122128956-122130257 | 7q31.32 | Yes | 0.03 |
| RNF44 | edups | 50 | 5 | chr5:175886306-175897027 | 5q35.2 | No | 0.05 |
| RPS15 | gdups | 169 | 8 | chr19:1389363-1391492 | 19p13.3 | Yes | 8.05E-04 |
| RPS19 | edups | 187 | 3 | chr19:47055828-47067322 | 19q13.2 | No | 0.07 |
| RYR2 | edups | 20 | 3 | chr1:235272128-236063911 | 1q43 | No | 0.34 |
| SBF1 | gdups | 208 | 4 | chr22:49232050-49260330 | 22q13.33 | Yes | 0.03 |
| SETD4 | gdups | 202 | 2 | chr21:36328709-36373557 | 21q22.12 | Yes | 0.17 |
| SH3TC1 | edups | 41 | 2 | chr4:8251960-8293725 | 4p16.1 | No | 0.07 |
| SIRT4 | edups | 119 | 2 | chr12:119224546-119235430 | 12q24.31 | Yes | 0.17 |
| SKIV2L2 | edels | 46 | 6 | chr5:54639594-54757163 | 5q11.2 | No | 0.02 |
| SLC16A5 | edups | 159 | 3 | chr17:70595650-70613841 | 17q25.1 | No | 0.07 |
| SLC18A1 | edels | 70 | 2 | chr8:20046652-20084997 | 8p21.3 | No | 0.17 |
| SLC22A18 | edups | 92 | 3 | chr11:2877527-2903052 | 11p15.4 | No | 0.03 |

(continued)

| gene | class | locus | AGRE.Cases.Unrelated | Map.Position.March.2006. | Chr.Band | ACRD? | Combined.P |
|------|-------|-------|---------------------|--------------------------|----------|-------|------------|
| SLC25A1 | gdups | 203 | 3 | chr22:17543092-17546260 | 22q11.21 | Yes | 1.96E-03 |
| SLC25A34 | edels | 8 | 3 | chr1:15935396-15940471 | 1p36.21 | No | 0.34 |
| SLC26A11 | gdups | 164 | 3 | chr17:75808824-75841890 | 17q25.3 | No | 0.07 |
| SLC28A1 | edups | 137 | 2 | chr15:83228913-83290033 | 15q25.3 | Yes | 0.17 |
| SLC2A4RG | gdups | 196 | 3 | chr20:61841655-61845846 | 20q13.33 | Yes | 0.07 |
| SLC45A1 | edups | 5 | 2 | chr1:8300756-8326814 | 1p36.23 | No | 0.17 |
| SLC6A15 | edels | 115 | 3 | chr12:83777402-83830705 | 12q21.31 | No | 0.19 |
| SLC7A10 | gdups | 184 | 3 | chr19:38391410-38408596 | 19q13.11 | Yes | 0.07 |
| SLC9A5 | gdups | 148 | 12 | chr16:65840356-65863594 | 16q22.1 | No | 2.27E-05 |
| SLCO1A2 | edels | 112 | 3 | chr12:21311651-21439638 | 12p12.1 | No | 0.98 |
| SLCO1B3 | edups | 111 | 2 | chr12:20854905-20960925 | 12p12.2 | No | 0.17 |
| SMARCA4 | edups | 176 | 2 | chr19:10932606-11033953 | 19p13.2 | No | 0.17 |
| SNRPN | gdups | 132 | 5 | chr15:22619887-22776293 | 15q11.2 | Yes | 9.28E-06 |
| SNURF | gdups | 132 | 5 | chr15:22652824-22770696 | 15q11.2 | Yes | 9.28E-06 |
| SNX25 | edups | 44 | 2 | chr4:186368278-186527942 | 4q35.1 | Yes | 0.17 |
| SPACA5B | gdups | 211 | 3 | chrX:47875014-47876939 | Xp11.23 | Yes | 0.07 |
| SPON2 | edels | 40 | 5 | chr4:1150725-1156602 | 4p16.3 | Yes | 0.11 |
| SPRED3 | gdups | 186 | 4 | chr19:43572779-43578711 | 19q13.2 | No | 0.03 |
| SPRN | gdups | 90 | 2 | chr10:135084160-135088111 | 10q26.3 | Yes | 0.37 |
| SRL | edups | 142 | 3 | chr16:4179378-4232077 | 16p13.3 | No | 0.07 |
| SSSCA1 | edels | 101 | 3 | chr11:65094519-65095793 | 11q13.1 | No | 0.03 |
| SSX5 | gdups | 211 | 3 | chrX:47930600-47941143 | Xp11.23 | Yes | 0.07 |
| STEAP3 | edups | 29 | 2 | chr2:119697854-119739698 | 2q14.2 | No | 0.37 |
| STIP1 | gdups | 100 | 5 | chr11:63709873-63728596 | 11q13.1 | No | 0.01 |
| SUCLG2 | edels | 37 | 2 | chr3:67507835-67787728 | 3p14.1 | Yes | 0.07 |
| SYNGR2 | gdups | 162 | 3 | chr17:73676266-73680604 | 17q25.3 | No | 0.07 |
| TCP10L | gdups | 200 | 3 | chr21:32870733-32879714 | 21q22.11 | No | 0.07 |
| THAP11 | gdups | 150 | 3 | chr16:66433714-66435598 | 16q22.1 | No | 0.07 |
| TJP3 | gdups | 173 | 5 | chr19:3672735-3701807 | 19p13.3 | No | 0.01 |
| TMEM112 | gdups | 138 | 3 | chr16:843635-960985 | 16p13.3 | Yes | 0.07 |
| TMEM138 | gdups | 98 | 2 | chr11:60886432-60893254 | 11q12.2 | No | 0.17 |
| TNFRSF14 | gdups | 1 | 4 | chr1:2479153-2486757 | 1p36.32 | Yes | 0.03 |
| TNFRSF8 | edups | 7 | 3 | chr1:12046021-12126851 | 1p36.22 | No | 0.07 |

EP 2 376 655 B1

(continued)

| gene | class | locus | AGRE.Cases.Unrelated | Map.Position.March.2006. | Chr.Band | ACRD? | Combined.P |
|------|-------|-------|---------------------|--------------------------|----------|-------|------------|
| TPPP | edels | 45 | 8 | chr5:712978-746510 | 5p15.33 | Yes | 4.61E-03 |
| TRPM1 | gdups | 133 | 2 | chr15:29080845-29181216 | 15q13.3 | Yes | 0.37 |
| TRPT1 | gdups | 100 | 5 | chr11:63747848-63750257 | 11q13.1 | No | 0.01 |
| TSC2 | edups | 139 | 2 | chr16:2037991-2078713 | 16p13.3 | No | 0.17 |
| TSNAXIP1 | gdups | 150 | 3 | chr16:66398511-66419471 | 16q22.1 | No | 0.07 |
| TSSK2 | gdups | 203 | 3 | chr22:17498790-17500134 | 22q11.21 | Yes | 1.96E-03 |
| TXNRD2 | gdups | 203 | 3 | chr22:18243040-18309359 | 22q11.21 | Yes | 1.96E-03 |
| UBE2O | edups | 161 | 4 | chr17:71897491-71960883 | 17q25.1 | No | 0.03 |
| UBE3A | gdups | 132 | 5 | chr15:23133489-23235221 | 15q11.2 | Yes | 9.28E-06 |
| UBR1 | edups | 135 | 2 | chr15:41022398-41185578 | 15q15.2 | No | 0.37 |
| UFD1L | gdups | 203 | 3 | chr22:17817701-17846726 | 22q11.21 | Yes | 1.96E-03 |
| UGT1A5 | edels | 33 | 2 | chr2:234191093-234346688 | 2q37.1 | No | 0.17 |
| UNC93B1 | edels | 103 | 3 | chr11:67515151-67528169 | 11q13.2 | No | 0.03 |
| UNCX4.1 | gdups | 63 | 3 | chr7:1239180-1242734 | 7p22.3 | Yes | 0.07 |
| UNQ2446 | gdups | 150 | 4 | chr16:66476282-66477772 | 16q22.1 | No | 0.03 |
| URP2 | gdups | 100 | 5 | chr11:63730782-63747939 | 11q13.1 | No | 0.01 |
| USH2A | edels | 17 | 3 | chr1:213862859-214663361 | 1q41 | Yes | 0.07 |
| UTRN | edups | 58 | 2 | chr6:144654566-145215859 | 6q24.2 | Yes | 0.17 |
| VCX2 | gdups | 210 | 3 | chrX:8097985-8099308 | Xp22.31 | No | 0.07 |
| VPS37B | gdups | 120 | 4 | chr12:121915835-121946665 | 12q24.31 | Yes | 0.03 |
| WASF3 | edups | 121 | 5 | chr13:26029840-26161080 | 13q12.13 | No | 0.05 |
| WDR78 | edups | 14 | 3 | chr1:67051162-67163158 | 1p31.3 | No | 0.07 |
| WNT7A | edups | 36 | 3 | chr3:13835085-13896619 | 3p25.1 | No | 0.07 |
| XG | edels | 209 | 2 | chrX:2680115-2743955 | Xp22.33 | Yes | 0.17 |
| ZC3H7B | edups | 204 | 5 | chr22:40027475-40086053 | 22q13.2 | Yes | 0.01 |
| ZDHHC1 | gdups | 149 | 3 | chr16:65985829-66007878 | 16q22.1 | No | 0.07 |
| ZNF208 | gdups | 182 | 2 | chr19:21940737-21985561 | 19p12 | Yes | 0.54 |
| ZNF257 | edels | 183 | 2 | chr19:22027106-22064084 | 19p12 | Yes | 0.37 |
| ZNF37A | gdups | 82 | 2 | chr10:38423281-38452282 | 10p11.21 | No | 0.17 |
| ZNF676 | edels | 183 | 2 | chr19:22153743-22171593 | 19p12 | Yes | 0.17 |
| ZNF74 | gdups | 203 | 3 | chr22:19078418-19092752 | 22q11.21 | Yes | 0.02 |

While certain preferred embodiments of the present invention have been described and specifically exemplified above, it is not intended that the invention be limited to such embodiments.

**Claims**

1. A method for detecting a propensity for developing a neurological disorder, the method comprising: detecting the presence of at least one copy number variation (CNV) in a target polynucleotide obtained from a patient wherein if said CNV is present, said patient has an increased risk for developing a neurological disorder, wherein said deletion containing CNV is BZRAP1 Benzodiazapine receptor (peripheral) associated protein 1 17q22-q23 chrl7:53733592-53761151; and wherein the neurological disorder is autism or autism spectrum disorder.

2. A method according to claim 1 comprising detecting the presence of at least one further deletion containing CNV selected from
MDGA2 MAM domain containing glycosylphosphatidylinositol anchor 214q21.3 chr14:46,170,380-47,422,368,
CLCNKA chloride channel Ka chr1: 16221072-16233132,
NTRK1 Neurotrophic tyrosine kinase, receptor, type 1 1q21-q22 chr1: 155,013,407-155,202,154, USH2A Usher syndrome 2A (autosomal recessive, mild) 1q41 chr1:213,752,880-214,875,391,
NRXN1 Neurexin 1 2p16.3 chr2:49,712,184-51,360,413,
GALNT13 UDP-N-acetyl-alpha-D-galactosamine:polypeptide N-acetylgal 2q23.3-q24.1 chr2:153,854,689-155,600,757,
GMPS Guanine monophosphate synthetase 3q24 chr3:157,059,820-157,149,414,
SPON2 Spondin 2, extracellular matrix protein 4p16.3 chr4:1,124,285-1,183,034,
LRBA LPS-responsive vesicle trafficking, beach and anchor containin4q31.3 chr4: 151,217,225-152,344,150,
TPPP Tubulin polymerization promoting protein 5p15.3 chr5:567,501-892,810,
SKIV2L2 Superkiller viralicidic activity 2-like 2 (S. cerevisiae) 5q11.2 chr5:54,522, 183-54,873,752,
KIAA1586 KIAA1586 6p12.1 chr6:56,980,593-57,066,702,
BTN2A1 Butyrophilin, subfamily 2, member A1 6p22.1 chr6:26566167-26577844,
BXDC1 Brix domain containing 1 6q21 chr6:111409983-111453487,
LAMA2 Laminin, alpha 2 (merosin, congenital muscular dystrophy) 6q22-q23 chr6:128,945,101-130,370,307,
DGKB Diacylglycerol kinase, beta 90kDa 7p21.2 chr7:14,015,810-15,013,734,

RNF133 Ring finger protein 133 7q31.32 chr7:122,118,508-122,132,937,
RNF148 Ring finger protein 148 7q31.33 chr7:122,118,508-122,132,937,
SLC18A1 Solute carrier family 18 (vesicular monoamine), member 1 8p21.3 chr8:19,874,095-20,257,554,
COL27A1 Collagen, type XXVII, alpha 1 9q32 chr9:115958051-116112796,
OR2AG1 Olfactory receptor, family 2, subfamily AG, member 1 11p15.4 chr11:6762845-6763795,
OR2AG2 Olfactory receptor, family 2, subfamily AG, member 2 11p15.4 chr11:6745814-6746764,
SSSCA1 Sjogren syndrome/scleroderma autoantigen 1 11q13.1 chr11:65094518-65095815,
FAM89B Family with sequence similarity 89, member B 11q23 chr11:65,094,554-65,100,079,
PRB3 Proline-rich protein BstNI subfamily 3 12p13.2 chr12:11310124-11313908,
KRT3 Keratin 3 12q12-q13 chr12:51,444,040-51,501,855,
SLC6A15 Solute carrier family 6, member 15 12q21.3 chr12:83,670,976-83,958,489,
DACH1 Dachshund homolog 1 (Drosophila) 13q22 chr13:70910098-71339331,
LOC650137 Seven transmembrane helix receptor 15q11.2 chr15:19,812,808-20,018,007,
OR4M2 Olfactory receptor, family 4, subfamily M, member 2 15q11.2 chr15:19,812,808-20,018,007,
OR4N4 Hypothetical LOC727924 15q11.2 chr15:19,812,808-20,018,007,
LOC162073 hypothetical protein LOC162073 16p12.3 chr16:19,008,005-19,060,144,
DLGAP1 Discs, large (Drosophila) homolog-associated protein 1 18p11.3 chr18:3,393,512-3,965,460,
FLJ44894 Homo sapiens cDNA FLJ44894 fis, clone BRAMY3000692, m 19p12 chr19:20,227,461-20,491 ,547,
CYP4F22 Cytochrome P450, family 4, subfamily F, polypeptide 22 19p13.12 chr19: 15480335-15524128,
GRIK5 Glutamate receptor, ionotropic, kainate 5 19q13.2 chr19:47,126,828-47,329,282,
GYG2 Glycogenin 2 Xp22.3 chrX:2,656,547-2,925,352,
XG Xg pseudogene, Y-linked 2 Xp22.33 chrX:2,656,547-2,925,353,
FGF13 Fibroblast growth factor 13 Xq26.3 chrX:137,421,326-138,459,367,
SPANXB1 SPANX family, member B2 Xq27.1 chrX: 139,908,245-139,941,724, and
SPANXB2 SPANX family, member B2 Xq27.1 chrX: 139,908,245-139,941,724; or the group of CNVs consisting of CNVs set forth in Table 6.

3. The method of claim 1 or claim 2, wherein the target nucleic acid is amplified prior to detection.

4. The method of claim 1 or claim 2, wherein the step of detecting the presence of said CNV is performed using a process selected from the group consisting of detection of specific hybridization, measurement of allele size, restriction fragment length polymorphism analysis, allele-specific hybridization analysis, single base primer extension reaction, and sequencing of an amplified polynucleotide.

5. The method of claim 1 or claim 2, wherein in the target nucleic acid is DNA.

6. The method of claim 1 or claim 2, wherein polynucleotides comprising said CNV are obtained from an isolated cell obtained from said patient.

7. A method for identifying therapeutic agents which alter neuronal signaling and/or morphology, comprising

    a) providing cells comprising at least one CNV ;
    b) providing cells which comprise the cognate wild type sequences corresponding to the CNV of step a);
    c) contacting the cells of steps a) and b) with a test agent and
    d) analyzing whether said agent alters neuronal signaling and/or morphology of cells of step a) relative to those of step b), thereby identifying agents which alter neuronal signaling and morphology; wherein said at least one CNV is

    BZRAP1 Benzodiazapine receptor (peripheral) associated protein 1 17q22-q23 chr17:53733592-53761151.

8. The method of claim 7 wherein the at least one CNV further comprises one or more CNVs selected from
MDGA2 MAM domain containing glycosylphosphatidylinositol anchor 214q21.3 chr14:46,170,380-47,422,368,
CLCNKA chloride channel Ka chr1: 16221072-16233132,
NTRK1 Neurotrophic tyrosine kinase, receptor, type 1 1q21-q22 chr1: 155,013,407-155,202,154, USH2A Usher syndrome 2A (autosomal recessive, mild) 1q41 chr1:213,752,880-214,875,391,
NRXN1 Neurexin 1 2p16.3 chr2:49,712,184-51,360,413,
GALNT13 UDP-N-acetyl-alpha-D-galactosamine:polypeptide N-acetylgal 2q23.3-q24.1 chr2:153,854,689-155,600,757,
GMPS Guanine monophosphate synthetase 3q24 chr3:157,059,820-157,149,414,
SPON2 Spondin 2, extracellular matrix protein 4p16.3 chr4:1,124,285-1,183,034,
LRBA LPS-responsive vesicle trafficking, beach and anchor containin4q31.3 chr4:151,217,225-152,344,150,
TPPP Tubulin polymerization promoting protein 5p15.3 chr5:567,501-892,810,
SKIV2L2 Superkiller viralicidic activity 2-like 2 (S. cerevisiae) 5q11.2 chr5:54,522,183-54,873,752,
KIAA1586 KIAA1586 6p12.1 chr6:56,980,593-57,066,702,
BTN2A1 Butyrophilin, subfamily 2, member A1 6p22.1 chr6:26566167-26577844, BXDC1 Brix domain containing 1 6q21 chr6:111409983-111453487,
LAMA2 Laminin, alpha 2 (merosin, congenital muscular dystrophy) 6q22-q23 chr6:128,945,101-130,370,307,
DGKB Diacylglycerol kinase, beta 90kDa 7p21.2 chr7:14,015,810-15,013,734,
RNF133 Ring finger protein 133 7q31.32 chr7:122,118,508-122,132,937,
RNF148 Ring finger protein 148 7q31.33 chr7:122,118,508-122,132,937,
SLC18A1 Solute carrier family 18 (vesicular monoamine), member 1 8p21.3 chr8:19,874,095-20,257,554,
COL27A1 Collagen, type XXVII, alpha 1 9q32 chr9:115958051-116112796,
OR2AG1 Olfactory receptor, family 2, subfamily AG, member 1 11p15.4 chr11:6762845-6763795,
OR2AG2 Olfactory receptor, family 2, subfamily AG, member 2 11p15.4 chr11:6745814-6746764,
SSSCA1 Sjogren syndrome/scleroderma autoantigen 1 11q13.1 chr11:65094518-65095815,
FAM89B Family with sequence similarity 89, member B 11q23 chr11:65,094,554-65,100,079,
PRB3 Proline-rich protein BstNI subfamily 3 12p13.2 chr12:11310124-11313908,
KRT3 Keratin 3 12q12-q13 chr12:51,444,040-51,501,855,
SLC6A15 Solute carrier family 6, member 15 12q21.3 chr12:83,670,976-83,958,489,
DACH1 Dachshund homolog 1 (Drosophila) 13q22 chr13:70910098-71339331,
LOC650137 Seven transmembrane helix receptor 15q11.2 chr15:19,812,808-20,018,007,
OR4M2 Olfactory receptor, family 4, subfamily M, member 2 15q11.2 chr15:19,812,808-20,018,007,
OR4N4 Hypothetical LOC727924 15q11.2 chr15:19,812,808-20,018,007,
LOC162073 hypothetical protein LOC162073 16p12.3 chr16:19,008,005-19,060,144,
DLGAP1 Discs, large (Drosophila) homolog-associated protein 1 18p11.3 chr18:3,393,512-3,965,460,

FLJ44894 Homo sapiens cDNA FLJ44894 fis, clone BRAMY3000692, m 19p12 chr19:20,227,461-20,491 ,547,
CYP4F22 Cytochrome P450, family 4, subfamily F, polypeptide 22 19p13.12 chr19: 15480335-15524128,
GRIK5 Glutamate receptor, ionotropic, kainate 5 19q13.2 chr19:47,126,828-47,329,282,
GYG2 Glycogenin 2 Xp22.3 chrX:2,656,547-2,925,352,
XG Xg pseudogene, Y-linked 2 Xp22.33 chrX:2,656,547-2,925,353,
FGF13 Fibroblast growth factor 13 Xq26.3 chrX:137,421,326-138,459,367,
SPANXB1 SPANX family, member B2 Xq27.1 chrX: 139,908,245-139,941,724, and
SPANXB2 SPANX family, member B2 Xq27.1 chrX: 139,908,245-139,941,724; or the group of CNVs consisting of
CNVs set forth in Table 6.

9. The method of claim 8 wherein said agent has efficacy for the treatment of neurodevelopmental disorders.

**Patentansprüche**

1. Verfahren zum Detektieren einer Tendenz zur Entwicklung einer neurologischen Störung, wobei das Verfahren Folgendes umfasst: Detektieren der Gegenwart zumindest einer Kopienzahlvariation (CNV) in einem Zielpolynucleotid, das von einem Patienten erhalten wurde, wobei der Patient, wenn die CNV vorliegt, ein erhöhtes Risiko für die Entwicklung einer neurologischen Störung aufweist, wobei die Deletion enthaltende CNV BZRAP1 Benzodiazapin-Rezeptor-assoziiertes (peripheres) Protein 1 17q22-q23 chr17:53733592-53761151 ist; und wobei die neurologische Störung Autismus oder eine Autismus-Spektrum-Störung ist.

2. Verfahren nach Anspruch 1, das das Detektieren der Gegenwart von zumindest einer weiteren Deletion enthaltenen CNV umfasst, die aus Folgendem ausgewählt ist:

MDGA2 MAM-Domäne mit Glykosylphosphatidylinosit-Anker 214q21.3 chr14:46,170,380-47,422,368,
CLCNKA Chloridkanal Ka chr1 :16221072-16233132,
NTRK1 Neurotrophische Tyrosinkinase, Rezeptor, Typ 1 1q21-q22 chr1 :155,013,407-155,202,154, USH2A Usher-Syndrom 2A (autosomal rezessiv, mild) 1q41 chr1:213,752,880-214,875,391,
NRXN1 Neurexin 1 2p16.3 chr2:49,712,184-51,360,413,
GALNT13 UDP-N-Acetyl-alpha-D-Galaktosamin:Polypeptid N-Acetylgal 2q23.3-q24.1 chr2:153,854,689-155,600,757,
GMPS Guaninmonophosphat-Synthetase 3q24 chr3:157,059,820-157,149,414,
SPON2 Spondin 2, extrazelluläres Matrixprotein 4p16.3 chr4:1,124,285-1,183,034,
LRBA LPS-responsiver Vesikeltransport, enthaltend Strand und Anker 4q31.3 chr4:151,217,225-152,344,150,
TPPP Tubulinpolymerisation-förderndes Protein 5p15.3 chr5:567,501-892,810,
SKIV2L2 Superkiller viralizidische Aktivität 2-like 2 (S. cerevisiae) 5q11.2 chr5:54,522,183-54,873,752,
KIAA1586 KIAA1586 6p12.1 chr6:56,980,593-57,066,702,
BTN2A1 Butyrophilin, Unterfamilie 2, Mitglied A1 6p22.1 chr6:26566167-26577844,
BXDC1 mit Brix-Domäne 1 6q21 chr6:111409983-111453487,
LAMA2 Laminin, Alpha 2 (Merosin, angeborene Muskeldystrophie) 6q22-q23 chr6:128,945,101-130,370,307,
DGKB Diacylglycerinkinase, Beta 90 kDa 7p21.2 chr7:14,015,810-15,013,734,
RNF133 Ringfinger-Protein 133 7q31.32 chr7:122,118,508-122,132,937,
RNF148 Ringfinger-Protein 148 7q31.33 chr7:122,118,508-122,132,937,
SLC18A1 Gelöste Trägerfamilie 18 (vesikuläres Monoamin), Mitglied 1 8p21.3 chr8:19,874,095-20,257,554,
COL27A1 Kollagen, Typ XXVII, Alpha 1 9q32 chr9:115958051-116112796,
OR2AG1 Geruchsrezeptor, Familie 2, Unterfamilie AG, Mitglied 1 11p15.4 chr11:6762845-6763795,
OR2AG2 Geruchsrezeptor, Familie 2, Unterfamilie AG, Mitglied 2 11p15.4 chr11:6745814-6746764,
SSSCA1 Sjögren-Syndrom/Sklerodermie-Autoantigen 1 11q13.1 chr11:65094518-65095815,
FAM89B Familie mit Sequenzähnlichkeit 89, Mitglied B 11q23 chr11:65,094,554-65,100,079,
PRB3 Prolin-reiches Protein BstNI Unterfamilie 3 12p13.2 chr12:11310124-11313908,
KRT3 Keratin 3 12q12-q13 chr12:51,444,040-51,501,855,
SLC6A15 Gelöste Trägerfamilie 6, Mitglied 15 12q21.3 chr12:83,670,976-83,958,489,
DACH1 Dackel-Homolog 1 (Drosophila) 13q22 chr13:70910098-71339331,
LOC650137 Sieben-Transmembran-Helix-Rezeptor 15q11.2 chr15:19,812,808-20,018,007,
OR4M2 Geruchsrezeptor, Familie 4, Unterfamilie M, Mitglied 2 15q11.2 chr15:19,812,808-20,018,007,
OR4N4 Hypothetisches LOC727924 15q11.2 chr15:19,812,808-20,018,007,
LOC162073 hypothetisches Protein LOC162073 16p12.3 chr16:19,008,005,005-19,060,144,

DLGAP1 Scheiben, großes (Drosophila) homolog-assoziiertes Protein 1 18p11.3 chr18:3,393,512-3,965,460,
FLJ44894 Homo sapiens cDNA FLJ44894 fis, Klon BRAMY3000692, m 19p12 chr19:20,227,461-20,491,547,
CYP4F22 Cytochrom P450, Familie 4, Unterfamilie F, Polypeptid 22 19p13.12 chr19:15480335-15524128,
GRIK5 Glutamatrezeptor, ionotrop, Kainat 5 19q13.2 chr19:47,126,828-47,329,282,
GYG2 Glykogenin 2 Xp22.3 chrX:2,656,547-2,925,352,
XG Xg Pseudogen, Y-verknüpft 2 Xp22.33 chrX:2,656,547-2,925,353,
FGF13 Fibroblasten-Wachstumsfaktor 13 Xq26.3 chrX:137,421,326-138,459,367,
SPANXB1 SPANX-Familie, Mitglied B2 Xq27.1 chrX:139,908,245-139,941,724 und
SPANXB2 SPANX-Familie, Mitglied B2 Xq27.1 chrX:139,908,245-139,941,724; oder
der Gruppe von CNV, die aus in Tabelle 6 angegebenen CNV besteht.

3. Verfahren nach Anspruch 1 oder Anspruch 2, wobei die Zielnucleinsäure vor dem Detektieren amplifiziert wird.

4. Verfahren nach Anspruch 1 oder Anspruch 2, wobei der Schritt des Detektierens der Gegenwart der CNV unter Verwendung eines Vorgangs durchgeführt wird, der aus der aus der Detektion spezifischer Hybridisierung, Messung der Allelgröße, Restriktionsfragmentlängen-Polymorphismusanalyse, allelspezifischer Hybridisierungsanalyse, Einzel-Base-Primer-Extensionsreaktion und Sequenzierung eines amplifizierten Polynucleotids bestehenden Gruppe ausgewählt ist.

5. Verfahren nach Anspruch 1 oder Anspruch 2, wobei die Zielnucleinsäure DNA ist.

6. Verfahren nach Anspruch 1 oder Anspruch 2, wobei die Polynucleotide, die die CNV umfassen, von einer isolierten Zelle erhalten werden, die von dem Patienten erhalten wurde.

7. Verfahren zum Identifizieren von Therapeutika, die die neuronale Signalgebung und/oder Morphologie verändern, das Folgendes umfasst:

a) Bereitstellen von zumindest eine CNV enthaltenden Zellen;
b) Bereitstellen von Zellen, die die verwandten Wildtypsequenzen umfassen, die der CNV aus Schritt a) entsprechen;
c) Kontaktieren der Zellen aus Schritt a) und b) mit einem Testmittel und
d) Analysieren, ob das Mittel die neuronale Signalgebung und/oder Morphologie der Zellen aus Schritt a) in Bezug auf die aus Schritt b) verändert, dadurch Identifizieren von Mitteln, die die neuronale Signalgebung und Morphologie verändern, wobei zumindest eine CNV Folgendes ist:

BZRAP1 Benzodiazapin-Rezeptor-assoziiertes (peripheres) Protein 1 17q22-q23 chr17:53733592-53761151.

8. Verfahren nach Anspruch 7, wobei die zumindest eine CNV weiters eine oder mehrere CNV umfasst, die aus Folgendem ausgewählt sind:

MDGA2 MAM-Domäne mit Glykosylphosphatidylinosit-Anker 214q21.3 chr14:46,170,380-47,422,368,
CLCNKA Chloridkanal Ka chr1:16221072-16233132,
NTRK1 Neurotrophische Tyrosinkinase, Rezeptor, Typ 1 1q21-q22 chr1:155,013,407-155,202,154, USH2A Usher-Syndrom 2A (autosomal rezessiv, mild) 1q41 chr1:213,752,880-214,875,391,
NRXN1 Neurexin 1 2p16.3 chr2:49,712,184-51,360,413,
GALNT13 UDP-N-Acetyl-alpha-D-Galaktosamin:Polypeptid N-Acetylgal 2q23.3-q24.1 chr2:153,854,689-155,600,757,
GMPS Guaninmonophosphat-Synthetase 3q24 chr3:157,059,820-157,149,414,
SPON2 Spondin 2, extrazelluläres Matrixprotein 4p16.3 chr4:1,124,285-1,183,034,
LRBA LPS-responsiver Vesikeltransport mit Strand und Anker 4q31.3 chr4:151,217,225-152,344,150,
TPPP Tubulinpolymerisation-förderndes Protein 5p15.3 chr5:567,501-892,810,
SKIV2L2 Superkiller viralizidische Aktivität 2-like 2 (S. cerevisiae) 5q11.2 chr5:54,522,183-54,873,752,
KIAA1586 KIAA1586 6p12.1 chr6:56,980,593-57,066,702,
BTN2A1 Butyrophilin, Unterfamilie 2, Mitglied A1 6p22.1 chr6:26566167-26577844,
BXDC1 mit Brix-Domäne 1 6q21 chr6:111409983-111453487,
LAMA2 Laminin, Alpha 2 (Merosin, angeborene Muskeldystrophie) 6q22-q23 chr6:128,945,101-130,370,307,
DGKB Diacylglycerinkinase, Beta 90 kDa 7p21.2 chr7:14,015,810-15,013,734,
RNF133 Ringfinger-Protein 133 7q31.32 chr7:122,118,508-122,132,937,

RNF148 Ringfinger-Protein 148 7q31.33 chr7:122,118,508-122,132,937,
SLC18A1 Gelöste Trägerfamilie 18 (vesikuläres Monoamin), Mitglied 1 8p21.3 chr8:19,874,095-20,257,554,
COL27A1 Kollagen, Typ XXVII, Alpha 1 9q32 chr9:115958051-116112796,
OR2AG1 Geruchsrezeptor, Familie 2, Unterfamilie AG, Mitglied 1 11p15.4 chr11:6762845-6763795,
OR2AG2 Geruchsrezeptor, Familie 2, Unterfamilie AG, Mitglied 2 11p15.4 chr11:6745814-6746764,
SSSCA1 Sjögren-Syndrom/Sklerodermie-Autoantigen 1 11q13.1 chr11:65094518-65095815,
FAM89B Familie mit Sequenzähnlichkeit 89, Mitglied B 11q23 chr11:65,094,554-65,100,079,
PRB3 Prolin-reiches Protein BstNI Unterfamilie 3 12p13.2 chr12:11310124-11313908,
KRT3 Keratin 3 12q12-q13 chr12:51,444,040-51,501,855,
SLC6A15 Gelöste Trägerfamilie 6, Mitglied 15 12q21.3 chr12:83,670,976-83,958,489,
DACH1 Dackel-Homolog 1 (Drosophila) 13q22 chr13:70910098-71339331,
LOC650137 Sieben-Transmembran-Helix-Rezeptor 15q11.2 chr15:19,812,808-20,018,007,
OR4M2 Geruchsrezeptor, Familie 4, Unterfamilie M, Mitglied 2 15q11.2 chr15:19,812,808-20,018,007,
OR4N4 Hypothetisches LOC727924 15q11.2 chr15:19,812,808-20,018,007,
LOC162073 hypothetisches Protein LOC162073 16p12.3 chr16:19,008,005,005-19,060,144,
DLGAP1 Scheiben, großes (Drosophila) homolog-assoziiertes Protein 1 18p11.3 chr18:3,393,512-3,965,460,
FLJ44894 Homo sapiens cDNA FLJ44894 fis, Klon BRAMY3000692, m 19p12 chr19:20,227,461-20,491,547,
CYP4F22 Cytochrom P450, Familie 4, Unterfamilie F, Polypeptid 22 19p13.12 chr19:15480335-15524128,
GRIK5 Glutamatrezeptor, ionotrop, Kainat 5 19q13.2 chr19:47,126,828-47,329,282,
GYG2 Glykogenin 2 Xp22.3 chrX:2,656,547-2,925,352,
XG Xg Pseudogen, Y-verknüpft 2 Xp22.33 chrX:2,656,547-2,925,353,
FGF13 Fibroblasten-Wachstumsfaktor 13 Xq26.3 chrX:137,421,326-138,459,367,
SPANXB1 SPANX-Familie, Mitglied B2 Xq27.1 chrX:139,908,245-139,941,724 und
SPANXB2 SPANX-Familie, Mitglied B2 Xq27.1 chrX:139,908,245-139,941,724; oder
der Gruppe von CNV, die aus in Tabelle 6 angegebenen CNV besteht.

9. Verfahren nach Anspruch 8, wobei das Mittel eine Wirksamkeit für die Behandlung von neurologischen Entwicklungsstörungen aufweist.

## Revendications

1. Procédé pour détecter une propension à développer un trouble neurologique, le procédé comprenant : la détection de la présence d'au moins une variation du nombre de copies (CNV) dans un polynucléotide cible obtenu chez un patient, dans lequel si ladite CNV est présente, ledit patient présente un risque accru de développer un trouble neurologique, dans lequel ladite CNV contenant une délétion est BZRAP1, protéine 1 associée aux récepteurs des benzodiazépines (périphériques), 17q22-q23 chr17:53733592-53761151 ; et dans lequel le trouble neurologique est l'autisme ou un trouble du spectre de l'autisme.

2. Procédé selon la revendication 1, comprenant la détection de la présence d'au moins une CNV contenant une délétion supplémentaire sélectionnée parmi
MDGA2, domaine MAM contenant une ancre glycosylphosphatidylinositol, 214q21.3 chr14:46,170,380-47,422,368,
CLCNKA, canal chlorure Ka, chr1:16221072-16233132,
NTRK1, récepteur tyrosine kinase neurotrophique type 1, 1q21-q22 chr1:155,013,407-155,202,154, USH2A, syndrome de Usher 2A (autosomique récessif, léger), 1q41 chr1:213,752,880-214,875,391,
NRXN1, neuréxine 1, 2p16.3 chr2:49,712,184-51,360,413,
GALNT13, UDP-N-acétyl-alpha-D-galactosamine:polypeptide N-acétylgal, 2q23.3-q24.1 chr2:153,854,689-155,600,757,
GMPS, guanine monophosphate synthétase, 3q24 chr3:157,059,820-157,149,414,
SPON2, Spondine 2, protéine matricielle extracellulaire, 4p16.3 chr4:1,124,285-1,183,034,
LRBA, protéine de trafic vésiculaire sensible au LPS, contenant un domaine BEACH et une ancre, 4q31.3 chr4:151,217,225-152,344,150,
TPPP, protéine favorisant la polymérisation de la tubuline, 5p15.3 chr5:567,501-892,810,
SKIV2L2, protéine 2 de type « superkiller » à activité virucide 2 (S. cerevisiae), 5q11.2 chr5:54,522, 183-54,873,752,
KIAA1586, KIAA1586, 6p12.1 chr6:56,980,593-57,066,702,
BTN2A1, butyrophiline, sous-famille 2, membre A1, 6p22.1 chr6:26566167-26577844,
BXDC1, protéine 1 contenant un domaine Brix, 6q21 chr6:111409983-111453487,
LAMA2, laminine, alpha 2 (mérosine, dystrophie musculaire congénitale), 6q22-q23 chr6:128,945,101-130,370,307,

DGKB, diacylglycérol kinase, bêta 90 kDa, 7p21.2 chr7:14,015,810-15,013,734,

RNF133, protéine contenant un domaine de doigt RING 133, 7q31.32 chr7:122,118,508-122,132,937,

RNF148, protéine contenant un domaine de doigt RING 148, 7q31.33 chr7:122,118,508-122,132,937,

SLC18A1, transporteur de solutés de la famille 18 (monoamine vésiculaire), membre 1, 8p21.3 chr8:19,874,095-20,257,554,

COL27A1, collagène, type XXVII, alpha 1, 9q32 chr9:115958051-116112796,

OR2AG1, récepteur olfactif, famille 2, sous-famille AG, membre 1, 11p15.4 chr11:6762845-6763795,

OR2AG2, récepteur olfactif, famille 2, sous-famille AG, membre 2, 11p15.4 chr11:6745814-6746764,

SSSCA1, auto-antigène 1 du syndrome de Sjögren/sclérodermie, 11q13.1 chr11:65094518-65095815,

FAM89B, famille présentant une similarité de séquence 89, membre B, 11q23 chr11:65,094,554-65,100,079,

PRB3, protéine riche en proline, sous-famille BstNI 3, 12p13.2 chr12:11310124-11313908,

KRT3, kératine 3, 12q12-q13 chr12:51,444,040-51,501,855,

SLC6A15, transporteur de solutés de la famille 6, membre 15, 12q21.3 chr12:83,670,976-83,958,489,

DACH1, homologue 1 de Dachshund (Drosophila), 13q22 chr13:70910098-71339331,

LOC650137, récepteur en hélice à sept domaines transmembranaires, 15q11.2 chr15:19,812,808-20,018,007,

OR4M2, récepteur olfactif, famille 4, sous-famille M, membre 2, 15q11.2 chr15:19,812,808-20,018,007,

OR4N4, protéine hypothétique LOC727924, 15q11.2 chr15:19,812,808-20,018,007,

LOC162073, protéine hypothétique LOC162073, 16p12.3 chr16:19,008,005-19,060,144,

DLGAP1, protéine 1 homologue associée aux grands disques (Drosophila), 18p11.3 chr18:3,393,512-3,965,460,

FLJ44894, ADNc d'Homo sapiens FLJ44894 fis, clone BRAMY3000692, m, 19p12 chr19:20,227,461-20,491,547,

CYP4F22, Cytochrome P450, famille 4, sous-famille F, polypeptide 22, 19p13.12 chr19:15480335-15524128,

GRIK5, récepteur du glutamate, ionotrope, kaïnate 5, 19q13.2 chr19:47,126,828-47,329,282,

GYG2, Glycogénine 2, Xp22.3 chX:2,656,547-2,925,352,

XG, pseudogène Xg, lié à Y 2, Xp22.33 chrX:2,656,547-2,925,353,

FGF13, facteur de croissance des fibroblastes 13, Xq26.3 chrX:137,421,326-138,459,367,

SPANXB1, famille SPANX, membre B2, Xq27.1 chrX:139,908,245-139,941,724, et

SPANXB2, famille SPANX, membre B2, Xq27.1 chrX:139,908,245-139,941,724 ; ou le groupe de CNV consistant en les CNV décrites dans le Tableau 6.

3. Procédé selon la revendication 1 ou la revendication 2, dans lequel l'acide nucléique cible est amplifié avant la détection.

4. Procédé selon la revendication 1 ou la revendication 2, dans lequel l'étape de détection de la présence de ladite CNV est réalisée en utilisant un procédé sélectionné dans le groupe consistant en la détection d'une hybridation spécifique, une mesure de la taille d'un allèle, une analyse de polymorphisme de longueur des fragments de restriction, une analyse d'hybridation spécifique à un allèle, une réaction d'extension d'amorce d'une seule base, et le séquençage d'un polynucléotide amplifié.

5. Procédé selon la revendication 1 ou la revendication 2, dans lequel l'acide nucléique cible est de l'ADN.

6. Procédé selon la revendication 1 ou la revendication 2, dans lequel les polynucléotides comprenant ladite CNV sont obtenus à partir d'une cellule isolée obtenue chez ledit patient.

7. Procédé pour identifier des agents thérapeutiques qui altèrent la signalisation et/ou la morphologie neuronale, comprenant

a) la mise à disposition de cellules comprenant au moins une CNV ;
b) la mise à disposition de cellules qui comprennent les séquences de type sauvage apparentées correspondant à la CNV de l'étape a) ;
c) la mise en contact des cellules des étapes a) et b) avec un agent de test et
d) l'analyse du fait que lesdits agents altèrent ou non la signalisation et/ou la morphologie neuronale des cellules de l'étape a) par rapport à celles de l'étape b), en identifiant ainsi des agents qui altèrent la signalisation et la morphologie neuronale ; dans lequel ladite au moins une CNV est

BZRAP1, protéine 1 associée aux récepteurs des benzodiazépines (périphériques), 17q22-q23 chr17:53733592-53761151.

8. Procédé selon la revendication 7, dans lequel la au moins une CNV comprend en outre une ou plusieurs CNV sélectionnées parmi

MDGA2, domaine MAM contenant une ancre glycosylphosphatidylinositol, 214q21.3 chr14:46,170,380-47,422,368,

CLCNKA, canal chlorure Ka, chr1:16221072-16233132,

NTRK1, récepteur tyrosine kinase neurotrophique type 1, 1q21-q22 chr1:155,013,407-155,202,154, USH2A, syndrome de Usher 2A (autosomique récessif, léger), 1q41 chr1:213,752,880-214,875,391,

NRXN1, neuréxine 1, 2p16.3 chr2:49,712,184-51,360,413,

GALNT13, UDP-N-acétyl-alpha-D-galactosamine:polypeptide N-acétylgal, 2q23.3-q24.1 chr2:153,854,689-155,600,757,

GMPS, guanine monophosphate synthétase, 3q24 chr3:157,059,820-157,149,414,

SPON2, Spondine 2, protéine matricielle extracellulaire, 4p16.3 chr4:1,124,285-1,183,034,

LRBA, protéine de trafic vésiculaire sensible au LPS, contenant un domaine BEACH et une ancre, 4q31.3 chr4:151,217,225-152,344,150,

TPPP, protéine favorisant la polymérisation de la tubuline, 5p15.3 chr5:567,501-892,810,

SKIV2L2, protéine 2 de type « superkiller » à activité virucide 2 (S. cerevisiae), 5q11.2 chr5:54,522, 183-54,873,752,

KIAA1586, KIAA1586, 6p12.1 chr6:56,980,593-57,066,702,

BTN2A1, butyrophiline, sous-famille 2, membre A1, 6p22.1 chr6:26566167-26577844,

BXDC1, protéine 1 contenant un domaine Brix, 6q21 chr6:111409983-111453487,

LAMA2, laminine, alpha 2 (mérosine, dystrophie musculaire congénitale), 6q22-q23 chr6:128,945,101-130,370,307,

DGKB, diacylglycérol kinase, bêta 90 kDa, 7p21.2 chr7:14,015,810-15,013,734,

RNF133, protéine contenant un domaine de doigt RING 133, 7q31.32 chr7:122,118,508-122,132,937,

RNF148, protéine contenant un domaine de doigt RING 148, 7q31.33 chr7:122,118,508-122,132,937,

SLC18A1, transporteur de solutés de la famille 18 (monoamine vésiculaire), membre 1, 8p21.3 chr8:19,874,095-20,257,554,

COL27A1, collagène, type XXVII, alpha 1, 9q32 chr9:115958051-116112796,

OR2AG1, récepteur olfactif, famille 2, sous-famille AG, membre 1, 11p15.4 chr11:6762845-6763795,

OR2AG2, récepteur olfactif, famille 2, sous-famille AG, membre 2, 11p15.4 chr11:6745814-6746764,

SSSCA1, auto-antigène 1 du syndrome de Sjögren/sclérodermie, 11q13.1 chr11:65094518-65095815,

FAM89B, famille présentant une similarité de séquence 89, membre B, 11q23 chr11:65,094,554-65,100,079,

PRB3, protéine riche en proline, sous-famille BstNI 3, 12p13.2 chr12:11310124-11313908,

KRT3, kératine 3, 12q12-q13 chr12:51,444,040-51,501,855,

SLC6A15, transporteur de solutés de la famille 6, membre 15, 12q21.3 chr12:83,670,976-83,958,489,

DACH1, homologue 1 de Dachshund (Drosophila), 13q22 chr13:70910098-71339331,

LOC650137, récepteur en hélice à sept domaines transmembranaires, 15q11.2 chr15:19,812,808-20,018,007,

OR4M2, récepteur olfactif, famille 4, sous-famille M, membre 2, 15q11.2 chr15:19,812,808-20,018,007,

OR4N4, protéine hypothétique LOC727924, 15q11.2 chr15:19,812,808-20,018,007,

LOC162073, protéine hypothétique LOC162073, 16p12.3 chr16:19,008,005-19,060,144,

DLGAP1, protéine 1 homologue associée aux grands disques (Drosophila), 18p11.3 chr18:3,393,512-3,965,460,

FLJ44894, ADNc d'Homo sapiens FLJ44894 fis, clone BRAMY3000692, m, 19p12 chr19:20,227,461-20,491,547,

CYP4F22, Cytochrome P450, famille 4, sous-famille F, polypeptide 22, 19p13.12 chr19:15480335-15524128,

GRIK5, récepteur du glutamate, ionotrope, kaïnate 5, 19q13.2 chr19:47,126,828-47,329,282,

GYG2, Glycogénine 2, Xp22.3 chrX:2,656,547-2,925,352,

XG, pseudogène Xg, lié à Y 2, Xp22.33 chrX:2,656,547-2,925,353,

FGF13, facteur de croissance des fibroblastes 13, Xq26.3 chrX:137,421,326-138,459,367,

SPANXB1, famille SPANX, membre B2, Xq27.1 chrX:139,908,245-139,941,724, et

SPANXB2, famille SPANX, membre B2, Xq27.1 chrX:139,908,245-139,941,724 ; ou le groupe de CNV consistant en les CNV décrites dans le Tableau 6.

9. Procédé selon la revendication 8, dans lequel ledit agent est efficace pour le traitement de troubles du neurodéveloppement.

## Figure 1

Figure 2

# Figure 3

BZRAP1

NRXN1

## Figure 4A

**Figure 4B**

# Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4683195 A
- US 4800195 A
- US 4965188 A
- WO 8403564 A
- US 6057098 A
- US 5965456 A
- US 5877399 A
- US 5612486 A
- US 5387742 A
- US 5811633 A
- US 5849999 A

### Non-patent literature cited in the description

- **KUSENDA et al.** Cytogenet Genome Res. 2008, vol. 123, 36-43
- **ROPERS.** Current Opinion in Genetics & Development. Current Biology Ltd, 2008, vol. 18, 241-250
- **COSTA E SILVA.** *Metabolism, Clinical and Experimental,* 2008, vol. 57, S40-S43
- **DAVID ST CLAIR.** Schizophrenia Bulletin. Oxford University Press, 2008, vol. 35, 9-12
- **LUI et al.** *Biol. Psychiatry,* 2008, vol. 64, 561-570
- **LEVY et al.** *PLoS Biology,* October 2007, vol. 5 (10
- **FEUK et al.** *Nature,* 2006, vol. 444, 444-54
- **IAFRATE et al.** *Nature Genetics,* 2004, vol. 36, 949-51
- **SEBAT et al.** *Science,* 2004, vol. 305, 525-8
- **TUZUN et al.** *Genome Res.,* 2006, vol. 16, 949-961
- **SAMBROOK et al.** Molecular Cloning. Cold Spring Harbor Laboratory, 1989
- Current Protocols in Molecular Biology. John Wiley & Sons, 1995
- Molecular Cloning: A Laboratory Manual or Current Protocols. **SAMBROOK et al.** Molecular Biology. 1989, 16.3-17.44
- *Current Protocols in Molecular Biology,* 1989
- **HODGSON.** *Bio/Technology,* 1991, vol. 9, 19-21
- **ERICKSON et al.** *Science,* 1990, vol. 249, 527-533
- **WELLS.** *Meth. Enzym.,* 1991, vol. 202, 390-411
- **EVANS et al.** *Nature,* 1981, vol. 292, 154-156
- **BRADLEY et al.** *Nature,* 1984, vol. 309, 255-258
- **GOSSLER et al.** *Proc. Natl. Acad. Sci.,* 1986, vol. 83, 9065-9069
- **FROHMAN et al.** *Cell,* 1989, vol. 56, 145-147
- **BRADLEY et al.** *Bio/Technology,* 1992, vol. 10, 534-539
- **BORCHELT et al.** *Genet. Anal.,* 1996, vol. 13 (6), 159-163
- **ABRAHAMS BS ; GESCHWIND DH.** Advances in autism genetics: on the threshold of a new neurobiology. *Nat Rev Genet,* 2008, vol. 9, 341-355
- **BAILEY A ; LE COUTEUR A ; GOTTESMAN I ; BOLTON P ; SIMONOFF E et al.** Autism as a strongly genetic disorder: evidence from a British twin study. *Psychol Med,* 1995, vol. 25, 63-77
- **STEFFENBURG S ; GILLBERG C ; HELLGREN L ; ANDERSSON L ; GILLBERG IC et al.** A twin study of autism in Denmark, Finland, Iceland, Norway and Sweden. *J Child Psychol Psychiatry,* 1989, vol. 30, 405-416
- **CANTOR RM ; KONO N ; DUVALL JA ; ALVAREZ-RETUERTO A ; STONE JL et al.** Replication of autism linkage: fine-mapping peak at 17q21. *Am J Hum Genet,* 2005, vol. 76, 1050-1056
- **VORSTMAN JA ; STAAL WG ; VAN DAALEN E ; VAN ENGELAND H ; HOCHSTENBACH PF et al.** Identification of novel autism candidate regions through analysis of reported cytogenetic abnormalities associated with autism. *Mol Psychiatry,* 2006, vol. 11, 118-28
- **SZATMARI P ; PATERSON AD ; ZWAIGENBAUM L ; ROBERTS W ; BRIAN J et al.** Mapping autism risk loci using genetic linkage and chromosomal rearrangements. *Nat Genet,* 2007, vol. 39, 319-328
- **SEBAT J ; LAKSHMI B ; MALHOTRA D ; TROGE J ; LESE-MARTIN C et al.** Strong association of de novo copy number mutations with autism. *Science,* 2007, vol. 316, 445-449
- **MARSHALL CR ; NOOR A ; VINCENT JB ; LIONEL AC ; FEUK L et al.** Structural variation of chromosomes in autism spectrum disorder. *Am J Hum Genet,* 2008, vol. 82, 477-488

- **JACQUEMONT ML ; SANLAVILLE D ; REDON R ; RAOUL O ; CORMIER-DAIRE V et al.** Array-based comparative genomic hybridisation identifies high frequency of cryptic chromosomal rearrangements in patients with syndromic autism spectrum disorders. *J Med Genet,* 2006, vol. 43, 843-849
- **KUMAR RA ; KARAMOHAMED S ; SUDI J ; CONRAD DF ; BRUNE C et al.** Recurrent 16p11.2 microdeletions in autism. *Hum Mol Genet,* 2007
- **WEISS LA ; SHEN Y ; KORN JM ; ARKING DE ; MILLER DT et al.** Association between Microdeletion and Microduplication at 16p11.2 and Autism. *N Engl J Med,* 2008
- **SHARP AJ ; MEFFORD HC ; LI K ; BAKER C ; SKINNER C et al.** A recurrent 15q13.3 microdeletion syndrome associated with mental retardation and seizures. *Nat Genet,* 2008, vol. 40, 322-328
- **MEFFORD HC ; SHARP AJ ; BAKER C ; ITSARA A ; JIANG Z et al.** Recurrent rearrangements of chromosome 1q21.1 and variable pediatric phenotypes. *N Engl J Med,* 2008, vol. 359, 1685-1699
- **ALARCON M ; ABRAHAMS BS ; STONE JL ; DUVALL JA ; PEREDERIY JV et al.** Linkage, Association, and Gene-Expression Analyses Identify CNTNAP2 as an Autism-Susceptibility Gene. *Am J Hum Genet,* 2008, vol. 82, 150-159
- **KIM HG ; KISHIKAWA S ; HIGGINS AW ; SEONG IS ; DONOVAN DJ et al.** Disruption of neurexin 1 associated with autism spectrum disorder. *Am J Hum Genet,* 2008, vol. 82, 199-207
- **MORROW EM ; YOO SY ; FLAVELL SW ; KIM TK ; LIN Y et al.** Identifying autism loci and genes by tracing recent shared ancestry. *Science,* 2008, vol. 321, 218-223
- **GLESSNER JT ; WANG K ; CAI G ; KORVATSKA O ; KIM CE et al.** Autism genome-wide copy number variation reveals ubiquitin and neuronal genes. *Nature,* 2009
- **GESCHWIND DH ; SOWINSKI J ; LORD C ; IVERSEN P ; SHESTACK J et al.** The autism genetic resource exchange: a resource for the study of autism and related neuropsychiatric conditions. *Am J Hum Genet,* 2001, vol. 69, 463-466
- **NALLS MA ; SIMON-SANCHEZ J ; GIBBS JR ; PAISAN-RUIZ C ; BRAS JT et al.** Measures of autozygosity in decline: globalization, urbanization, and its implications for medical genetics. *PLoS Genet,* 2009, vol. 5, e1000415
- **WANG K ; LI M ; HADLEY D ; LIU R ; GLESSNER J et al.** PennCNV: an integrated hidden Markov model designed for high-resolution copy number variation detection in whole-genome SNP genotyping data. *Genome Res,* 2007, vol. 17, 1665-1674
- **CHRISTIAN SL ; BRUNE CW ; SUDI J ; KUMAR RA ; LIU S et al.** Novel submicroscopic chromosomal abnormalities detected in autism spectrum disorder. *Biol Psychiatry,* 2008, vol. 63, 1111-1117
- **CAI G ; EDELMANN L ; GOLDSMITH JE ; COHEN N ; NAKAMINE A et al.** Multiplex ligation-dependent probe amplification for genetic screening in autism spectrum disorders: Efficient identification of known microduplications and identification of a novel microduplication in ASMT. *BMC Med Genomics,* 2008, vol. 1, 50
- **MARTIN CL ; DUVALL JA ; ILKIN Y ; SIMON JS ; ARREAZA MG et al.** Cytogenetic and molecular characterization of A2BP1/FOX1 as a candidate gene for autism. *Am J Med Genet B Neuropsychiatr Genet,* 2007, vol. 144, 869-876
- **BOND J ; ROBERTS E ; MOCHIDA GH ; HAMPSHIRE DJ ; SCOTT S et al.** ASPM is a major determinant of cerebral cortical size. *Nat Genet,* 2002, vol. 32, 316-320
- **BAKKALOGLU B ; O'ROAK BJ ; LOUVI A ; GUPTA AR ; ABELSON JF et al.** Molecular Cytogenetic Analysis and Resequencing of Contactin Associated Protein-Like 2 in Autism Spectrum Disorders. *Am J Hum Genet,* 2008, vol. 82, 165-173
- **STRAUSS KA ; PUFFENBERGER EG ; HUENTELMAN MJ ; GOTTLIEB S ; DOBRIN SE et al.** Recessive symptomatic focal epilepsy and mutant contactin-associated protein-like 2. *N Engl J Med,* 2006, vol. 354, 1370-1377
- **FENG J ; SCHROER R ; YAN J ; SONG W ; YANG C et al.** High frequency of neurexin Ibeta signal peptide structural variants in patients with autism. *Neurosci Lett,* 2006, vol. 409, 10-13
- **YAN J ; NOLTNER K ; FENG J ; LI W ; SCHROER R et al.** Neurexin 1alpha structural variants associated with autism. *Neurosci Lett,* 2008, vol. 438, 368-370
- **JAMAIN S ; QUACH H ; BETANCUR C ; RASTAM M ; COLINEAUX C et al.** Mutations of the X-linked genes encoding neuroligins NLGN3 and NLGN4 are associated with autism. *Nat Genet,* 2003, vol. 34, 27-29
- **COMOLETTI D ; DE JACO A ; JENNINGS LL ; FLYNN RE ; GAIETTA G et al.** The Arg451Cys-neuroligin-3 mutation associated with autism reveals a defect in protein processing. *J Neurosci,* 2004, vol. 24, 4889-4893
- **LAUMONNIER F ; BONNET-BRILHAULT F ; GOMOT M ; BLANC R ; DAVID A et al.** X-linked mental retardation and autism are associated with a mutation in the NLGN4 gene, a member of the neuroligin family. *Am J Hum Genet,* 2004, vol. 74, 552-557
- **YAN J ; OLIVEIRA G ; COUTINHO A ; YANG C ; FENG J et al.** Analysis of the neuroligin 3 and 4 genes in autism and other neuropsychiatric patients. *Mol Psychiatry,* 2005, vol. 10, 329-332
- **SCHEIFFELE P ; FAN J ; CHOIH J ; FETTER R ; SERAFINI T.** Neuroligin expressed in nonneuronal cells triggers presynaptic development in contacting axons. *Cell,* 2000, vol. 101, 657-669

- **GRAF ER ; ZHANG X ; JIN SX ; LINHOFF MW ; CRAIG AM.** Neurexins induce differentiation of GABA and glutamate postsynaptic specializations via neuroligins. *Cell,* 2004, vol. 119, 1013-1026
- **SADAKATA T ; WASHIDA M ; IWAYAMA Y ; SHOJI S ; SATO Y et al.** Autistic-like phenotypes in Cadps2-knockout mice and aberrant CADPS2 splicing in autistic patients. *J Clin Invest,* 2007, vol. 117, 931-943
- **WALSH T ; MCCLELLAN JM ; MCCARTHY SE ; ADDINGTON AM ; PIERCE SB et al.** Rare structural variants disrupt multiple genes in neurodevelopmental pathways in schizophrenia. *Science,* 2008, vol. 320, 539-543
- **WANG Y ; SUGITA S ; SUDHOF TC.** The RIM/NIM family of neuronal C2 domain proteins. Interactions with Rab3 and a new class of Src homology 3 domain proteins. *J Biol Chem,* 2000, vol. 275, 20033-20044
- **ZOGHBI HY.** Postnatal neurodevelopmental disorders: meeting at the synapse. *Science,* 2003, vol. 302, 826-830
- **LITWACK ED ; BABEY R ; BUSER R ; GESEMANN M ; O'LEARY DD.** Identification and characterization of two novel brain-derived immunoglobulin superfamily members with a unique structural organization. *Mol Cell Neurosci,* 2004, vol. 25, 263-274
- **FERNANDEZ T ; MORGAN T ; DAVIS N ; KLIN A ; MORRIS A et al.** Disruption of contactin 4 (CNTN4) results in developmental delay and other features of 3p deletion syndrome. *Am J Hum Genet,* 2004, vol. 74, 1286-1293
- **ROOHI J ; MONTAGNA C ; TEGAY DH ; PALMER LE ; DEVINCENT C et al.** Disruption of contactin 4 in three subjects with autism spectrum disorder. *J Med Genet,* 2009, vol. 46, 176-182
- **WANG K ; ZHANG H ; MA D ; BUCAN M ; GLESSNER JT et al.** Common genetic variants on 5p14.1 associate with autism spectrum disorders. *Nature,* 2009
- **COOK EH JR ; LINDGREN V ; LEVENTHAL BL ; COURCHESNE R ; LINCOLN A et al.** Autism or atypical autism in maternally but not paternally derived proximal 15q duplication. *Am J Hum Genet,* 1997, vol. 60, 928-934
- **POTOCKI L ; BI W ; TREADWELL-DEERING D ; CARVALHO CM ; EIFERT A et al.** Characterization of Potocki-Lupski syndrome (dup(17)(p11.2p11.2)) and delineation of a dosage-sensitive critical interval that can convey an autism phenotype. *Am J Hum Genet,* 2007, vol. 80, 633-649
- Identification and characterization of the tuberous sclerosis gene on chromosome 16. *Cell,* vol. 75, 1305-1315
- **SLAGER RE ; NEWTON TL ; VLANGOS CN ; FINUCANE B ; ELSEA SH.** Mutations in RAI1 associated with Smith-Magenis syndrome. *Nat Genet,* 2003, vol. 33, 466-468
- **BOLTON P ; MACDONALD H ; PICKLES A ; RIOS P ; GOODE S et al.** A case-control family history study of autism. *J Child Psychol Psychiatry,* 1994, vol. 35, 877-900
- **BISHOP DV ; MAYBERY M ; MALEY A ; WONG D ; HILL W et al.** Using selfreport to identify the broad phenotype in parents of children with autistic spectrum disorders: a study using the Autism-Spectrum Quotient. *J Child Psychol Psychiatry,* 2004, vol. 45, 1431-1436
- **MILLAR JK ; WILSON-ANNAN JC ; ANDERSON S ; CHRISTIE S ; TAYLOR MS et al.** Disruption of two novel genes by a translocation co-segregating with schizophrenia. *Hum Mol Genet,* 2000, vol. 9, 1415-1423
- **SACHS NA ; SAWA A ; HOLMES SE ; ROSS CA ; DELISI LE et al.** A frameshift mutation in Disrupted in Schizophrenia 1 in an American family with schizophrenia and schizoaffective disorder. *Mol Psychiatry,* 2005, vol. 10, 758-764
- **RISCH N ; SPIKER D ; LOTSPEICH L ; NOURI N ; HINDS D et al.** A genomic screen of autism: evidence for a multilocus etiology. *Am J Hum Genet,* 1999, vol. 65, 493-507
- **RZHETSKY A ; WAJNGURT D ; PARK N ; ZHENG T.** Probing genetic overlap among complex human phenotypes. *Proc Natl Acad Sci U S A,* 2007, vol. 104, 11694-11699
- **BODMER W ; BONILLA C.** Common and rare variants in multifactorial susceptibility to common diseases. *Nat Genet,* 2008, vol. 40, 695-701
- **YANG S ; WANG K ; GREGORY B ; BERRETTINI W ; WANG LS et al.** Genomic landscape of a three-generation pedigree segregating affective disorder. *PLoS ONE,* 2009, vol. 4, e4474
- **JI W ; FOO JN ; O'ROAK BJ ; ZHAO H ; LARSON MG et al.** Rare independent mutations in renal salt handling genes contribute to blood pressure variation. *Nat Genet,* 2008, vol. 40, 592-599
- **DENNIS G JR ; SHERMAN BT ; HOSACK DA ; YANG J ; GAO W et al.** DAVID: Database for Annotation, Visualization, and Integrated Discovery. *Genome Biol,* 2003, vol. 4, 3